# FASCICULE DE BREVET EUROPEEN

(11) **EP 1 799 691 B1**
(45) Date de publication et mention de la délivrance du brevet: **21.12.2011**
(21) Numéro de dépôt: 05807393.3
(22) Date de dépôt: 29.09.2005
(51) Int. Cl.: C07D 519/00, A61K 31/437, A61P 35/00

(54) **NOUVEAUX DERIVES BIS-AZAINDOLES, LEUR PREPARATION ET LEUR UTILISATION PHARMACEUTIQUE COMME INHIBITEURS DE KINASES**
NEUE 2-AZAINDOL-DERIVATE, DEREN HERSTELLUNG UND PHARMAZEUTISCHE VERWENDUNG ALS KINASE-INHIBITOREN
NOVEL BIS-AZAINDOLE DERIVATIVES, PREPARATION AND PHARMACEUTICAL USE THEREOF AS KINASE INHIBITORS

(30) Priorité: 01.10.2004 FR 0410386
(43) Date de publication de la demande: 27.06.2007
(73) Titulaire: Aventis Pharma S.A., 92160 Antony (FR)
(72) Inventeur: NEMECEK, Conception, F-94320 Thiais (FR); METZ, William, A., NJ 08807 (US); WENTZLER, Sylvie c/o sanofi-aventis Département Brevets, F-75013 PARIS (FR); LESUISSE, Dominique, F-93100 Montreuil (FR); EL-AHMAD, Youssef, F-94000 Créteil (FR)
(74) Mandataire: Sartorius, Jérome
(86) Numéro de dépôt international: PCT/FR2005/002410
(87) Numéro de publication internationale: WO 2006/037875

(56) Documents cités:
- WO-A-03/000688
- WO-A-03/000695
- WO-A-03/024969
- WO-A-03/044015

## Description

La présente invention concerne de nouveaux dérivés bis-azaindoles , leur procédé de préparation, leur application comme médicaments, les compositions pharmaceutiques les renfermant et l'utilisation pharmaceutique de tels dérivés pour la prévention et le traitement d'affections capables d'être modulées par l'inhibition de l'activité de protéines kinases.

La présente invention concerne de nouveaux dérivés bis-azaindoles possédant des effets inhibiteurs de protéines kinases.

Les produits de la présente invention peuvent ainsi notamment être utilisés pour la prévention ou le traitement d'affections capables d'être modulées par l'inhibition de l'activité de protéines kinases.

L'inhibition et la régulation de protéines kinases constituent un nouveau puissant mécanisme d'action pour le traitement notamment d'un grand nombre de tumeurs solides et liquide.

De telles affections que peuvent traiter les produits de la présente demande sont donc tout particulièrement les tumeurs solides.

De telles protéines kinases appartiennent notamment au groupe suivant:
IGF1, Raf, EGF, PDGF, VEGF, Tie2, KDR, Flt1-3, FAK, Src, Abl, cKit, cdk1-9, Auroral-2, cdc7, Akt, Pdk, S6K, Jnk, IR, FLK-1, FGFR1, FGFR2, FGFR3, FGFR4, FGFR5, PLK, Pyk2, CDK7, CDK2 et EGFR.
De telles protéines kinases appartiennent plus particulièrement au groupe suivant: IGF1, cdc7, Auroral-2, Src, Jnk, FAK, KDR, IR, Tie2, CDK7., CDK2 et EGFR.

On indique particulièrement la protéine kinase IGF1 (Insulin Growth Factor-1).

La présente invention concerne ainsi particulièrement de nouveaux inhibiteurs du récepteur IGF-1R qui peuvent être utilisés pour des traitements en oncologie.

Le cancer reste une maladie pour laquelle les traitements existants sont clairement insuffisants. Certaines protéines kinases jouent un rôle important dans de nombreux cancers. L'inhibition de telles protéines kinases est potentiellement importante dans la chimiothérapie de cancers notamment pour supprimer la croissance ou la survie de tumeurs. La présente invention concerne donc l'identification de nouveaux produits qui inhibent de telles protéines kinases.

Les protéines kinases participent aux événements de signalisation qui contrôlent l'activation, la croissance et la différentiation des cellules en réponse, soit à des médiateurs extracellulaires, soit à des changements de l'environnement. En générale, ces kinases appartiennent à deux groupes: celles qui phosphorylent préférentiellement les résidus sérines et/ou thréonine et celles qui phosphorylent préférentiellement les résidus tyrosines [S.K.Hanks and T.Hunter, FASEB. J., 1995, 9, pages 576-596]. Les sérine/thréonine kinases sont par exemple, les isoformes des protéines kinases C [A.C.Newton, J. Biol. Chem., 1995, 270, pages 28495-28498] et un groupe de kinases dépendantes des cyclines, comme cdc2 [J.Pines, Trends in Biochemical Sciences, 1995, 18, pages 195-197]. Les tyrosine kinases comprennent les récepteurs aux facteurs de croissance comme le récepteur au facteur de croissance épidermal (EGF) [S.Iwashita and M.Kobayashi, Cellular Signalling, 1992, 4, pages 123-132], et des kinases cytosoliques comme p56tck, p59fYn, ZAP-70 et les kinases csk [C. Chan et. al., Ann. Rev. Immunol., 1994, 12, pages 555-592].

Des niveaux anormalement élevés d'activité protéine kinase ont été impliqués dans de nombreuses maladies, résultant de fonctions cellulaires anormales. Ceci peut provenir soit directement soit indirectement, d'un disfonctionnement dans les mécanismes de contrôle de l'activité kinase, lié par exemple à une mutation, une sur-expression ou une activation inapproprié de l'enzyme, ou par une sur- ou sous-production de cytokines ou des facteurs de croissance, également impliqués dans la transduction des signaux en amont ou en aval des kinases. Dans tous ces cas, une inhibition sélective de l'action des kinases laisse espérer un effet bénéfique.

Le récepteur de type 1 pour l'insulin-like growth factor (IGF-I-R) est un récepteur transmembranaire à activité tyrosine kinase qui se lie en premier lieu à l'IGFI mais aussi à l'IGFII et à l'insuline avec une plus faible affinité. La liaison de l'IGF1 à son récepteur entraîne une oligomérisation du récepteur, l'activation de la tyrosine kinase, l'autophosphorylation intermoléculaire et la phosphorylation de substrats cellulaires (principaux substrats : IRS1 et Shc). Le récepteur activé par son ligand induit une activité mitogènique dans les cellules normales. Cependant IGF-I-R joue un rôle important dans la croissance dite anormale.

Plusieurs rapports cliniques soulignent le rôle important de la voie IGF-I dans le développement des cancers humains :
IGF-I-R est souvent trouvé sur-exprimé dans de nombreux types tumoraux (sein, colon, poumon, sarcome, myélome, ...) et sa présence est souvent associée à un phénotype plus agressif.

De fortes concentrations d'IGF1 circulant sont fortement corrélées à un risque de cancer dé la prostate, poumon et sein.

De plus, il a été largement documenté que IGF-I-R est nécessaire à l'établissement et au maintien du phénotype transformé in vitro comme in vivo[Baserga R, Exp. Cell. Res., 1999, 253, pages 1-6]. L'activité kinase d'IGF-I-R est essentielle à l'activité de transformation de plusieurs oncogènes: EGFR, PDGFR, l'antigène grand T du virus SV40, Ras activé, Raf, et v-Src. L'expression d'IGF-I-R dans des fibroblastes normaux induit un phénotype néoplasique, qui peut ensuite entraîner la formation de tumeur in vivo. L'expression d'IGF-I-R joue un rôle important dans la croissance indépendante du substrat. IGF-I-R a également été montré comme un protecteur dans l'apoptose induite par chimiothérapie-, radiation-, et l'apoptose induite par des cytokines. De plus, l'inhibition d'IGF-I-R endogène par un dominant négatif, la formation de triple hélice ou l'expression d'un antisens provoque une suppression de l'activité transformante *in vitro* et la diminution de la croissance de tumeurs dans les modèles animaux.
La présente invention a pour objet les produits de formule (I): 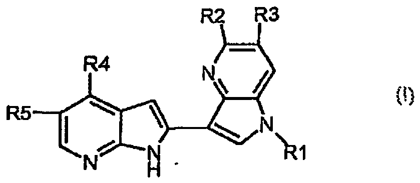 choisis parmi les composés suivants:
3 - -(4-chloro-1H-pyrrolo[2,3-b]-pyridin-2-yl)-5,6-diméthoxy-1-methyl-1H-pyrrolo[3,2-b]-pyridine ;
3 - (4-Chloro-1H-pyrrolo[2,3-b]pyridin-2-yl)-5,6-diméthoxy-1-(2-morpholin-4-yléthyl)-1H-pyrrolo[3,2-b]pyridine ;
3-(4-Chloro-1H-pyrrolo[2,3-b]pyridin-2-yl)-5,6-dimethoxy-1-[2-(4-méthylpipérazin-1-yl) éthyl]-1H-pyrrolo[3,2-b]pyridine ;
3-(5-fluoro-1H-pyrrolo[2,3-b]pyridin-2-yl)5,6-diméthoxy-1-(2-morpholin-4-yl-éthyl)-1H-pyrrolo[3,2-b]pyridine ;
3-(4-chloro-5-fluoro-1H-pyrrolo[2,3-b] pyridin-2-yl)-5,6-diméthoxy-1-(2-morpholin-4-yl-éthyl)-1H-pyrrolo[3,2-b]pyridine ;
3-(4-Chloro-5-fluoro-1H-pyrrolo[2,3-b]pyridin-2-yl)-5,6-diméthoxy-1-[2-(4-méthylpipérazin-1-yl)-éthyl]-1H-pyrrolo[3,2-b]pyridine;
3-[4-Chloro-5-fluoro-1H-pyrrolo[2,3-b] pyridin-2-yl]-5,6-diméthoxy-1-(3-pipéridin-1-yl-propyl)-1H-pyrrolo[3,2-b] pyridine ;
1-{3-[3-(4-Chloro-5-fluoro-1H-pyrrolo[2,3-b]pyridin-2-yl)-5,6-diméthoxy-pyrrolo[3,2-b] pyridin-1-yl]-propyl}-pipéridin-4-ol;
C-(1-{3-[3-(4-chloro-5-fluoro-1H-pyrrolo[2,3-b]pyridin-2-yl)-5,6-diméthoxy-pyrrolo[3,2-b] pyridin-1-yl]-propyl}-pipéridin-4-yl)-méthylamine;
3-(4-Chloro-5-fluoro-1H-pyrrolo[2,3-b]pyridin-2-yl)-5,6-diméthoxy-1-[3-(4-méthyl-pipérazin-1-yl)-propyl]-1H-pyrrolo[3,2-b]pyridine;
C-(1-{2[3-(4-Chloro-1H-pyrrolo[2,3-b]pyridin-2-yl)-5,6-diméthoxy-pyrrolo[3,2-b]pyridin-1-yl]-éthyl}-pipéridin-4-yl)-méthylamine;
[3-(4-chloro-1H-pyrrolo[2,3-b]pyridin-2-yl)-5,6-diméthoxy-pyrrolo[3,2-b]pyridin-1-yl]-acétate de potassium ;
2-[3-(4-Chloro-1H-pyrrolo[2,3-b]pyridin-2-yl)-5,6-diméthoxy-pyrrolo[3,2-b]pyridin-1-yl]-1-(4-méthyl-pipérazin-1-yl)-éthanone ;
2-[3-(4-Chloro-1H-pyrrolo[2,3-b]pyridin-2-yl)-5,6-diméthoxy-pyrrolo[3,2-b]pyridin-1-yl]-1-(4-hydroxy-pipéridin-1-yl)-éthanone ;
2-[3-(4-Chloro-1H-pyrrolo[2,3-b]pyridin-2-yl)-5,6-diméthoxy-pyrrolo[3,2-b]pyridin-1-yl]-1-morpholin-4-yl-éthanone;
2-(1-{3-[3-(4-Chloro-1H-pyrrolo[2,3-b] pyridin-2-yl)-5,6-diméthoxy-pyrrolo[3,2-b]pyridin-1-yl]-propyl}-pipéridin-4-yl)-éthanol ;
1-{3-[3-(4-Chloro-1H-pyrrolo[2,3-b]pyridin-2-yl)-5,6-diméthoxy-pyrrolo[3,2-b]pyridin-1-yl] -propyl}-pipéridin-4-ol;
{3-[3-(4-Chloro-1H-pyrrolo[2,3-b]pyridin-2-yl)-5,6-diméthoxy-pyrrolo[3,2-b]pyridin-1-yl]-propyl}-diéthyl-amine ;
3-(4-Chloro-1H-pyrrolo[2,3-b]pyridin-2-yl)-5,6-diméthoxy-1-[3-(4-méthyl-perhydro-1,4-diazépin-1-yl)-propyl]-1H-pyrrolo[3,2-b]pyridine ;
3-(4-Chloro-1H-pyrrolo[2,3-b]pyridin-2-yl)-5,6-diméthoxy-1-(3-pipéridin-1-yl-propyl)1H-pyrrolo[3,2-b]pyridine ;
2-(5,6-Diméthoxy-1H-pyrrolo[3,2-b]pyridin-3-yl)-1H-pyrrolo[2,3-b]pyridine-4-carbonitrile ;
2-(5,6-Diméthoxy-1-méthyl-1H-pyrrolo[3,2-b]pyridin-3-yl)-1H-pyrrolo[2,3-b]pyridine-4-carbonitrile ;
2-[3-(4-Chloro-1H-pyrrolo[2,3-b]pyridin-2-yl)-5,6-diméthoxy-pyrrolo[3,2-b]pyridin-1-yl]-N-methoxy-N-méthyl-acétamide ;
2-(5,6-diméthoxy-1-méthyl-1H-pyrrolo[3,2-b]pyridin-3-yl)-1H-pyrrolo[2,3-b]pyridin-4-carbaldéhyde ;
4-{[2-(5,6-Diméthoxy-1-méthyl-1H-pyrrolo[3,2-b]pyridin-3-yl)-1H-Pyrrolo[2,3-b]pyridin-4-ylméthyl]-amino}-phénol ;
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Le terme patient désigne les êtres humains mais aussi les autres mammifères.

Lorsque les produits de formule (I) comportent un radical amino salifiable par un acide il est bien entendu que ces sels d'acides font également partie de l'invention. On peut citer les sels fournis avec les acides chlorhydrique ou méthanesulfonique par exemple.

Les sels d'addition avec les acides minéraux ou organi-ques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituant fixés, soit sur des doubles liaisons, soit sur des cycles, que l'on appelle souvent isomérie géométrique ou isomérie cis-trans. Le terme stéréoisomère est utilisé dans la présente demande dans son sens le plus large et concerne donc l'ensemble des composés indiqués ci-dessus. Parmi les produits préférés de l'invention, on peut citer particulièrement les produits de formule (I) dont les noms suivent :
- le 3-(4-chloro-1H-pyrrolo[2,3-*b*]pyridin-2-yl)-5,6-diméthoxy-1-méthyl-1H-pyrrolo[3,2-*b*]pyridine, (30)
- le 3-(4-Chloro-1H-pyrrolo[2,3-b]pyridin-2-yl)-5,6-diméthoxy-1-(2-morpholin-4-yléthyl)-1H-pyrrolo[3,2-b]-pyridine, (34)
- le 3-(4-Chloro-1H-pyrrolo[2,3-b]pyridin-2-yl)-5,6-diméthoxy-1-[2-(4-méthylpipérazin-1-yl)éthyl]-1H-pyrrolo-[3,2-b]pyridine, (36)
- le 3-(5-fluoro-1H-pyrrolo[2,3-b]pyridin-2-yl)-5,6-diméthoxy-1-(2-morpholin-4-yl-éthyl)-1H-pyrrolo[3,2-b]pyridine
- le 3-(4-chloro-5-fluoro-1H-pyrrolo[2,3-b]pyridin-2-yl)-5,6-diméthoxy-1-(2-morpholin-4-yl-éthyl)-1H-pyrrolo[3,2-b]pyridine
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

Parmi les produits préférés de l'invention, on peut citer particulièrement les produits de formule (I) dont les noms suivent :
le 3-(4-Chloro-5-fluoro-1H-pyrrolo[2,3-b]pyridin-2-yl)-5,6-diméthoxy-1-[2-(4-méthylpipérazin-1-yl)-éthyl]-1H-pyrrolo[3,2-b]pyridine
- le 3-[4-Chloro-5-fluoro-1H-pyrrolo[2,3-b]pyridin-2-yl]-5,6-diméthoxy-1-(3-pipéridin-1-yl-propyl)-1H-pyrrolo[3,2-b]pyridine
- le 1-{3-[3-(4-Chloro-5-fluoro-1H-pyrrolo[2,3-b]pyridin-2-yl)-5,6-diméthoxy-pyrrolo[3,2-b]pyridin-1-yl]-propyl}-pipéridin-4-ol
- le C-(1-{2-[3-(4-Chloro-1H-pyrrolo[2,3-b]pyridin-2-yl)-5,6-diméthoxy-pyrrolo[3,2-b]pyridin-1-yl]-éthyl}-pipéridin-4-yl)-méthylamine
- le 2-[3-(4-Chloro-1H-pyrrolo[2,3-b]pyridin-2-yl)-5,6-diméthoxy-pyrrolo[3,2-b]pyridin-1-yl]-1-(4-méthyl-pipérazin-1-yl)-éthanone
- le {3-[3-(4-Chloro-1H-pyrrolo[2,3-b]pyridin-2-yl)-5,6-diméthoxy-pyrrolo[3,2-b]pyridin-1-yl]-propyl}-diéthylamine
- le 3-(4-Chloro-1H-pyrrolo[2,3-b]pyridin-2-yl)-5,6-diméthoxy-1-[3-(4-méthyl-perhydro-1,4-diazépin-1-yl)-propyl]-1H-pyrrolo[3,2-b]pyridine
- le 2-(5,6-Diméthoxy-1-méthyl-1H-pyrrolo[3,2-b]pyridin-3-yl)-1H-pyrrolo[2,3-b]pyridine-4-carbonitrile Exemple 32
- le 2-[3-(4-Chloro-1H-pyrrolo[2,3-b]pyridin-2-yl)-5,6-diméthoxy-pyrrolo[3,2-b]pyridin-1-yl]-N-méthoxy-N-méthyl-acétamide
- le 4-{[2-(5,6-Diméthoxy-1-méthyl-1H-pyrrolo[3,2-b]pyridin-3-yl)-1H-pyrrolo[2,3-b]pyridin-4-ylméthyl]-amino}-phénol
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

Les produits objet de la présente invention sont doués de propriétés pharmacologiques intéressantes: on a constaté qu'ils possédaient notamment des propriétés inhibitrices de protéines kinases.

Parmi ces protéines kinases, on cite notamment IGF1R.

Des tests illustrent l'activité inhibitrice de produits de la présente invention vis-à-via de telles protéines kinases.

Ces propriétés rendent donc les produits de formule générale (I) de la présente invention utilisables comme médicaments pour le traitement de tumeurs malignes.

Les produits de formule (I) peuvent également être utilisés dans le domaine vétérinaire.

L'invention a donc pour objet l'application, à titre de médicaments, des produits de formule générale (I) pharmaceutiquement acceptables.

L'invention a particulièrement pour objet l'application à titre de médicaments, des produits de formule (I) décrits en exemples dans la partie expérimentale et notamment ceux dont les noms suivent :
- le 3-(4-chloro-1H-pyrrolo[2,3-b]pyridin-2-yl)-5,6-diméthoxy-1-méthyl-1H-pyrrolo[3,2-b]pyridine ;
- le 3-(4-Chloro-1H-pyrrolo[2,3-b]pyridin-2-yl)-5,6-diméthoxy-1-(2-morpholin-4-yléthyl)-1H-pyrrolo[3,2-b]-pyridine ;
- le 3-(4-Chloro-1H-pyrrolo[2,3-b]pyridin-2-yl)-5,6-diméthoxy-1-[2-(4-méthylpipérazin-1-yl)éthyl]-1H-pyrrolo-[3,2-b]pyridine ;
- le 3-(5-fluoro-1H-pyrrolo[2,3-b]pyridin-2-yl)-5,6-diméthoxy-1-(2-morpholin-4-yl-éthyl)-1H-pyrrolo[3,2-b]pyridine) ;
- le 3-(4-chloro-5-fluoro-1H-pyrrolo[2,3-b]pyridin-2-yl)-5,6-diméthoxy-1-(2-morpholin-4-yl-éthyl)-1H-pyrrolo[3,2-b]pyridine ;
lesdits produits de formule (I) étant sous toutes les formes isomères possibles racémiques, énantiomères et diastéréo-isoméres, ainsi que les sels d'addition avec les acides minéraux et organiques ou avec les bases minérales et organiques pharmaceutiquement acceptables desdits produits de formule (I).

L'invention a notamment pour objet l'application à titre de médicaments, des produits de formule (I) dont les noms suivent :
- le 3-(4-Chloro-5-fluoro-1H-pyrrolo[2,3-b]pyridin-2-yl)-5,6-diméthoxy-1-[2-(4-méthylpiperazin-1-yl)-éthyl]-1H-pyxrolo[3,2-b]pyridine
- le 3-[4-Chloro-5-fluoro-1H-pyrrolo[2,3-b]pyridin-2-yl]-5,6-diméthoxy-1-(3-pipéridin-1-yl-propyl)-1H-pyrrolo[3,2-b]pyridine
- le 1-{3-[3-(4-Chloro-5-fluoro-1H-pyrrolo[2,3-b]pyridin-2-yl)-5,6-diméthoxy-pyrrolo-[3,2-b]pyridin-1-yl]-propyl)-pipéridin-4-ol
- le C-(1-{2-[3-(4-Chloro-1H-pyrrolo[2,3-b]pyridin-2-yl)-5,6-diméthoxy-pyrrolo[3,2-b]pyridin-1-yl-éthyl}-pipéridin-4-yl)-méthylamine
- le 2-[3-(4-Chloro-1H-pyrrolo[2,3-b]pyridin-2-yl)-5,6-diméthoxy-pyrrolo[3,2-b]pyridin-1-yl]-1-(4-méthylpipérazin-1-yl)-éthanone
- le {3-[3-(4-Chloro-1H-pyrrolo[2,3-b]pyridin-2-yl)-5,6-diméthoxy-pyrrolo[3,2-b]pyridin-1-yl]-propyl}-diéthylamine
- le 3-(4-Chloro-1H-pyrrolo[2,3-b]pyridin-2-yl)-5,6-diméthoxy-1-[3-(4-méthyl-perhydro-1,4-diazépin-1-yl)-propyl]-1H-pyrrolo[3,2-b]pyridine
- le 2-(5,6-Diméthoxy-1-méthyl-1H-pyrrolo[3,2-b]pyridin-3-yl)-1H-pyrrolo[2,3-b]pyridine-4-carbonitrile Exemple 32
- le 2-[3-(4-Chloro-1H-pyrrolo[2,3-b]pyridin-2-yl)-5,6-diméthoxy-pyrrolo[3,2-b]pyridin-1-yl]-N-méthoxy-N-méthyl-acétamide
- le 4-{[2-(5,6-Diméthoxy-1-méthyl-1H-pyrrolo[3,2-b]pyridin-3-yl)-1H-pyrrolo[2,3-b]pyridin-4-ylméthyl] amino}-phénol
lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

Les produits peuvent être administrés par voie parentérale, buccale, perlinguale, rectale ou topique.

L'invention a aussi pour objet les compositions pharmaceutiques, caractérisées en ce qu'elles renferment, à titre de principe actif, un au moins des médicaments de formule générale (I)

Ces compositions peuvent être présentées sous forme de solutions ou de suspensions injectables, de comprimés, de comprimés enrobés, de capsules, de sirops, de suppositoires, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

La présente invention concerne ainsi l'utilisation de produits de formule (I) tels que définis ci -dessus ou de sels pharmaceutiquement acceptables desdits produits de formule (I) pour la préparation de médicaments destinés à inhiber l'activité de protéines kinases et notamment d'une protéine kinase.

La présente invention concerne ainsi 1' utilisation de produits de formule (I) telle que définie ci-dessus ou de sels pharmaceutiquement acceptables desdits produits de formule (I) dans laquelle la protéine kinase est une protéine tyrosine kinase.

La présente invention concerne ainsi l'utilisation de produits de formule (I) telle que définie ci-dessus ou de sels pharmaceutiquement acceptables desdits produits de formule (I) dans laquelle la protéine kinase est choisie dans le groupe suivant:
IGF1, Raf, EGF, PDGF, VEGF, Tie2, KDR, Flt1-3, FAK, Src, Abl, cKit, cdk1-9, Auroral-2, cdc7, Akt, Pdk, S6K, Jnk,
IR, FLK-1, FGFR1, FGFR2, FGFR3, FGFR4, FGFR5, PLK, Pyk2 CDK7, CDK2 et EGFR.

La présente invention concerne ainsi l'utilisation de produits de formule (I) telle que définie ci-dessus ou de sels pharmaceutiquement acceptables desdits produits de formule (I) dans laquelle la protéine kinase est plus particulièrement choisie dans le groupe suivant :
IGF1, cdc7, Auroral-2, Src, Jnk, FAK, KDR, IR, Tie2, CDK7, CDK2 et EGFR.

La présente invention concerne ainsi particulièrement l'utilisation de produits de formule (I) telle que définie ci-dessus ou de sels pharmaceutiquement acceptables desdits produits de formule (I) dans laquelle la protéine kinase est IGF1R.

La présente invention concerne également l'utilisation de produits de formule (I) telle que définies ci-dessus ou de sels pharmaceutiquement acceptables desdits produits de formule (I) dans laquelle la protéine kinase est dans une culture cellulaire et également cette utilisation dans un mammifère.

La présente invention concerne ainsi l'utilisation de produits de formule (I) telle que définie ci-dessus ou de sels pharmaceutiquement acceptables desdits produits de formule (I) pour la préparation d'un médicament destiné à prévenir ou traiter une maladie caractérisée par le dérèglement de l'activité d'une protéine kinase et notamment une telle maladie chez un mammifère.

La présente invention concerne l'utilisation de produits de formule (I) telle que définie ci-dessus ou de sels pharmaceutiquement acceptable desdits produits de formule (I) pour la préparation d'un médicament destiné à prévenir ou traiter une maladie appartenant au groupe suivant: désordres de la prolifération de vaisseaux sanguins, désordres fibrotiques, désordres de la prolifération de cellules mésangiales, l'acromégalie, désordres métaboliques, allergies, asthme, maladie de crohn's, thromboses, maladies du système nerveux, rétinopathies, psoriasis, arthrite rhumatoïde, diabètes, dégénération musculaire, gériatrie, dégénerescence musculaire due à l'âge, maladies en oncologie, cancers.

La présente invention concerne ainsi l'utilisation de produits de formule (I) telle que définie ci-dessus ou de sels pharmaceutiquement acceptables desdits produits de formule (I) pour la préparation d'un médicament destiné à traiter des maladies en oncologie.

La présente invention concerne particulièrement l'utilisation de produits de formule (I) telle que définie ci-dessus ou de sels pharmaceutiquement acceptables desdits produits de formule (I) pour la préparation d'un médicament destiné à traiter des cancers.

Parmi ces cancers, la présente invention s'intéresse tout particulièrement au traitement des tumeurs solides et au traitement de cancers résistants aux agents cytotoxiques

Parmi ces cancers, la présente invention concerne tout particulièrement le traitement du cancer du sein, de l'estomac, du colon, des poumons, des ovaires, de l'utérus, du cerveau, du rein, du larynx, du système lymphatique, de la thyroïde, du tractus uro-génital, du tractus incluant vésicule et prostate, du cancer des os, du pancréas, les mélanomes.

La présente invention s'intéresse encore plus particulièrement au traitement du cancer du sein, du colon et des poumons.

La présente invention concerne aussi l'utilisation de produits de formule (I) telle que définie ci-dessus ou de sels pharmaceutiquement acceptables desdits produits de formule (I) pour la préparation d'un médicament destiné à la chimiothérapie de cancers.

A titre de médicaments selon la présente invention destinés à la chimiothérapie de cancers, les produits de formule (I) selon la présente invention peuvent être utilisés seuls ou en association avec chimiothérapie ou radiothérapie ou alternativement en association avec d'autres agents thérapeutiques.

La présente invention concerne ainsi notamment les compositions pharmaceutiques telles que définies ci-dessus contenant en plus, des principes actifs d'autres médicaments de chimiothérapie contre le cancer.

De tels agents thérapeutiques peuvent être des agents anti-tumoraux utilisés communément.

Comme exemples d'inhibiteurs connus de protéines kinases, on peut citer notamment la butyrolactone, le flavopiridol, la 2- (2-hydroxyéthylamino)-6-benzylamino-9-méthylpurine, l'olomucine, le Glivec ainsi que l'Iressa.

Les produits de formule (I) selon la présente invention peuvent ainsi également être avantageusement utilisés en combinaison avec des agents anti-prolifératifs : à titre d'exemples de tels agents anti-prolifératifs mais sans toutefois se limiter à cette liste, on peut citer les inhibiteurs d'aromatase, les antiestrogènes, les inhibiteurs de topoisomérase I, les inhibiteurs de topoisomérase II, les agents actifs sur les microtubules, les agents d'alkylation, les inhibiteurs d'histone désacétylase, les inhibiteurs de farnésyl transférase, les inhibiteurs de COX-2, les inhibiteurs de MMP, les inhibiteurs de mTOR , les antimétabolites antinéoplastique, les composés du platine, les composés faisant décroître l'activité des protéines kinases et également les composés anti-angiogéniques, les agonistes de la gonadoréline, les anti-androgènes, les bengamides, les biphophonates et le trastuzumab.

On peut citer ainsi à titre d'exemples, des agents anti-microtubules comme les taxoides, vinka-alkaloides, des agents d'alkylation tels que cyclophosphamide, des agents DNA-intercalant comme le cis-platinum, des agents interactifs sur topoisomérase comme la camptothécine et dérivés, les anthracyclines comme l'adriamycine, des antimétabolites comme le 5-fluorouracile et dérivés et analogues.

La présente invention concerne donc des produits de formule (I) comme inhibiteurs de protéines kinases, lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

La présente invention concerne particulièrement des produits de formule (I) tels que définis ci-dessus comme inhibiteurs de IGF1R.

Les produits de départ de synthèse des produits de formule (I) selon la présente invention peuvent être connus, décrits dans les documents de l'homme du métier, disponibles dans le commerce ou peuvent être préparés selon des méthodes usuelles connues de l'homme de métier ou encore selon les méthodes décrites dans les schémas ci-joints.

La partie expérimentale ci-après donne plus particulièrement une illustration des exemples 4 à 8, 10 à 12, 15, 16, 19, 21 à 27, 29 à 33, 38, 49 et 67 de la présente demande

Schéma 4 : Synthèse de la 3-(4-chloro-1H-pyrrolo[2,3-b]-pyridin-2-yl)-5,6-diméthoxy-1-méthyl-1H-pyrrolo[3,2-b]-pyridine, (exemple 4 ou produit 30)

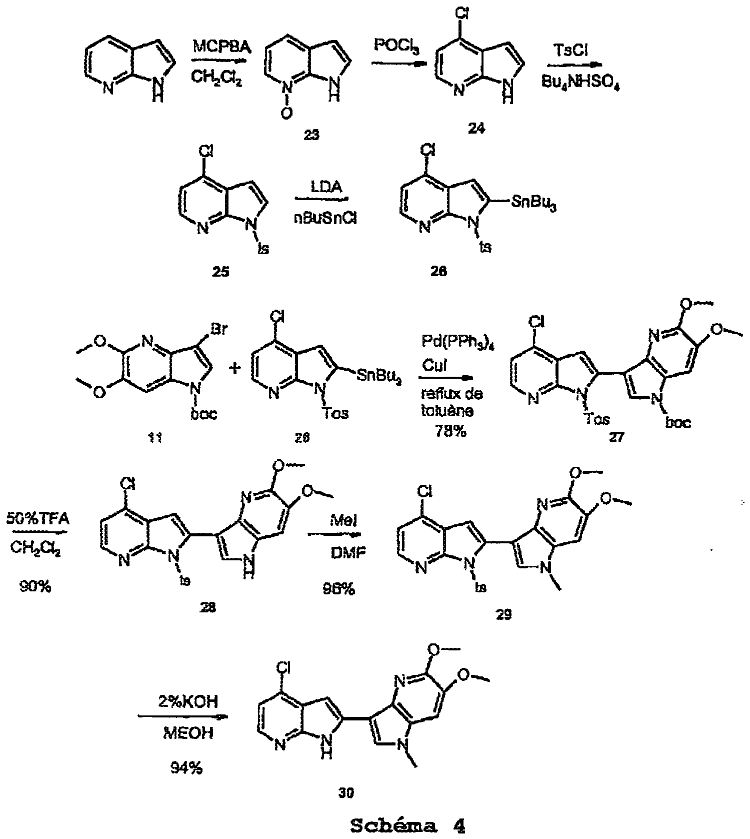

Schéma 5 : synthèse du 3-(4-Chloro-1H-pyrrolo[2,3-b]pyridin-2-yl)-5,6-diméthoxy-1-(2-morpholin-4-yléthyl)-1H-pyrrolo[3,2-b]pyridine, 34. (exemple 5 ou produit 34)

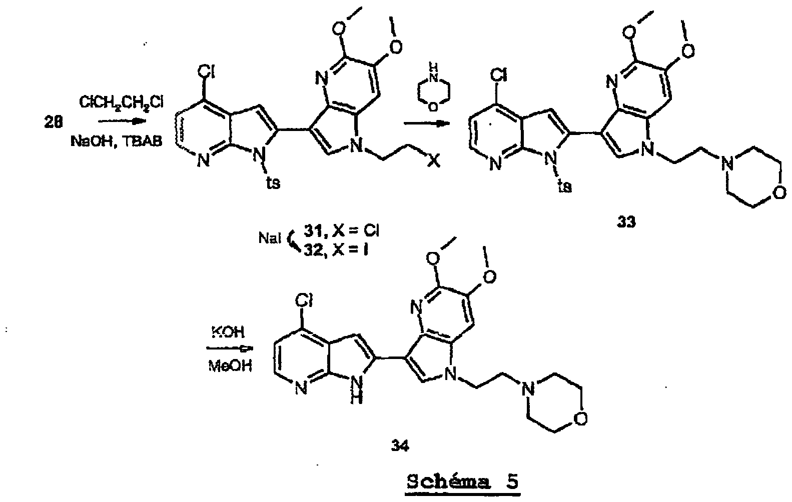

Schéma 6 : synthèse du_3-(4-Chloro-1H-pyrrolo[2,3-b]pyridin-2-yl)-5,6-diméthoxy-1-[2-(4-méthylpipérazin-1-yl)éthyl]-1H-pyrrolo[3,2-b]pyridine, 36. (exemple 6 ou produit 36)

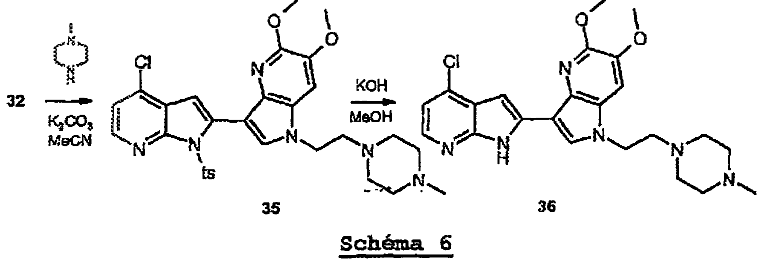

Dans les préparations qui suivent des exemples 1 à 8 pour la spectrométrie de Masse, les spectres ont été réalisés en impact électronique (IE) et/ou en désorption-ionisation chimique (IC) (gaz réactant :ammoniac) sur un spectromètre Finnigan SSQ 7000.

Méthodes de synthèse générales pour la préparation des exemples 1 à 8. Sauf indication contraire, les produits ont été obtenus auprès de fournisseurs industriels et employés sans autre purification. Toutes les réactions ont été menées sous une atmosphère inerte avec des réactifs et des solvants anhydres. La chromatographie éclair a été réalisée sur un Isco CombiFlash Companion^{™} au moyen de cartouches de silice garnies au préalable Advantage FlashReady^{™} et des systèmes de solvants décrits. La chromatographie sur couche mince a été réalisée avec des plaques 60F-254 enduites de gel de silice de 0,25 mm (EM) et visualisées avec de la vapeur d'iode, de la lumière UV ou un réactif colorant tel qu'une solution de KMnO₄.

Les spectres infrarouges (IR) ont été enregistrés dans un spectromètre IR-TF Nexus 670 (Nicolet) avec des échantillons préparés de la manière indiquée, et sont exprimés en nombres d'onde (cm⁻¹). Les spectres de RMN-¹H. ont été enregistrés dans des spectromètres Varian Gemini et/ou Mercury 300, Unity 400 ou Unity plus et/ou Inova à 500 MHz, les déplacements chimiques (δ) étant exprimés en ppm par rapport au tétraméthylsilane (0,0 ppm) ou au chloroforme (CDCl₃, 7,26 ppm) comme référence. Les spectres de RMN-¹³C ont été enregistrés dans un instrument Varian Unity (fréquence 13C de 100,57 MHz), les déplacements chimiques (δ) étant exprimés en ppm par rapport à CDCl₃ (77,0 ppm), sauf indication contraire. Les spectres de masse (SM) ont été obtenus dans un spectromère de masse Finnigan MAT modèle TSQ 700 par ionisation chimique à 120 eV avec du méthane (CI, 120 eV). La chromatographie liquide - spectrométrie de masse (CL-SM) a été réalisée sur un LCT Micromass doté d'une interface avec un gestionnaire de liquide Gilson 215. L'analyse par' spectrométrie de masse haute résolution (spectres de masse exacts) a été réalisée en mode ESI à une résolution massique de 10 000 au moyen d'un spectromètre de masse Micromass QTOF. Les valeurs exactes des masses ont été déterminées pour les ions moléculaires protonés (M + 1) où M désigne l'ion moléculaire.

Les exemples dont la préparation suit, qu'il s'agisse de préparations de produits ou de préparations de compositions pharmaceutiques, illustrent la présente invention sans toutefois la limiter.

### Exemple 4 : Synthèse de la 3-(4-chloro-1H-pyrrolo[2,3-b]-pyridin-2-yl)-5,6-diméthoxy-1-méthyl-1H-pyrrolo[3,2-b]-pyridine, 30.

### stade 4 (a) : 1H-Pyrrolo[2,3-b]pyridin-7-ol, 23.

A une solution de MCPBA (77%, 21,52 g, 96,0 mmol) dans CH₂Cl₂ (140 mL) agitée à 0°C, sous N₂, on ajoute du 7-azaindole (5,67 g, 48,0 mmol) en solution dans CH₂Cl₂ (47 mL), goutte à goutte en l'espace de 35 min. On laisse le milieu réactionnel se réchauffer à la température ambiante et on l'agite pendant 1,5 h. Le mélange réactionnel est ensuite filtré et concentré sous vide. L'huile résiduelle est traitée avec Et₂O (50 mL) et le précipité solide filtré, mis en suspension dans H₂O (50 mL) et alcalinisé avec une solution aqueuse saturée de K₂CO₃ jusqu'à pH 10. La phase aqueuse est ensuite extraite avec CHCl₃ (7x), séchée (MgSO₄) et concentrée, pour donner **2**3 (3,18 g, 49%) sous forme d'huile marron: RMN-¹H (DMSO-d₆) □ 12,5 (s large, 1H), 8,14 (s large, 1H), 7,70 (d, 1H, J = 7,5 Hz), 7,45 (d, 1H, J = 3,0 Hz), 7,06 (t, 1H, J = 6,9 Hz), 6,58 (d, 1H, J = 3,0 Hz), m/z obs. = 135,1 (M+1), 118,1 (M-16).

### Stade 4(b) : 4-Chloro-1H-pyrrolo[2,3-b]pyridin, 24.

De l'azaindole **23** (3,18 g, 0,0237 mmol) est porté au reflux dans POCl₃ (50 mL) pendant 8 h, après quoi le POCl₃ est éliminé du milieu réactionnel par distillation sous vide et le résidu traité avec H₂O et NaHCO₃ saturé. Le précipité est recueilli par filtration et séché à l'air. Une purification par chromatographie éclair conduit à **24** (0,84 g, 22%) sous forme de solide cristallin blanc: RMN-¹H (CDCl₃) □ 9,5 (s large, 1H), 8,18 (d, 1H, J = 5,4 Hz), 7,36 (dd, 1H, J = 3,5, 2,3 Hz), 7,11 (d, 1H, J = 5,1 Hz), 6,61 (dd, 1H, J = 3,6, 1,8 Hz), CCM (EtOAc à 40%/heptane) Rf = 0,10, m/z obs. = 152,86 (M+1), 154,86, 193,93 (M+Na).

### Stade 4(c) : 4-Chloro-1-(toluène-4-sulfonyl)-1H-pyrrolo[2,3-b]pyridine, 25.

A une solution de 4-chloroazaindole **24** (0,84 g, 5,50 mmol), de TsC1 (1,154 g, 6,053 mmol) et de Bu₄NHSO₄ (16 µL, 50% dans H₂O) dans du toluène (16 mL), on ajoute NaOH (3,6 g, 90,2 mmol) sous forme de solution dans H₂O (14 mL). Après 2,5 h d'agitation du milieu réactionnel à la température ambiante, le milieu réactionnel à deux phases est ensuite extrait avec EtOAc (2x), séché (MgSO₄) et concentré sous vide. Une chromatographie éclair donne 25 (1,33 g, 78%) sous forme de solide blanc: RMN-¹H (CDCl₃) □ 8,28 (d, 1H, J = 5,1 Hz), 8,04 (dd, 2H, J = 8,5, 2,0 Hz), 7,75 (d, 2H, J = 4,0 Hz), 7,26 (d, 1H, Je 8,4 Hz), 7,17 (d, 1H, J = 5,1 Hz), 6,67 (d, 1H, J = 4,2 Hz), 2,37 (s, 3H), CCM (EtOAc à 40%/heptane) Rf = 0,50, m/z obs..= 307 (M+1), 308,9.

### Stade 4(d) : 4-Chloro-1-(toluène-4-sulfonyl)-2-tributylstannanyl-1H-pyrrolo[2,3-b]pyridine, 26.

A l'azaindole **25** (0,3 g, 0,98 mmol) dans du THF (15 mL), agité à -78°C, sous N₂, on ajoute LDA (1,5 équiv., 0,74 mL, 1,47 mmol) goutte à goutte en l'espace de 5 min. Après 0,5 h d'agitation du milieu réactionnel, on ajoute goutte à goutte Bu₃SnCl (475 mg, 400 µL, 1,47 mmol) et on agite le milieu réactionnel pendant encore 0,5 h. On laisse ensuite le milieu réactionnel se réchauffer à la température ambiante, on stoppe la réaction avec H₂O, on dilue avec EtOAc (200 mL), on lave avec de la saumure, on sèche (MgSO₄) et on concentre. Le résidu est soumis à une chromatographie éclair pour donner **26** (0,46 g, 79%) sous forme d'huile incolore: RMN-¹H (CDCl₃) □ 8,15 (d, 1H, J = 5,1 Hz), 7,97 (dd, 2H, J = 8,0, 1,5 Hz), 7,27 (d, 2H, J = 8,4 Hz), 7,09 (d, 1H, J = 5,2 Hz), 6,75 (s, 1H), 2,36 (s, 3H), 1,65-1,20 (m, 12H), 0,93 (t, 9H), CCM (EtOAc à 40%/heptane) Rf = 0,65.

### Stade 4 (e) : Ester t-butylique d'acide 3-[4-chloro-1-(toluène-4-sulfonyl) -1H-pyrrolo [2,3-b]pyridin-2-yl] -5, 6-diméthoxypyrrolo[3,2-b]pyridine-1-carboxylique, 27.

Dans une solution de diméthoxyazaindole **11** (0,25 g, 0,70 mmol) et d'azaindole **26** (0,458 g, 0,77 mmol) dans du toluène (15 mL), on fait barboter N₂ pendant 15 min, puis on ajoute CuI (14 mg, 0,07 mmol) et Pd(PPh₃)₄ (81 mg, 0,07 mmol) et on agite le mélange avec le courant de N₂ pendant 0,5 h, puis on chauffe à 120°C sous N₂ pendant 20 h. Une purification du mélange réactionnel sur SiO₂ (chromatographie éclair, cartouche de 40 g, élution avec EtOAc à 10-30%/heptane) donne le bisazaindole 27 (0,28 g, 68%) sous forme de solide blanc: RMN-¹H (CDCl₃) □ 8,29 (d, 1H, J = 5,4 Hz), 7,86 (s apparent chevauchant d, 3H), 7,15 (m, 4H), 6,94 (s, 1H), 3,97 (s, 6H), 2,32 (s, 3H), 1,71 (s, 9H), CCM (EtOAc à 40%/heptane) Rf = 0,40.

### Stade 4 (f) : 3-[4-Chloro-1-(toluènee-4-sulfontyl)-1H-pyrrolo [2,3-b]pyridin-2-yl]-5,6-diméthoxy-1H-pyrrolo[3,2-b]pyridine, 28.

On dissout du bisazaindole **27** (0,28 g, 0,48 mmol) dans TFA à 50%/CH₂Cl₂ (8,0 mL) et on agite à la température ambiante pendant 5 h. Le milieu réactionnel est concentré sous vide, et le résidu dissous dans EtOAc (100 mL), lavé avec NaHCO₃ saturé (3x), séché (MgSO₄) et concentré, pour donner **28** (0,222 g, 96%) sous forme de solide amorphe: CCM (EtOAc à 40%/heptane) Rf = 0,05.

### stade 4 (g) : 3-[4-Chloro-1-(toluène-4-sulfonyl)-1H-pyrrolo[2,3-b]pyridin-2-yl]-5,6-diméthoxy-1-méthyl-1H-pyrrolo[3,2-b]pyridine, 29.

Au bisazaindole **28** (37,3 mg, 0,0772 mol) agité dans du DMF (5,0 mL) à 0°C, on ajoute KOH (5,0 équiv., 21,6 mg) suivi de MeI (5,0 équiv., 55,0 mg, 24 µL) et on agite le milieu réactionnel à pendant 1,5 h. On laisse ensuite le milieu réactionnel se réchauffer à la température ambiante, on le dilue avec EtOAc, on le lave avec NaHCO₃ saturé, on le sèche (MgSO₄) et on le concentre, pour obtenir 29 (40 mg, 100%) sous forme de solide amorphe: CCM (EtOAc à 40%/heptane) Rf = 0,10.

### stade 4(h) : 3-(4-Chloro-1H-pyrrolo [2,3-b]pyridin-2-yl)-5,6-dimikthoxy-1-méthyl-1H-pyrrolo[3,2-b]pyridine, 30.

On porte au reflux du bisazaindole **29** (40 mg, 0,081 mmol) dans KOH à 4%/MeOH (10 mL) pendant 5,5 h et on concentre ensuite la réaction sous vide. Une purification sur SiO₂ (MeOH à 2-4%/CH₂Cl₂) conduit à 30 (13 mg, 47,3%) sous forme de solide blanc: RMN-¹H (CDCl₃) □ 10,92 (s large, 1H), 8,07 (d, 1H, J = 5,3 Hz), 7,49 (s, 1H), 7,05 (s apparent chevauchant d, 2H, J = 5,2 Hz), 6,62 (s, 1H), 4,22 (s, 3H), 3,96 (s, 3H), 3,81 (s, 3H), CCM (EtOAc à 40%/heptane), Rf = 0,40, m/z obs. = 343,3 (M+1), 345,3.

### Exemle 5 : synthèse du 3- (4-Chloro-1H-pyrrolo [2,3-b)pyridin-2-yl) -5,6-diméthoxy-1- (2-morpholin-4-yléthyl)-1H-pyrrolo[3,2-b]pyridine, 34.

### Stade 5 (a) : 1-(2-Chloroéthyl)-3-[4-chloro-1-(toluène-4-sulfonyl)-1H-pyrrolo{2,3-b]pyridin-2-yl]-5,6-diméthoxy13-pyrrolo[3,2-b]pyridine, 31.

On chauffe un mélange de bisazaindole 28 (180 mg, 0,373 mmol), de 1, 2-dichloroéthane (2,0 mL), de NaOH 9N (2,0 mL) et de bromure de tétrabutylammonium (16 mg), sous agitation, à 50°C pendant 16 h. On laisse ensuite refroidir le milieu réactionnel à la température ambiante, on l'extrait avec CH₂Cl₂ (3x), on le sèche (Na₂SO₄) et on le concentre sous vide. Une purification sur SiO₂ par chromatographie éclair donne **31** (110 mg, 54%) sous forme de solide jaune: RMN-¹H (CDCl₃) □ 8,40 (d, 1H, J = 5,4 Hz), 7,76 (d, 1H, J = 5,2 Hz), 7,70 (d, 2H, J = 8,4 Hz), 7,59 (s, 1H), 7,15 (m, 2H), 7,06 (d, 2H, J = 8,4 Hz), 7,02 (s, 1H), 4, 51 (t, 2H, J = 6,4 Hz), 4,06 (s, 3H), 4,00 (s, 3H), 3,90 (t, 2H, J = 6,4 Hz), 2,30 (s, 3H), CCM (EtOAc à 40%/heptane) Rf = 0,20.

### stade 5 (b) : 3-[4-Chloro-1-(toluène-4-sulfonyl)-1H-pyrrolo[2,3-b]pyridin-2-yl-1-(2-iodoéthyl)-5,6-diméthoxy-19-pyrrolo[3,2-b]pyridine, 32.

On dissout du chlorobisazaindole **31** (110 mg, 0,202 mmol) et NaI (33,5 mg, 0,222 mmol) dans de la 2-butanone (10 mL) et on porte au reflux pendant 24 h. Le milieu réactionnel est ensuite concentré sous vide, le résidu dissous dans EtOAc, lavé avec de la saumure, séché (Na₂SO₄) et concentré, pour donner 32 (124 mg, 96,6%) sous forme de solide jaune: RMN-¹H (CDCl₃) □ 8,25 (d, 1H, J = 5,4 Hz), 7,71 (d, 2H, J = 8,4 Hz), 7,65 (s, 1H), 7,10 (m, 5H), 4,53 (t, 2H, J = 6,4 Hz), 4,03 (s, 3H), 3,99 (s, 3H), 3,50 (t, 2H, J = 6,4 Hz), 2,31 (s, 3H), CCM (EtOAc à 40%/heptane) Rf = 0,20, m/z obs. = 636,9 (M+1), 638,9, 639,94.

### Stade 5 (c) : 3-[4-Chloro-1-(toluéne-4-sulfonyl)-1H-Pyrrolo[2, 3-b]pyridin-2-yl]-5,6-diméthoxy-1-(2-morpholin-4-yléthyl)-1H-pyrrolo[3,2-b]pyridine, 33.

A du bisazaindole **32** (54 mg, 0,0848 mmol) dans CH₃CN (6,0 mL), on ajoute de la morpholine (3,5 équiv., 25,9 mg, 26,1 µmol, 0,30 mmol) et K₂CO₃ (11,7 mg, 0,085 mmol). Après avoir chauffé à 60°C pendant 30 h, on concentre le milieu réactionnel sous vide et on soumet le résidu de produit brut à une chromatographie éclair sur SiO₂ (MeOH à 2-4%/CH₂Cl₂) pour obtenir **33** (40 mg, 79,2%) sous forme de solide ivoire : RMN-¹H (CDCl₃) □ 8,26 (d, 1H, J = 5,4 Hz), 7,72 (d, 2H, J = 8,4 Hz) , 7,66 (s, 1H), 7,16 (m, 3H), 7,09 (d, 2H, J = 8,1 Hz), 4,29 (t, 2H, J = 6,6 Hz) , 4,05 (s, 3H), 4,01 (s, 3H), 3,73 (m, 4H), 2,86 (t, 2H, J = 6,6 Hz), 2,55 (m, 4H), 2,33 (s, 3H), CCM (MeOH à 4%/CH₂Cl₂, 0,25% de NH₃) Rf = 0,25, m/z obs. = 597,56 (M+1) .

### Stade 5(d) : 3-(4-Chloro-1H-pyrrolo[2,3-b]pyridin-2-yl)-5, 6-diméthoxy-1-(2-morpholin-4-yléthyl) -1H-pyrrolo[3,2-b]pyridine, 34.

On traite du bisazaindole **33** (40 mg, 0,067 mmol) avec KOH à 3%/MeOH (10 mL) et on porte au reflux pendant 16 h. Le milieu réactionnel est concentré sous vide et le résidu partagé entre EtOAc et NaHCO₃ saturé. La phase organique est lavée avec NaHCO₃ saturé, séchée (Na₂SO₄) et soumise à une chromatographie éclair sur SiO₂ (MeOH à 2-4%/CH₂Cl₂) pour donner **34** (19,0 mg, 64,2%) sous forme de solide blanc: RMN-¹H (CDCl₃) □ 10, 94 (s large, 1H), 8, 07 (d, 1H, J = 5,0 Hz), 7,62 (s, 1H), 7,14 (s, 1H), 7,05 (d, 1H, J = 5,3 Hz), 6,66 (d, 1H, J = 1,9 Hz), 4,23 (s chevauchant t, 5H, J = 6,4 Hz), 3,97 (s, 3H), 3,70 (t, 4H, J = 4,6 Hz), 2,79 (t, 2H, J = 6,4 Hz), 2, 50 (t, 4H, J = 4,6 Hz), CCM (MeOH à 4%/CH₂Cl₂, 0,25% de NH₃) Rf = 0,25, m/z obs. 442,1 (M+1).

### Exemple 6: synthèse du 3-(4-chloro-1H-pyrrolo[2,3-b]pyridin-2-yl)-5,6-diméthoxy-1-[2-(4-méthylpiperazin-1-yl)éthyl]-1H-pyrrolo[3,2-b]pyridine, 36.

**stade 6(a): 3-[4-Chloro-1-(toluéne-4-sulfonyl)-1H-pyrrolo[2,3-b]pyridin-2-yl]-5,6-diméthozy-1-[2-(4-méthylpipérazin-1-yl)éthyl]-1H-Pyrrolo[3,2-b]pyridine, 35.** A du bisazaindole **32** (64 mg, 0,10 mmol) dans CH₃CN (6,0 mL), on ajoute de la 1-méthylpipérazine (3,5 équiv., 35,2 mg, 39,1 µmol, 0,352 mmol) et K₂CO₃ (13,8 mg, 0,10 mmol). Après chauffage à 60°C pendant 48 h, le milieu réactionnel est concentré sous vide et le résidu de produit brut soumis à une chromatographie éclair sur SiO₂ (MeOH à 2-4%/CH₂Cl₂/heptane) pour donner **35** (40 mg, 64,3%) sous forme de solide blanc: RMN-¹H (CDCl₃) □ 8,23 (d, 1H, J = 5, 4 Hz), 7, 68 (d, 2H, J = 8,1 Hz), 7,61 (s, 1H), 7,10 (m, 5H), 4,25 (t, 2H, J = 6,6 Hz), 4,01 (s, 3H), 3,97 (s, 3H), 2,83 (t, 2H, J = 6,9 Hz), 2,57-2,46 (m large, 8H), 2,28 (s, 3H), CCM (MeOH à 10%/CH₂Cl₂, 0,25% de NH₃) Rf = 0,45, m/z obs. = 609,04 (M+1).

**Stade 6(b) : 3-(4-Chloro-1H-pyrrolo[2,3-b]pyridin-2-yl)-5,6-diméthoxy-1-[2-(4-méthylpipérazin-1-yl)éthyl]-1H-pyrrolo[3,2-b]pyridine, 36**. On porte au reflux du bisazaindole **35** (40 mg, 0,066 mmol) dans KOH à 3%/MeOH (10 mL) pendant 16 h, puis on concentre sous vide. Le résidu est dissous dans EtOAc, lavé avec NaHCO₃ saturé, séché (Na₂SO₄) et soumis à une chromatographie éclair sur SiO₂ (MeOH à 4%/CH₂Cl₂, 0,25% de NH₃) pour donner **36** (18 mg, 60,2%) sous forme de solide blanc: RMN-¹H (CDCl₃.□ 10,94 (s large, 1H), 8,07 (d, 1H, J = 5,2Hz) , 7,61 (s, 1H), 7,15 (s, 1H), 7,05 (d, 1H, J= 5,2 Hz), 6,65 (d, 1H, J = 2 Hz), 4,23 (s chevauchant t, 5H, J = 6,5 Hz), 3,97 (s, 3H), 2,79 (t, 2H, J = 6,5 Hz), 2,55-2,45 (m large, 8H), 2,29 (s, 3H), CCM (MeOH à 10%/CH₂Cl₂, 0,25% de NH₃) Rf = 0,35, m/z obs. = 455,11 (M+1).

### EXEMPLE 7: 3-(5-fluoro-1H-pyrrolo[2,3-b]pyridin-2-yl)-5,6-diméthoxy-1-(2-morpholin-4-yl-éthyl)-1H-pyrrolo[3,2-b]pyridine

**Stade 7 (a): 5-fluoro-3-iodo-pyridin-2-ylamineLa 5-**fluoro-3-iodo-pyridin-2-ylamine peut être préparée de la manière suivante:
Un mélange de 9,9g de 5-fluoro-pyridin-3-ylamine et de 21,85g de N-iodosuccinimide dans 400cm³ d'acide acétique est agité environ 6 heures à une température voisine de 70°C . Après concentration à sec sous pression réduite (13kPa), le résidu est repris par 250cm³ d'eau; le pH est amené à environ 8 par addition de d'hydrogénocarbonate de sodium. La phase aqueuse est extraite par cinq fois 150cm³ de dichlorométhane. Les phases organiques réunies sont lavées par trois fois 100cm³ d'eau, puis par cinq fois 50cm³ d'une solution aqueuse à 10% de thiosulfate de sodium, séchées sur sulfate de sodium, filtrées et concentrées à sec sous pression réduite (13kPa). On obtient ainsi, après flash chromatographie sur colonne de silice (éluant: dichlorométhane), 11g de 5-fluoro-3-iodo-pyridin-2-ylamine sous forme d'un solide dont les caractéristiques sont les suivantes:
   - point de fusion: fondant à 76°C (banc Kofler) - Spectre RMN 1H (300MHz )- δ en ppm- dans le DMSO-d6: 5,98 (s large, 2H); de 7,93 à 7,98 (m, 2H).
   - Spectre de masse: SM-EI: 238(+)=M(+)

### Stade 7 (b): 5-fluoro-3-triméthylsilanyléthynyl-pyridin-2-ylamine

Le 5-fluoro-3-triméthylsilanyléthynyl-pyridin-2-ylamine peut être de la manière suivante:

A une solution de 14g de 5-fluoro-3-iodo-pyridin-2-ylamine dans 440cm³ de diméthylformamide, dégazée à l'argon, sont ajoutés 12,47cm³ d'éthynyl-triméthyl-silane, 2,24g d'iodure de cuivre, 2,74g de chlorure de lithium, 41,33cm³ de triéthylamine et 2,15g de chlorure de [1,1'-bis(diphénylphosphino)ferrocène]palladium(II). La solution obtenue est portée à une température voisine de 55°C pendant environ 5 heures. Après retour à une température voisine de 20°C, le mélange est concentré sous pression réduite (13kPa) ; le résidu est repris par 300cm³ d'eau, extrait par trois fois 100cm³ d'acétate d'éthyle. Les phases organiques réunies sont lavées par trois fois 100cm³ d'eau, séchées sur sulfate de magnésium, filtrées et concentrées sous pression réduite (13kPa). On obtient ainsi, après flash chromatographie sur colonne de silice (éluant: dichlorométhane), 7,91g de 5-fluoro-3-triméthylsilanyléthynyl-pyridin-2-ylamine sous forme d'un solide dont les caractéristiques sont les suivantes:
- point de fusion: fondant à 65°C (banc Köfler)
- spectre de masse: IE m/z=208 M⁺ ; m/z=193 (M- CH₃)⁺ pic de base

### Stade 7 (c): 5-fluoro-1H-pyrrolo[2,3-b]pyridine

Le 5-fluoro-1H-pyrrolo[2,3-b]pyridine peut être préparé de la manière suivante:
Un mélange de 3,80g de 5-fluoro-3-triméthylsilanyléthynyl-pyridin-2-ylamine et de 3,40g de tert-butylate de potassium dans 100cm³ de 1-méthyl-pyrrolidin-2-one est porté au voisinage de 130°C pendant environ 4 heures. Après retour à une température voisine de 20°C, le mélange est versé sur 1000cm³ d'une solution aqueuse saturée en chlorure de sodium et extrait par 5 fois 250cm³ d'oxyde de diéthyle. Les phases organiques sont rassemblées, lavées par 5 fois 100cm3 d'une solution aqueuse saturée en chlorure de sodium, séchées sur sulfate de magnésium, filtrées et concentrées à sec sous pression réduite (13kPa). On obtient 2,35g de 5-fluoro-1H-pyrrolo[2,3-b]pyridine sous forme d'un solide dont les caractéristiques sont les suivantes:
   - point de fusion: fondant à 110°C (Banc-Köfler)
   - spectre de masse: IE m/z=136 M^{+.} pic de base m/z=109 (M - HCN)⁺

### Stade 7 (d): 5-fluoro-1-(toluène-4-sulfonyl)-1H-pyrrolo[2,3-b]pyridine

Le 5-fluoro-1-(toluène-4-sulfonyl)-1H-pyrrolo[2,3-b]pyridine peut être préparé de la manière suivante:

Un mélange de 2,30g de 5-fluoro-1H-pyrrolo[2,3-b]pyridine, de 3,54g de chlorure de 4-méthyl-benzènesulfonyle, de 7,56g d'hydroxyde de sodium dissous dans 55cm³ d'eau, de 0,115g d'hydrogénosulfate de tétrabutylammonium dans 125cm³ de toluène est agité pendant environ 24 heures au voisinage de 20°C. Le mélange est dilué par 500cm³ d'acétate d'éthyle ; la phase organique est lavée par trois fois 200cm³ d'eau, séchée sur sulfate de magnésium, filtrée et concentrée à sec sous pression réduite (13kPa). Le résidu est purifié par flash chromatographie sur colonne de silice [éluant: dichlorométhane]. On obtient 3,85g de 5-fluoro-1-(toluène-4-sulfonyl)-1H-pyrrolo[2,3-b]pyridine sous forme d'un solide dont les caractéristiques sont les suivantes:
- point de fusion: fondant à 160°C (Banc-Köfler)
- spectre de masse: IE m/z=290 M^{+.} ; m/z=226 (M - SO₂)^{+.}; m/z=155 C₇H₇O₂S⁺; m/z=91C₇H₇⁺ pic de base

### stade 7 (a) : 5-fluoro-1-(toluène-4-sulfonyl)-2-tributylstannanyl-1H-pyrrolo[2,3-b]pyridine

Le 5-fluoro-1-(toluène-4-sulfonyl)-2-tributylstannanyl-1H-pyrrolo[2,3-b]pyridine peut être préparé comme dans l'exemple 1 stade 1(k) mais à partir de 2g de 5-fluor-1-(toluéne-4-sulfonyl)-1H-pyrrolo[2,3-b]pyridine, de 2,58g de chlorure de tributylétain, de 4,95cm³ d'une solution 1,6M de n-butyllithium dans l'hexane dans 100cm³ de tétrahydrofurane. On obtient ainsi, après flash chromatographie sur colonne de silice [éluant: cyclohexane/acétate d'éthyle (95/5 en volumes)], 2,70g de 5-fluoro-1-(toluène-4-sulfonyl)-2-tributylstannanyl-1H-pyrrolo[2,3-b]pyridine sous forme d'une huile dont les caractéristiques sont les suivantes:
- Rf CCM silice [éluant: cyclohexane/acétate d'éthyle/triéthylamine (95/5/1 en volumes)] = 0,41
- Spectre RMN 1H (400MHz )- δ en ppm- dans le DMSO-d6: 0,89 (t, J = 7,0 Hz, 9H) ; de 1,15 à 1,70 (m, 18H) ; 2,37 (s, 3H) ; 6,87 (m, 1H) ; 7,43 (d large, J = 8,5 Hz, 2H) ; 7,86 (d large, J = 8,5 Hz, 2H) ; 7,90 (dd, J = 3,0 et 9,0 Hz, 1H) ; 8,26 (dd, J = 1,5 et 3,0 Hz, 1H)
- Spectre de Masse: SM-EI: 579(+)=M(+)

### Stade 7 (f) : ester ter-butylique de l'acide 3-[5-fluoro-1-(toluéné-4-sulfonyl)-1H-pyrrolo[2,3-b]pyridin-2-yl]-5,6-diméthoxy-pyrrolo[3,2-b]pyridine-1-carboxylic

L'ester ter-butylique de l'acide 3-[5-fluoro-1-(toluène-4-sulfonyl)-1H-pyrrolo[2,3-b]pyridin-2-yl]-5,6-diméthoxypyrrolo[3,2-b]pyridine-1-carboxylic peut être préparé comme dans l'exemple 1 stade 1(1)mais à partir de 1,30g de 5-fluoro-1-(toluène-4-sulfonyl)-2-tributylstannanyl-1H-pyrrolo[2,3-b]pyridine, de 0,73g de l'ester ter-butylique de l'acide 3-bromo-5,6-diméthoxy-pyrrolo[3,2-b]pyridine-1-carboxylique, de 0,039g d'iodure de cuivre, de 0,236g de palladium tétrakis(triphénylphosphine) dans 60cm³ de toluène. On obtient ainsi, après flash chromatographie sur colonne de silice [éluant: cyclohexane/acétate d'éthyle (70/30 en volumes)], 0,48g de l'ester ter-butylique de l'acide 3-[5-fluoro-1-(toluène-4-sulfonyl)-1H-pyrrolo[2,3-b]pyridin-2-yl]-5,6-diméthoxy-pyrrolo[3,2-b]pyridine-1-carboxylic sous forme d'une meringue dont les caractéristiques sont les suivantes:
- Rf CCM silice [éluant: cyclohexane/acétate d'éthyle (50/50 en volumes)]= 0,39
- Spectre RMN 1H (300MHz )- δ en ppm- dans le DMSO-d6: 1,71 (s, 9H) ; 2,33 (s, 3H) ; 3,87 (s, 3H) ; 3,92 (s, 3H) ; 7,03 (s, 1H) ; 7,34 (d large, J = 8,5 Hz, 2H) ; 7,76 (d large, J = 8,5 Hz, 2H) ; 7,91 (s, 1H) ; 7,96 (dd, J = 2,5 et 8,5 Hz, 1H) ; 8,02 (s, 1H) ; 8,36 (dd, J = 1,5 et 2,5 Hz, 1H).

### Stade 7 (g) : 3-[5-fluoro-1-(toluène-4-sulfonyl)-1H-pyrrolo[2,3-b]pyridin-2-yl]-5,6-diméthoxy-1H-pyrrolo[3,2-b]pyridine

Le 3-[5-fluoro-1-(toluène-4-sulfonyl)-1H-pyrrolo(2,3-b]pyridin-2-yl]-5,6-diméthoxy-1H-pyrrolo[3,2-b]pyridine peut être préparé comme dans l'exemple 1 stade 1(m) mais à partir de 0,78g de l'ester ter-butylique de l'acide 3-[5-fluoro-1-(toluène-4-sulfonyl)-1H-pyrrolo(2,3-b]pyridin-2-yl]-5,6-diméthoxy-pyrrolo[3,2-b]pyridine-1-carboxylique, de 10cm³ d'acide trifluoroacétique dans 45cm³ de dichlorométhane. On obtient ainsi, après flash chromatographie sur colonne de silice [éluant: dichlorométhane/acétate d'éthyle (95/5 en volumes)], 0,40g de 3-[5-fluoro-1-(toluène-4-sulfonyl)-1H-pyrrolo[2,3-b]pyridin-2-yl]-5,6-diméthoxy-1H-pyrrolo[3,2-b]pyridine sous forme d'un solide dont les caractéristiques sont les suivantes:
- Rf CCM silice [éluant: dichlorométhane/acétate d'éthyle (90/10 en volumes)]= 0,29
- Spectre RMN 1H (300MHz )- δ en ppm- dans le DMSO-d6: 2,31 (s, 3H) ; 3,86 (s, 3H) ; 3,88 (s, 3H) ; 6,98 (s, 1H); 7,28 (d large, J = 8,5 Hz, 2H) ; 7,40 (s large, 1H) ; 7,67 (m, 3H) ; 7,86 (dd, J = 3,0 et 8,5 Hz, 1H); 8,28 (dd, J = 1,5 et 3,0 Hz, 1H); 11,4 (s large, 1H)
- Spectre de Masse: SM-EI: 466(+)=M(+)

### Stade 7 (h): 1-(2-chloro-éthyl)-3-[5-fluoro-1-(toluène-4-sulfonyl)-1H-pyrrolo[2,3-b]pyridin-2-yl]-5,6-diméthoxy-1H-pyrsolo[3,2-b]pyridine

Le 1-(2-chloro-éthyl)-3-[5-fluoro-1-(toluène-4-sulfonyl)-1H-pyrrolo[2,3-b]pyridin-2-yl]-5,6-diméthoxy-1H-pyrrolo[3,2-b)pyridine peut être préparé comme dans l'exemple 2 stade 2(a)mais à partir de 0,40g de 3-[5-fluoro-1-(toluène-4-sulfonyl)-1H-pyrrolo[2,3-b]pyridin-2-yl]-5,6-diméthoxy-1H-pyrrolo[3,2-b]pyridine, de 0,0055g de bromure de tétrabutylammonium, de 0,32g d'hydroxyde de potassium et 0,276g de carbonate de potassium dans 30cm3 de 1,2-dichloro-éthane. On obtient ainsi 0,45g de 1-(2-chloro-éthyl)-3-[5-fluoro-1-(toluène-4-sulfonyl)-1H-pyrrolo[2,3-b]pyridin-2-yl]-5,6-diméthoxy-1H-pyrrolo[3,2-b]pyridine sous forme d'une meringue dont les caractéristiques sont les suivantes:
- Rf CCM silice [éluant: dichlorométhane/acétate d'éthyle (90/10 en volumes)]= 0,68
- Spectre RMN 1H (300MHz)- □ en ppm -dans le DMSO-d6: 2,31 (s, 3H) ; 3,87 (s, 3H) ; 3,90 (s, 3H) ; 4,08 t, J = 6,0 Hz, 2H) ; 4,65 (t, J = 6,0 Hz, 2H) ; 6,97 s, 1H) ; 7,27 (d large, J = 8,5 Hz, 2H) ; 7,67 (d large, J = 8,5 Hz, 2H) ; 7,71 (s, 1H) ; 7,77 (s, 1H) ; 7,87 (dd, J = 3,0 et 9,0 Hz, 1H) ; 8, 30 (dd, J = 1,5 et 3,0 Hz, 1H) .
- Spectre de Masse: SM-EI: 528 (+) /...=M (+) /... (1Cl présent)

### Stade 7 (i) : 3-[-fluoro-1-(toluène-4-sulfonyl)-1H-pyrrolo[2,3-b]pyridin-2-yl]-1-(2-iodo-éthyl)-5,6-diméthoxy-1H-pyrrolo[3,2-b]pyridine

Le 3-[5-fluoro-1-(toluène-4-sulfonyl)-1H-pyrrolo[2,3-b]pyridin-2-yl]-1-(2-iodo-éthyl)-5,6-diméthoxy-1H-pyrrolo[3,2-b]pyridine peut être préparé comme dans l'exemple 2 stade 2(b)mais à partir de 0,45g de 1-(2-chloro-éthyl)-3-[5-fluoro-1-(toluène-4-sulfonyl)-1H-pyrrolo[2,3-b]pyridin-2-yl]-5,6-diméthoxy-1H-pyrrolo[3,2-b]pyridine et de 0,19g d'iodure de sodium dans 40cm³ de 2-butanone. On obtient ainsi 0,53g de 3-[5-fluor-1-(toluène-4-sulfonyl)-1H-pyrrolo[2,3-b]pyridin-2-yl]-1-(2-iodo-éthyl)-5,6-diméthoxy-1H-pyrrolo[3,2-b]pyridine sous forme d'une meringue dont les caractéristiques sont les suivantes:
- Rf CCM silice [éluant: dichlorométhane/acétate d'éthyle (95/5 en volumes)]= 0,48
- Spectre RMN 1H (300MHz ) - δ en ppm- dans le DMSO-d6: 2,31 (s, 3H) ; 3,68 (t, J = 6,5 Hz, 2H) ; 3,87 (s, 3H) ; 3,90 (s, 3H) ; 4,65 (t, J = 6,5 Hz, 2H) ; 6,96 (s, 1H) ; 7,28 (d large, J = 8,5 Hz, 2H) ; 7,68 (d large, J = 8,5 Hz, 2H) ; 7,71 (s, 1H) ; 7,79 (s, 1H) ; 7,88 (dd large, J = 3,0 et 8,5 Hz, 1H) ; 8,31 (m, 1H).
- Spectre de Masse: SM-EI: 620(+)=M(+)

### Stade 7 (j) : 3-[5-fluoro-1-(toluéne-4-sulfonyl)-1H-pyrrolo[2,3-b]pyridin-2-yl]-5,6-diméthoxy-1-(2-morpholin-4-yl-éthyl)-1H-pyrrolo[3,2-b]pyridine

Le 3-[5-fluoro-1-(toluène-4-sulfonyl)-1H-pyrrolo[2,3-b]pyridin-2-yl]-5,6-diméthoxy-1-(2-morpholin-4-yl-éthyl)-1H-pyrrolo[3,2-b]pyridine peut être préparé comme dans l'exemple 2 stade 2(c) mais à partir de 0,53g de 3-[5-fluoro-1-(toluène-4-sulfonyl)-1H-pyrrolo[2,3-b]pyridin-2-yl]-1-(2-iodo-éthyl)-5,6-diméthoxy-1H-pyrrolo[3,2-b]pyridine, de 0,13g de carbonate de potassium et de 0,150g de morpholine dans 60cm³ d'acétonitrile. On obtient ainsi 0,47g de 3-[5-fluoro-1-(toluène-4-sulfonyl)-1H-pyrrolo[2,3-b]pyridin-2-yl]-5,6-diméthoxy-1-(2-morpholin-4-yl-éthyl)-1H-pyrrolo[3,2-b]pyridine sous forme d'une résine dont les caractéristiques sont les suivantes:
- Rf CCM silice [éluant: dichlorométhane/méthanol (95/5 en volume)]= 0,52
- Spectre RMN 1H (400MHz)- δ en ppm- dans le DMSO-d6: 2,30 (s, 3H) ; 2,49 (m partiellement masqué, 4H) ; 2,76 (t, J = 6,0 Hz, 2H) ; 3,57 (m, 4H) ; 3,87 (s, 3H) ; 3,89 (s, 3H) ; 4,38 (t, J = 6,0 Hz, 2H) ; 6,97 (s large, 1H) ; 7,24 (d large, J = 8,5 Hz, 2H) ; 7,63 (m, 3H) ; 7,76 (s, 1H) ; 7,87 (dd large, J = 3,0 et 9,5 Hz, 1H) ; 8,29 (dd, J = 2,0 et 3,0 Hz, 1H).

### Stade 7 (k) : 3-(5-fluoro-1H-pyrrolo [2,3-b]pyridin-2-yl)-5,6-diméthoxy-1-(2-morpholin-4-yl-éthyl)-1H-pyrrolo[3,2-b]pyridine

Le 3-(5-fluoro-1H-pyrrolo[2,3-b]pyridin-2-yl)-5,6-diméthoxy-1-(2-morpholin-4-yl-éthyl)-1H-pyrrolo[3,2-b]pyridine peut être préparé comme dans l'exemple 2 stade 2(d):Mais à partir de 0,470g de 3-[5-fluoro-1-(toluène-9-sulfonyl)-1H-pyrrolo[2,3-b]pyridin-2-yl]-5,6-diméthoxy-1-(2-morpholin-4-yl-éthyl)-1H-pyrrolo[3,2-b]pyridine, de 1g d'hydroxyde de potassium dans 100cm³ de méthanol. On obtient ainsi, après flash chromatographie sur colonne de silice [éluant: dichlorométhane/méthanol (98/2 en volumes)], 0,180g de 3-(5-fluoro-1H-pyrrolo[2,3-b]pyridin-2-yl)-5,6-diméthoxy-1-(2-morpholin-4-yl-éthyl)-1H-pyrrolo[3,2-b]pyridine sous forme d'un solide dont les caractéristiques sont les suivantes:
- point de fusion: fondant à 218°C (Banc Kofler)
- Spectre RMN 1H (400MHz)- δ en ppm- dans le DMSO-d6: 2,47 (m, 4H) ; 2, 71 (t, J = 6,5 Hz, 2H) ; 3, 58 (m, 4H) ; 3,89 (s, 3H) ; 4,07 (s, 3H) ; 4,33 (t, J = 6,5 Hz, 2H) ; 7,22 (d, J = 2,0 Hz, 1H) ; 7,65 (s, 1H) ; 7,75 (dd, J = 2,5 et 9,5 Hz, 1H) ; 8,03 (s, 1H) ; 8,06 (dd, J = 1,5 et 2,5 Hz, 1H) ; 11,85 (s large, 1H) .
- Spectre de Masse: SM-EI:425(+)=M(+)

### Exemple 8: 3-(4-chloro-5-fluoro-1H-pyrrolo[2,3-b]pyridin-2-yl)-5,6-diméthoxy-1-(2-morpholin-4-yl-éthyl)-1H-pyrrolo[3,2-b]pyridine

### stade 8(a): 5-fluoro-1H-pyrrolo[2,3-b]pyridine 7-oxyde

Le 5-fluoro-1*H*-pyrrolo[2,3-b]pyridine 7-oxyde peut être obtenu comme dans l'exemple 4 stade 4(a) mais à partir de 2,7g de 5-fluoro-1*H*-pyrrolo[2,3-b]pyridine, de 6,22g d'acide 3-chloro-per benzoïque dans 70cm³ de diméthoxyéthane. On obtient ainsi, après flash chromatographie sur colonne de silice [éluant: dichlorométhane/méthanol (95/5 en volumes)], 1,70g de 5-fluoro-1*H*-pyrrolo[2,3-b]pyridine 7-oxyde sous forme d'une poudre dont les caractéristiques sont les suivantes:
- point de fusion: fondant à 178°C (Banc Kofler)
- spectre IR: KBr 3128; 3085; 2919; 2863; 2734; 2629; 2406; 1588; 1507; 1349; 1256; 1206; 1129; 1077; 990; 804; 723; 670 et 466 cm⁻¹

### Stade 8(b): 4-chloro-5-fluoro-1H-pyrrolo[2,3-b]pyridine

Le 4-chloro-5-fluoro-1H-pyrrolo[2,3-b]pyridine peut être obtenu comme dans l'exemple 4 stade 4(b) mais à partir de 1,7g de 5-fluoro-1H-pyrrolo[2,3-b]pyridine 7-oxyde dans 10 cm³ d'oxychlorure de phosphore. On obtient ainsi 1,3g de 4-chloro-5-fluoro-1*H*-pyrrolo[2,3-b]pyridine pyridine sous forme d'un solide dont les caractéristiques sont les suivantes:
- Rf CCM silice [éluant: dichlorométhane/méthanol (98/2 en volumes)]= 0,19
- Spectre RMN 1H (300MHz )- δ en ppm- dans le DMSO-d6: 6,56 (m, 1H) ; 7,70 (m, 1H) ; 8,35 (d, J = 2,5 Hz, 1H) ; 12,15 (m large, 1H)
- Spectre de Masse: SM-EI:170(+)=M(+)

### Stade 8c: 4-chloro-5-fluoro-1-(toluène-4-sulfonyl)-1H-pyrrolo[2,3-b]pyridines

Le 4-chloro-5-fluoro-1-(toluène-4-sulfonyl)-1H-pyrrolo[2,3-b]pyridine peut être préparé comme dans l'exemple 7 stade 7(d) mais à partir de 1,30g de 4-chloro-5-fluoro-1H-pyrrolo[2,3-b]pyridine, de 1,60g de chlorure de 4-méthyl-benzènesulfonyle, de 3,40g d'hydroxyde de sodium dissous dans 16cm³ d'eau, de 0,052g d'hydrogénosulfate de tétrabutylammonium dans 200cm³ de toluène. On obtient ainsi, après flash chromatographie sur colonne de silice [éluant: dichlorométhane], 1,90g dé 4-chloro-5-fluoro-1-(toluène-4-sulfonyl)-1H-pyrrolo[2,3-b) pyridine sous forme d'une poudre dont les caractéristiques sont les suivantes:
- point de fusion: fondant à 125°C (Banc Kofler)
- Spectre RMN 1H (300MHz)- δ en ppm- dans le DMSO-d6: 2,36 (s, 3H) ; 6,93 (d, J = 3,0 Hz, 1H) ; 7,44 (d large, J = 8,0 Hz, 2H) ; 7,99 (d large, J = 8,0 Hz, 2H) ; 8,12 (d, J = 3,0 Hz, 1H) ; 8,52 (d, J = 2,5 Hz, 1H)
- Spectre de Masse: SM-EI:324(+)=M(+)

### Stade 8(d): 4-chloro-5-fluoro-1-(toluéne-4-sulfonyl)-2-tributylstannanyl-1H-pyrrolo[2,3-b]pyridine

Le 4-chloro-5-fluoro-1-(toluène-4-sulfonyl)-2-tributylstannanyl-1H-pyrrolo[2,3-b]pyridine peut être préparé comme dans l'exemple 7 stade 7(e) mais à partir de 3,8g de 4-chloro-5-fluoro-1-(toluène-4-sulfonyl)-1H-pyrrolo[2,3-b]pyridine, de 3,65cm³ de chlorure de tributylétain, de 7,92cm³ d'une solution 1,6M de ter-butyllithium dans l'hexane dans 100cm³ de tétrahydrofurane. On obtient ainsi, après flash chromatographie sur colonne de silice [éluant: cyclohexane/acétate d'éthyle (95/5 en volumes)], 5,66g de 4-chloro-5-fluoro-1-(toluène-4-sulfonyl)-2-tributylstannanyl-1H-pyrrolo[2,3-b]pyridine sous forme d'une huile dont les caractéristiques sont les suivantes:
- Rf CCM silice [éluant: cyclohexane/acétate d'éthyle (75/25 en volumes)]= 0,73
- Spectre RMN 1H (300MHz )- δ en ppm- dans le DMSO-d6: 0,88 (t, J = 7,5 Hz, 9H) : de 1,10 à 1,70 (m, 18H) ; 2,35 (s, 3H) ; 6,82 (m, 1H) ; 7,43 (d large, J = 8,0 Hz, 2H) ; 7,84 (d large, J = 8,0 Hz, 2H) ; 8,39 (d, J = 2,5 Hz, 1H)
- Spectre de Masse: SM-EI:614(+)=M₍+)

### Stade 8 (e) : Ester ter-butylique de l'acide 3-[4-chloro-5-fluoro-1-(toluène-4-sulfonyl)-1H-pyrrolo[2,3-b]pyridin-2-yl]-5,6-dimèthoxy-pyrrolo[3,2-b]pyridine-1-carboxylique

L'ester ter-butylique de l'acide 3-[4-chloro-5-fluoro-1-(toluène-4-sulfonyl)-1H-pyrrolo[2,3-b]pyridin-2-yl]-5,6-diméthoxy-pyrrolo[3,2--b]pyridine-1-carboxylique peut être préparé comme dans l'exemple 7 stade 7(f) mais à partir de 5,20g de 4-chloro-5-fluoro-1-(toluène-4-sulfonyl)-2-tributylstannanyl-1H-pyrrolo[2,3-b]pyridine, de 3,10g de l'ester ter-butylique de l'acide 3-bromo-5,6-diméthoxypyrrolo[3,2-b]pyridine-1-carboxylique, de 0,146g d'iodure de cuivre, de 0,889g de palladium tétrakis (triphénylphosphine) dans 160cm³ de toluène. On obtient ainsi, après flash chromatographie sur colonne de silice [éluant: cyclohexane/acétate d'éthyle (75/25 en volumes)], 3,30g de l'ester ter-butylique de l'acide 3-[4-chloro-5-fluoro-1-(toluène-4-sulfonyl)-1H-pyrrolo[2,3-b]pyridin-2-yl]-5,6-diméthoxy-pyrrolo[3,2-b]pyridine-1-carboxylique sous forme d'un solide dont les caractéristiques sont les suivantes:
- Rf CCM silice [éluant: cyclohexane/acétate d'éthyle (75/25 en volumes)]= 0,32
- Spectre RMN 1H (300MHz )- δ en ppm- dans le DMSO-d6: 1,71 (s, 9H) ; 2,32 (s, 3H) ; 3,85 (s, 3H) ; 3,90 (s, 3H) ; 7,13 (s, 1H) ; 7,34 (d large, 2H) ; 7,79 (d large, J = 8,0 Hz, 2H) ; 7,90 (s, 1H) ; 8,09 (s, 1H) ; 8,49 (d, J = 2,5 Hz, 1H)
- Spectre de Masse: SM-EI:600(+)=M(+)

### Stade 8(f): 3-[4-ohloro-5-fluoro-1-(toluène-4-sulfonyl)-1H-pyrrolo[2,3-b]pyridin-2-yl]-5,6-diméthoxy-1H-Pyrrolo[3,2-b]pyridine

Le 3-[4-chloro-5-fluoro-1-(toluène-4-sulfonyl)-1H-pyrrolo[2,3-b]pyridin-2-yl]-5,6-diméthoxy-1H-pyrralo[3,2-b]pyridine peut être préparé comme dans l'exemple 7 stade 7(g) mais à partir de 3,2g de l'ester ter-butylique de l'acide 3-[4-chloro-5-fluoro-1-(toluène-4-sulfonyl)-1H-pyrrolo[2,3-b]pyridin-2-yl]-5,6-diméthoxy-pyrrolo(3,2-b]pyridine-l-carboxylique, de 30cm³ d'acide trifluoroacétique dans 150cm³ de dichlorométhane. On obtient ainsi 2,50g de 3-[4-chloro-5-fluoro-1-(toluène-4-sulfonyl)-1H-pyrrolo[2,3-b]pyridin-2-yl]-5,6-diméthoxy-1H-pyrrolo[3,2-b]pyridine sous forme d'un solide dont les caractéristiques sont les suivantes:
- Rf CCM silice [éluant: cyclohexane/acétate d'éthyle (50/50 en volumes)]= 0,34.
- Spectre RMN 1H (300MHz )- δ en ppm- dans le DMSO-d6: 2,31 (s, 3H) ; 3,86 (s, 3H) ; 3,88 (s, 3H) ; 7,07 (s, 1H) ; 7,30 (d large, J = 8,5 Hz, 2H) ; 7,39 (s, 1H) ; 7,70 (d large, J = 8,5 Hz, 2H) ; 7,73 (s, 1H) ; 8,41 (d, J = 2,5 Hz, 1H) : 11,45 (s large, 1H)
- Spectre de Masse: SM-EI:501(k)=M(+)

### Stade 8 (g) : 1-(2-chloro-éthyl)-3-[4-chloro-5-fluoro-1-(toluène-4-sulfonyl)-1H-polo[2,3-b]pyridin-2-yl]-5,6-diméthoxy-1H-pyrrolo[3,2-b]pyridine

Le 1-(2-chloro-éthyl)-3-[4-chloro-5-fluoro-1-(toluène-4-sulfonyl)-1H-pyrrolo[2,3-b]pyridin-2-yl]-5,6-diméthoxy-1H-pyrrolo[3,2-b]pyridine peut être préparé comme dans l'exemple 7 stade 7(h) mais à partir de 0,50g de 3-[4-chloro-5-fluoro-1-(toluène-4-sulfonyl)-1H-pyrrolo[2,3-b]pyridin-2-yl]-5,6-diméthoxy-1H-pyrrolo[3,2-b]pyridine, de 0,0065g de bromure de tétrabutylammonium, de 0,44g d'hydroxyde de potassium et 0,32g de carbonate de potassium dans 50cm³ de 1,2-dichloro-éthane. On obtient ainsi 0,56g de 1-(2-chloro-éthyl)-3-[4-chloro-5-fluoro-1-(toluène-4-sulfonyl)-1H-pyrrolo[2,3-b]pyridin-2-yl]-5,6-diméthoxy-1H-pyrrolo[3,2-b]pyridine sous forme d'un solide dont les caractéristiques sont les suivantes:
- Rf CCM silice [éluant: cyclohexane /acétate d'éthyle (90/10 en volumes)]= 0,43
- Spectre RMN 1H (400MHz )- δ en ppm- dans le DMSO-d6: 2,30 (s, 3H) ; 3,86 (s, 3H) ; 3,89 (s, 3H) ; 4,07 (t, J= 6,0 Hz, 2H) ; 4,64 (t, J = 6,0 Hz, 2H) ; 7,06 (s, 1H) ; 7,29 (d large, J = 8,0 Hz, 2H) ; de 7,68 à 7,73 (m, 3H) ; 7,85 (s, 1H) ; 8, 45 (d, J = 2,5 Hz, 1H)
- Spectre de Masse: SM-EI:562(+)=M(+)

### stade 8 (h) : 3-[4-chloro-5-fluoro-1-(toluène-4-sulfonyl) 1H-pyrrolo[2,3-b]pyridin-2-yl]-1-(2-iodo-éthyl)-5,6-diméthoxy-1H-pyrrolo[3,2-b]pyridine

Le 3-[4-chloro-5-fluoro-1-(toluène-4-sulfonyl)-1H-pyrrolo[2,3-b]pyridin-2-yl)-1-(2-iodo-éthyl)-5,6-diméthoxy-1H-pyrrolo[3,2-b]pyridine peut être préparé comme dans l'exemple 7 stade 7(i) mais à partir de 0,56g de 1-(2-chloro-éthyl)-3-[4-chloro-5-fluoro-1-(toluène-4-sulfonyl)-1H-pyrrylo[2,3-b]pyridin-2-yl]-5,6-diméthoxy-1H-pyrrolo[3,2-b]pyridine et de 0,22g d'iodure de sodium dans 80cm³ de 2-butanone. On obtient ainsi 0,36g de 3-[4-chloro-5-fluoro-1-(toluène-4-sulfonyl)-1H-pyrrolo[2,3-b]pyridin-2-yl]-1-(2-iodo-éthyl)-5,6-diméthoxy-1H-pyrrolo[3,2-b]pyridine sous forme d'un solide dont les caractéristiques sont les suivantes:
- Rf CCM silice [éluant: cyclohexane/acétate d'éthyle (98/2 en volumes)]= 0,44
- Spectre RMN 1H (300MHz)- δ en ppm- dans le DMSO-d6: 2,30 (s, 3H) ; 3,67 (t, J = 6,0 Hz, 2H) ; 3,86 (s, 3H) ; 3,89 (s, 3H) ; 4,64 (t, J = 6,0 Hz, 2H) ; 7,03 (s, 1R) ; 7,30 (d large, J = 9,0 Hz, 2H) de 7,67 à 7,73 (m, 3H) ; 7,84 (s, 1H) ; 8,43 (d, J = 2,5 Hz, 1H)
- Spectre de Masse: SM-EI:654(+)=M(+)

### Stade 8 (i) : 3-[4-ahloro-5-fluoro-1-(toluène-4-sulfonyl)-1H-pyrrolo[2,3-b]pyridin-2-yl]-5,6-diméthoxy-1-(2-morpholin-4-yl-éthyl)-1H-pyrrolo[3,2-b]pyridine

Le 3-[4-chloro-5-fluoro-1-(toluène-4-sulfonyl)-1H-pyrrolo[2,3-b]pyridin-2-yl]-5,6-diméthoxy-1-(2-morpholin-4-yl-éthyl)-1H-pyrrolo[3,2-b]pyridine peut être préparé comme dans l'exemple 7 stade 7(j) mais à partir de 0,35g de 3-[4-chloro-5-fluoro-1-(toluène-4-sulfonyl)-1H-pyrrolo[2,3-b]pyridin-2-yl]-1-(2-iodo-éthyl)-5,6-diméthoxy-1H-pyrrolo[3,2-b]pyridine, de 0,082g de carbonate de potassium et de 0,093g de morpholine dans 44cm³ d'acétonitrile. On obtient ainsi, après flash chromatographie sur colonne de silice [éluant: dichlorométhane/méthanol (98/2 en volumes)], 0,22g de 3-[4-chloro-5-fluoro-1-(toluène-4-sulfonyl)-1H-pyrrolo[2,3-b]pyridin-2-yl]-5,6-diméthoxy-1-(2-morpholin-4-yl-éthyl)-1H-pyrrolo[3,2-b]pyridine sous forme d'une résine dont les caractéristiques sont les suivantes:
- Rf CCM silice [éluant: dichlorométhane/méthanol (98/2 en volumes)]= 0,16
- Spectre RMN 1H (300MHz)- δ en ppm- dans le DMSO-d6: 2,30 (s, 3H) ; de 2,45 à 2,55 (m masqué, 4H) ; 2,76 (m, 2H) ; 3,55 (m, 4H) ; 3, 83 (s, 3H) ; 3, 89 (s, 3H) ; 4,38 (m, 2H) ; 7,07 (s, 1H) ; 7,28 (d large, J = 8,0 Hz, 2H) ; de 7,62 à 7,69 (m, 3H) ; 7,83 (s, 1H) ; 8,42 (d, J = 2,5 Hz, 1H)
- Spectre de Masse: SM-EI:613(+)=M(+)

### Stade 8(j): 3-(4-chloro-5-fluoro-1H-pyrrolo[2,3-b]pyridin-2-yl)-5,6-diméthoxy-1-(2-morpholin-4-yl-éthyl)-1H-pyrrolo[3,2-b]pyridine

Le 3-(4-chloro-5-fluoro-1H-pyrrolo[2,3-b]pyridin-2-yl)-5,6-diméthoxy-1-(2-morpholin-4-yl-éthyl)-1H-pyrrolo[3,2-b]pyridine peut être préparé comme dans l'exemple 7 stade 7(k) mais à partir de 0,220g de 3-[4-chloro-5-fluoro-1-(toluène-4-sulfonyl)-1H-pyrrolo[2,3-b]pyridin-2-yl]-5,6-diméthoxy-1-(2-morpholin-4-yl-éthyl)-1H-pyrrolo[3,2-b]pyridine, de 0,52g d'hydroxyde de potassium dans 40cm³ de méthanol. On obtient ainsi, après flash chromatographie sur colonne de silice [éluant: dichlorométhane/méthanol (98/2 en volumes)], 0,128g de 3-(4-chloro-5-fluoro-1H-pyrrolo[2,3-b]pyridin-2-yl)-5,6-diméthoxy-1-(2-morpholin-4-yl-éthyl)-1H-pyrrolo[3,2-b]pyridine sous forme d'un solide dont les caractéristiques sont les suivantes:
- point de fusion: fondant à 206°C (Banc Kofler)
- Spectre RMN 1H (300MHz)- δ en ppm- dans le DMSO-d6: 2,47 (m, partiellement masqué, 4H) ; 2,72 (t, J = 6,0 Hz, 2H) ; 3,58 (m, 4H) ; 3,88 (s, 3H) ; 4,06 (s, 3H) ; 4,33 (t, J = 6,0 Hz, 2H) ; 7,28 (s, 1H) ; 7,67 (s, 1H) ; 8,05 (s, 1H) ; 8, 19 (d, J = 2,5 Hz, 1H) ; 12,2 (m large, 1H)
- Spectre de Masse: SM-EI : 459 (+) =M (+) -

### Exemple____10 : 3-(4-Chloro-5-fluoro-1H-pyrrolo[2,3-b]pyridin-2-yl)-5,6-diméthoxy-1-[2-(4-méthylpipérazin-1-yl)-éthyl]-1H-pyrrolo[3,2-b]pyridine

### Stade 10 (a) : 3-[4-Chloro-5-fluoro-1-(toluéne-4-sulfonyl)-1H-pyrrolo[2,3-b]pyridin-2-yl]-5,6-diméthoxy-1-[2-(4-méthylpipérazin-1-yl)-éthyl]-1H-pyrrolo[3,2-b]pyridine

Le 3-[4-chloro-5-fluoro-1-(toluène-1-sulfonyl)-3H-pyrrolo[2,3-b]pyridin-2-yl]-5,6-diméthoxy-1-[2-(4-méthylpipérazin-1-yl)-éthyl]-1H-pyrrolo[3,2-b]pyridine peut être préparé comme dans l'exemple 2 stade 2(c) mais à partir de 0,18g de 3-[4-chloro-5-fluoro-1-(toluène-4-sulfonyl)-1H-pyrrolo[2,3-b]pyridin-2-yl]-7-(2-iodo-éthyl)-5,6-diméthoxy-1H-pyrrolo[3,2-b]pyridine, de 0,042g de carbonate de potassium et de 0,055g de 1-méthylpipérazine dans 25cm³ d'acétonitrile. On obtient ainsi, après flash chromatographie sur colonne de silice [éluant: dichlorométhane/méthanol (95/5 en volumes)], 0, 065g de 3-[4-chloro-5-fluoro-1-(toluène-4-sulfonyl)-1H-pyrrolo[2,3-b]pyridin-2-yl]-5,6-diméthoxy-1-[2-(4-méthylpipérazin-1-yl)-éthyl]-1H-pyrrolo[3,2-b]pyridine sous forme d'une résine dont les caractéristiques sont les suivantes:
- Rf CCM silice [éluant: dichlorométhane/méthanol (95/5 en volumes)]= 0,084
- Spectre de Masse: ES m/z=627 MH⁺

### stade 10 (b) : 3- (4-chloro-5-fluoro-1H-pyrrolo [2,3-b]pyridin-2-yl)-5,6-diméthoxy-1-[2-(4-méthylpipérazin-1-yl) -éthyl]-1H-pyrrolo[3,2-b]pyridine

Le 3-(4-chloro-5-fluoro-1*H*-pyrrolo[2,3-b]pyridin-2-yl)-5,6-diméthoxy-1-[2-(4-méthylpipérazin-1-yl)-éthyl]-1H-pyrrolo[3,2-b]pyridine peut être préparé comme dans l'exemple 2 stade 2(d) mais à partir de 0,065g de 3-[4-chloro-5-fluoro-1-(toluène-4-sulfonyl)-1H-pyrrolo(2,3-b]pyridin-2-yl]-5,6-diméthoxy-1-[2-(4-méthylpipérazin-1-yl)-éthyl]-1H-pyrrolo[3,2-b]pyridine, de 0,116g d'hydroxyde de potassium dans 25cm³ de méthanol. On obtient ainsi, après flash chromatographie sur colonne de silice [éluant: dichlorométhane/méthanol (90/10 en volumes)], 0,030g de 3-(4-chloro-5-fluoro-1H-pyrrolo[2,3-b]pyridin-2-yl)-5,6-diméthoxy-1-[2-(4-méthylpipérazin-1-yl)-éthyl]-1H-pyrrolo[3,2-b]pyridine sous forme d'un solide dont les caractéristiques sont les suivantes:
- Rf CCM silice [éluant: dichlorométhane/méthanol (90/10 en volumes)]= 0,15
- Spectre RMN 1H (300MHz)- δ en ppm- dans le DMSO-d6: 2,13 (s, 3H) ; 2,29 (m large, 4H) ; de 2,42 à 2,55 (m partiellement masqué, 4H) ; 2,70 (t large, J = 6,5 Hz, 2H) ; 3,88 (s, 3H) ; 4,05 (s, 3H) ; 4,31 (t large, J = 6,5 Hz, 2H) ; 7,26 (s large, 1H) ; 7,65 (s, 1H) ; 8,04 (s, 1H) ; 8,18 (d, J = 2,5 Hz, 1H) ; 12,15 (s large, 1H)
- Spectre de Masse: IE m/z=472 M^{+.} ; m/z=113 C₆H₁₃N₂⁺ pic de base ; m/z=70 C₄H₈N⁺

### Exemple 11 : 3-[4-Chloro-5-fluoro-1H-pyrrolo[2,3-b]pyridin-2-yl] -5,6-diméthoxy-1- (3-pipéridin-1-yl-propyl)-1H-pyrrolo[3,2-b]pyridine

### Stade 11 (a) : 3-[4-Chloro-5-fluoro-1-(toluène-4-sulfonyl)-1H-pyrrolo[2,3-b]pyridin-2-yl]-1-(3-chloro-propyl)-5,6-diméthoxy-1H-pyrrolo[3,2-b]pyridine

Le 3-[4-chloro-5-fluoro-1-(toluène-4-sulfonyl)-1H-pyrrolo[2,3-b]pyridin-2-yl]-1-(3-chloro-propyl)-5,6-diméthoxy-1H-pyrrolo[3,2-b]pyridine peut être préparé comme dans l'exemple 2 stade 2(a) mais à partir de 1,8g de 3-[4-chloro-5-fluoro-1-(toluène-4-sulfonyl)-1H-pyrrolo[2,3-b]pyridin-2-yl]-5,6-diméthoxy-1H-pyrrolo[3,2-b]pyridine, de 0,046g de bromure de tétrabutylammonium, de 1,34g de d'hydroxyde de potassium et 1,16g de carbonate de potassium dans 150cm³ de 1-bromo-3-chloropropane. On obtient ainsi 2g de 3-[4-chloro-5-fluoro-1-(toluène-4-sulfonyl)-1H-pyrrolo[2,3-b]pyridin-2-yl]-1-(3-chloro-propyl)-5,6-diméthoxy-1H-pyrrolo[3,2-b]pyridine sous forme d'une résine dont les caractéristiques sont les suivantes:
- Rf CCM silice [éluant: cyclohexane/acétate d'éthyle (50/50 en volumes)]= 0,63
- Spectre de Masse: ES m/z=577 MH⁺ pic de base

### Stade 11 (b) : 3-[4-Chloro-5-fluoro-1-(toluène-4-sulfonyl) 1H-pyrrolo[2,3-b]pyridin-2-yl]-1-(3-iodo-propyl)-5,6-diméthoxy-1H-pyrrolo[3,2-b]pyridine

Le 3-[4-chloro-5-fluoro-1-(toluène-4-sulfonyl)-1H-pyrrolo[2,3-b]pyridin-2-yl]-1-(3-iodo-proyl)-5,6-diméthoxy-1H-pyrrolo[3,2-b]pyridine peut être préparé comme dans l'exemple 2 stade 2(b) mais à partir de 2,07g de 3-[4-chloro-5-fluoro-1-(toluène-4-sulfonyl)-1H-pyrrolo[2,3-b]pyridin-2-yl]-1-(3-chloro-propyl)-5,6-diméthoxy-1H-pyrrolo[3,2-b]pyridine et de 1,07g d'iodure de sodium dans 150cm³ de 2-butanone. On obtient ainsi 2,3g de 3'-[4-chloro-5-fluoro-1-(toluène-4-sulfonyl)-1H-pyrrolo[2,3-b]pyridin-2-yl]-1-(3-iodo-proyl)-5,6-diméthoxy-1H-pyrrolo[3,2-b]pyridine sous forme d'une résine dont les caractéristiques sont les suivantes:
- Rf CCM silice [éluant: cyclohexane/acétate d'éthyle (50/50 en volume)]= 0,67
- Spectre de Masse: IE m/z=668 M^{+.} pic de base ; m/z=513 (M˙⁻ C₇H₇SO₂)⁺; m/z=386 (m/z=513 - I)^{+.}

### stade 11(c) : 3-[4-Chloro-5-fluoro-1-(toluène-4-sulfonyl)-1H-pyrrolo[2,3-b]pyridin-2-yl]-5,6-diméthoxy-(3-pipéridin-1-yl-propyl)-1H-pyrrolo[3,2-b]pyridine

Le 3-[4-chloro-5-fluoro-1-(toluène-4-sulfonyl)-1H-pyrrolo[2,3-b]pyridin-2-yl]-5,6-diméthoxy-(3-pipéridin-1-yl-propyl)-1H-pyrrolo[3,2-b]pyridine peut être préparé de la manière suivante:
une solution de 0,3g de 3-[4-chloro-5-fluoro-1-(toluène-4-sulfonyl)-1H-pyrrolo[2,3-b]pyridin-2-yl]-1-(3-iodo-proyl)-5,6-diméthoxy-1H-pyrrolo[3,2-b]pyridine, de 0,382g de pipéridine dans 40cm3 de dichlorométhane est portée au reflux pendant environ 6 heures. Après dilution du mélange réactionnel avec 100cm3 de dichlorométhane, la solution est lavée par trois fois 40cm3 d'eau, séchée sur sulfate de magnésium, filtrée et concentrée à sec sous pression réduite (13KPa). On obtient ainsi, après flash chromatographie sur colonne de silice [éluant: dichlorométhane/méthanol(98/2 en volumes)], 0,175g de 3-[4-chloro-5-fluoro-1-(toluène-4-sulfonyl)-1H-pyrrolo[2,3-b]pyridin-2-yl]-5,6-diméthoxy-(3-pipéridin-1-yl-propyl)-1H-pyrrolo[3,2-b]pyridine sous forme d'une résine dont les caractéristiques sont les suivantes:
   - Rf CCM silice [éluant: dichlorométhane/méthanol (90/10 en volumes)]= 0,26
   - Spectre de Masse: ES m/z=626 MH⁺ pic de base

### Stade 11 (d) : 3-[4-Chloro-5-fluoro-1H-pyrrolo[2,3-b]pyridin-2-yl]-5,6-diméthoxy-1-(3-pipéridin-1-yl-propyl)-1H-pyrrolo[3,2-b]pyridine

Le 3-[4-chloro-5-fluoro-1H-pyrrolo[2,3-b]pyridin-2-yl]-5,6-diméthoxy-1-(3-pipéridin-1-yl-propyl)-1H-pyrrolo[3,2-b]pyridine peut être préparé comme dans l'exemple 2 stade 2(d): mais à partir de 0,175g de 3-[4-chloro-5-fluoro-1-(toluène-4-sulfonyl)-1H-pyrrolo[2,3-b]pyridin-2-yl]-5,6-diméthoxy-(3-pipéridin-1-yl-propyl)-1H-pyrrolo[3,2-b]pyridine, de 0,314g d'hydroxyde de potassium dans 40cm³ de méthanol. On obtient ainsi, après flash chromatographie sur colonne de silice [éluant: dichlorométhane/méthanol (95/5 en volumes)], 0,086g de 3-[4-chloro-5-fluoro-1H-pyrrolo[2,3-b]pyridin-2-yl]-5,6-diméthoxy-1-(3-pipéridin-1-yl-propyl-1H-pyrrolo[3,2-b]pyridine sous forme d'un solide dont les caractéristiques sont les suivantes:
- point de fusion: fondant à 110-114°C (Banc Kofler)
- Spectre RMN 1H (300MHz)- δ en ppm- dans le DMSO-d6: 1,37 (m, 2H) ; 1,50 (m, 4H) ; 1,95 (m, 2H) ; 2,20 (t, J = 7,0 Hz, 2H) ; 2,28 (m, 4H) ; 3,88 (s, 3H) ; 4,05 (s, 3H) ; 4,24 (t, J = 7,0 Hz, 2H) ; 7,26 (d, J = 2,0 Hz, 1H) ; 7,59 (s, 1H) ; 8,04 (s, 1H) ; 8,18 (d, J = 2,5 Hz, 1H) ; 12,1 (s large, 1H)
- Spectre de Masse: IE m/z=471 M^{+.} ; m/z=360 (M - C₇H₁₃N) ⁺ pic de base ; m/z=98 C₆H₁₂N⁺

### Exemple 12 : 1-{3-[3-(4-Chloro-5-fluoro-1H-pyrrolo[2,3-b]pyridin-2-yl)-5,6-diméthoxy-pyrrolo[3,2-b]pyridin-1-yl]-propyl}-pipéridin-4-ol

### Stade 12 (a) : 1-(3-{3-[4-Chloro-5-fluoro-1-(toluène-4-sulfonyl) -1H-pyrrolo [2,3-b]pyridin-2-yl]-5,6-diméthoxy-pyrrolo[3,2-b]pyridin-1-yl}-propyl)-pipéridin-4-ol

Le 1-(3-{3-[4-chloro-5-fluoro-1-(toluène-4-sulfonyl)-1H-pyrrolo[2,3-b]pyridin-2-yl]-5,6-diméthoxy-pyrrolo[3,2-b]pyridin-1-yl}-propyl)-pipéridin-4-ol peut être préparé comme dans l'exemple 11 stade 11 (c) mais à partir de 0,300g de 3-[4-chloro-5-fluoro-1-(toluène-4-sulfonyl)-1H-pyrrolo[2,3-b]pyridin-2-yl)-1-(3-iodo-proyl)-5,6-diméthoxy-1H-pyrrolo[3,2-b]pyridine, de 0,454g de pipéridin-4-ol dans 40cm3 de dichlorométhane. On obtient ainsi, après flash chromatographie sur colonne de silice [éluant: dichlorométhane/méthanol(95/5 en volumes)], 0,173g de 1-(3-{3-[4-chloro-5-fluoro-1-(toluène-4-sulfonyl)-1H-pyrrolo[2,3-b]pyridin-2-yl]-5,6-diméthoxy-pyrrolo[3,2-b]pyridin-1-yl}-propyl)-pipéridin-4-ol sous forme d'une meringue dont les caractéristiques sont les suivantes:
- Rf CCM silice [éluant: dichlorométhane/méthanol (90/10 en volumes)]= 0,14
- Spectre de Masse: ES m/z=642 MH⁺ pic de base

### Stade 12 (b): 1-{3-[3-(4-chloro-5-fluoro-1H-pyrrolo[2,3-b]pyridin-2-yl)-5,6-diméthoxy-pyrrolo[3,2-b]pyridin-1-yl]-propyl}-pipéridin-4-ol

Le 1-13-[3-(4-chloro-5-fluoro-1H-pyrrolo[2,3-b]pyridin-2-yl)-5,6-diméthoxy-pyrrolo[3,2-b]pyridin-1-yl]-propyl)-pipéridin-4-ol peut être préparé comme dans l'exemple 2 stade (2d)mais à partir de 0,178g de 1-(3-{3-[4-chloro-5-fluoro-1-(toluène-4-sulfonyl)-1H-pyrrolo[2,3-b]pyridin-2-yl]-5,6-diméthoxy-pyrrolo[3,2-b]pyridin-1-yl}-propyl)-pipéridin-4-ol, de 0,31g d'hydroxyde de potassium dans 40cm³ de méthanol. On obtient ainsi, après flash chromatographie sur colonne de silice [éluant: dichlorométhane/méthanol (90/10 en volumes)], 0,102g de 1-{3-[3-(4-chloro-5-fluoro-1H-pyrrolo[2,3-b]pyridin-2-yl)-5,6-diméthoxy-pyrrolo[3,2-b]pyridin-1-yl]-propyl)-pipéridin-4-ol sous forme d'un solide dont les caractéristiques sont les suivantes:
- point de fusion: fondant à 188°C (Banc Kofler)
- Spectre RMN 1H (300MHz)- δ en ppm- dans le DMSO-d6: 1,41 (m, 2H) ; 1,71 (m, 2H) ; de 1,88 à 2,00 (m, 4H) ; 2,20 (t, J = 6,5 Hz, 2H) ; 2,65 (m, 2H) ; 3,43 (m, 1H) ; 3,88 (s, 3H) ; 4,05 (s, 3H) ; 4,23 (t, J = 6,5 Hz, 2H) ; 4,52 (d, J = 4,0 Hz, 1H) ; 7,26 (d, J = 2,0 Hz, 1H) ; 7,59 (s, 1H) ; 8,03 (s, 1H) ; 8,18 (d, J = 2,5 Hz, 1H) ; 12,15 (s large, 1H)
- Spectre de Masse: IE m/z=487 M^{+.} ; m/z=360 (M C₇H₁₃NO).⁺ pic de base ; m/z=114 C₅H₁₂NO⁺

### exemple 15 : C-(1-{3-[3-(4-chloro-5-fluoro-1H-pyrrolo[2,3-b]pyridin-2-yl)-5,6-diméthoxy-pyrrolo[3,2-b]pyridin-1-yl]-propyl}-pipéridin-4-yl)-méthylamine

### stade 15 (a) : c-[1-(3-{3-[4-chloro-5-fluoro-1-(toluène-4-sulfonyl)-1H-pyrrolo[2,3-b]pyridin-2-yl]-5,6-diméthoxy-pyrrolo[3,2-b]pyridin-1-yl)-propyl)-pipéridin-4-yl]-méthylamine

Le C-[1-(3-{3-[4-chloro-5-fluoro-1-(toluène-4-sulfonyl) 1H-pyrrolo[2,3-b]pyridin-2-yl]-5,6-diméthoxy-pyrrolo[3,2-b]pyridin-1-yl}-propyl)-pipéridin-4-yl]-méthylamine peut être préparé comme dans l'exemple 11 stade (c) mais à partir de 0,300g de 3-[4-chloro-5-fluoro-1-(toluène-4-sulfonyl)-1H-pyrrolo[2,3-b]pyridin-2-yl]-1-(3-iodo-proyl)-5,6-diméthoxy-1H-pyrrolo[3,2-b]pyridine, de 0,512g de C-pipéridin-4-yl-méthylamine dans 50cm3 de dichlorométhane. On obtient ainsi, après flash chromatographie sur colonne de silice [éluant: chloroforme/méthanol/ammoniaque aqueuse à 28% (12/3/0,5 en volumes)], 0,150g de C-[1-(3-{3-[4-chloro-5-fluoro-1-(toluène-4-sulfonyl)-1H-pyrrolo[2,3-b]pyridin-2-yl]-5,6-diméthoxy-pyrrolo[3,2-b]pyridin-1-yl}-propyl)-pipéridin-4-yl]-méthylamine sous forme d'une résine dont les caractéristiques sont les suivantes:
- Rf CCM silice [éluant: chloroforme / méthanol/ammoniaque aqueuse à 28% (12/3/0,5 en volumes)]= 0,60
- Spectre de Masse: IC m/z=655 MH⁺ pic de base

### Stade 15 (b) : C-(1-{3-[3-(4-chloro-5-fluoro-1H-pyrrolo[2,3-b]pyridin-2-yl)-5,6-diméthoxy-pyrrolo[3,2-b]pyridin-1-yl]-propyl}-pipéridin-4-yl)-méthylamine

Le C-(1-{3-[3-(4-chloro-5-fluoro-1H-pyrrolo[2,3-b]pyridin-2-yl)-5,6-diméthoxy-pyrrolo[3,2-b]pyridin-1-yl]-propyl}-pipéridin-4-yl)-méthylamine peut être préparé comme dans l'exemple 2 stade (2d) mais à partir de 0,150g de C-[1-(3-{3-[4-chloro-5-fluoro-1-(toluène-4-sulfonyl)-1H-pyrrolo[2,3-b]pyridin-2-yl)-5,6-diméthoxy-pyrrolo[3,2-b]pyridin-1-yl}-propyl)-pipéridin-4-yl]-méthylamine, de 0,256g d'hydroxyde de potassium dans 50cm³ de méthanol. On obtient ainsi, après flash chromatographie sur colonne de silice [éluant: chloroforme/méthanol ammoniacal 2M (90/10 en volumes)], 0,035g de C-'(1-{3-[3-(4-chloro-5-fluoro-1H-pyrrolo[2,3-b]pyridin-2-yl)-5,6-diméthoxy-pyrrolo[3,2-b]pyridin-1-yl]-propyl}-pipéridin-4-yl)-méthylamine sous forme d'un solide dont les caractéristiques sont les suivantes:
- point de fusion: fondant à 135°C (Banc Kofler)
- spectre RMN 1H (300MHz)- δ en ppm- dans le DMSO-d6: De 1,01 à 1,21 (m, 3H) ; 1,65 (m, 2H) ; 1,79 (m, 2H) ; 1,95 (m, 2H) ; 2,21 (t, J = 7,0 Hz, 2H) ; 2,40 (d, J = 5,5 Hz, 2H) ; 2,80 (m, 2H) ; 3,88 (s, 3H) ; 4,05 (s, 3H) ; 4,23 (t, J = 7,0 Hz, 2H) ; 7,26 (s, 1H) ; 7,59 (s, 1H) ; 8,03 (s, 1H) ; 8,18 (d, J = 2,5 Hz, 1H) ; de 11,4 à 12,4 (m très étalé, 1H).
- Spectre de Masse: IE m/z=500 M^{+.} ; m/z=360 (M - C₈H₁₆N₂)^{+.} pic de base

### Exemple 16 : 3-(4-Chloro-5-fluoro-1H-pyrrolo[2,3-b]pyridin-2-yl)-5,6-diméthoxy-1-[3-(4-méthyl-pipérazin-1-yl)-propyl]-1H-pyrrolo[3,2-b]pyridine

### Stade 16 (a) : 3-[4-Chloro-5-fluoro-1-(toluène-4-sulfonyl) -1H-pyrrolo[2,3-b]pyridin-2-yl] -5,6-diméthoxy-1-[3-(4-méthyl-pipérazin-1-yl)-propyl]-1H-pyrrolo[3,2-b]pyridine

Le 3-[4-chloro-5-fluoro-3-(toluène-4-sulfonyl)-1H-pyrrolo[2,3-b]pyridin-2-yl]-5,6-diméthoxy-1-[3-(4-méthylpipérazin-1-yl)-propyl]-1H-pyrrolo[3,2-b]pyridine peut être préparé comme dans l'exemple 2 stade 2(c) mais à partir de 0,300g de 3-[4-chloro-5-fluoro-1-(toluène-4-sulfonyl)-1H-pyrrolo[2,3-b]pyridin-2-yl]-1-(2-iodopropyl)-5,6-diméthoxy-1H-pyrrolo[3,2-b]pyridine, de 0,068g de carbonate de potassium et de 0,090g de 1-méthyl-pipérazine dans 50cm³ d'acétonitrile. On obtient 0,192g de 3-[4-chloro-5-fluoro-1-(toluène-4-sulfonyl)-1H-pyrrolo[2,3-b]pyridin-2-yl]-5,6-diméthoxy-1-(3-morpholin-4-yl-propyl)-1H-pyrrolo[3,2-b]pyridine sous forme d'une résine dont les caractéristiques sont les suivantes:
- Rf CCM silice [éluant: dichlorométhane/méthanol (90/10 en volumes)]= 0,23
- Spectre de Masse: IE m/z=640 M⁺ ; m/z=485 (M - C₇H₇SO₂)⁺ ; m/z=385 (m/z=485 - C₅H₁₂N₂)⁺ ; m/z=359 (m/z=485 - C₇H₁₄N₂)⁺ pic de base ; m/z=98 C₆H₁₂N⁺

### stade 16 (b) : 3-(4-Chloro-5-fluoro-1H-pyrrolo[2,3-b] pyridin-2-yl)-5,6-diméthoxy-1-[3-(4-méthyl-pipérazin-1-yl)-propyl]-1H-pyrrolo[3,2-b]pyridine

Le 3-(4-chloro-5-fluoro-1H-pyrrolo[2,3-b]pyridin-2-yl)-5,6-diméthoxy-1-[3-(4-méthyl-pipérazin-1-yl)-propyl]-1H-pyrrolo[3,2-b]pyridine peut être préparé comme dans l'exemple 2 stade (2d) mais à partir de 0,190g de 3-[4-chloro-5-fluoro-1-(toluène-4-sulfonyl)-1H-pyrrolo[2,3-bipyridin-2-yl]-5,6-diméthoxy-1-(3-morpholin-4-yl-propyl)-1H-pyrrolo[3,2-b]pyridine, de 0,332g d'hydroxyde de potassium dans 50cm³ de méthanol. On obtient ainsi, après flash chromatographie sur colonne de silice [éluant: dichlorométhane/méthanol (90/10 en volumes)], 0,054g de 3-(4-chloro-5-fluoro-1H-pyrrolo[2,3-b]pyridin-2-yl)-5,6-diméthoxy-1-[3-(4-méthyl-pipérazin-1-yl)-propyl]-1H-pyrrolo[3,2-b]pyridine sous forme d'un solide dont les caractéristiques sont les suivantes:
- point de fusion: fondant à 155°C (Banc Kofler)
- Spectre RMN 1H (300MHz)- δ en ppm- dans le DMSO-d6: 1,95 (m, 2H) ; 2,14 (s, 3H) ; 2,23 (t, J = 7,0 Hz, 2H) ; de 2,25 à 2,40 (m large, 8H) ; 3,88 (s, 3H) ; 4,05 (s, 3H) ; 4,24 (t, J = 7,0 Hz, 2H) ; 7,26 (s large, 1H) ; 7,59 (s, 1H) ; 8,03 (s, 1H) ; 8,18 (d, J = 2,5 Hz, 1H) ; 12,15 (s large, 1H).
- Spectre de Masse: ES m/z=487 MH⁺ pic de base

### Exemple 19 : C-(1-{2-[3-(4-Chloro-1H-pyrrolo[2,3-b]pyridin-2-yl)-5,6-diméthoxy-pyrrolo[3,2-b]pyridin-1-yl]-éthyl}-pipéridin-4-yl)-méthylamine

### Stade 19(a) : C-[1-(2-{3-[4-chloro-1-(toluène-4-sulfonyl)-1H-pyrrolo[2,3-b]pyridin-2-yl]-5,6-diméthoxy-pyrrolo[3,2-b]pyridin-1-yl}-éthyl)-pipéridin-4-yl]-méthylamine

Le C-[1-(2-{3-{4-chloro-1-(toluène-4-sulfonyl)-1H-pyrrolo [2,3-b]pyridin-2-yl]-5,6-diméthoxy-pyrrolo[3,2-b]pyridin-1-yl}-éthyl)-pipéridin-4-yl]-méthylamine peut être préparé comme dans l'exemple 5 stade 5(c) mais à partir de 0,3 g de 3-[4-chloro-1-(toluène-4-sulfonyl)-1H pyrrolo[2,3-b] pyridin-2-yl]-1-(2-iodo-éthyl)-5,6-diméthoxy-1H-pyrrolo[3,2-b]pyridine dans 40 cm³ de CH₂Cl₂ et 0,538 g de 4-aminométhyl pipéridine. On obtient ainsi 0,108 g de C-[1-(2-{3-[4-chloro-1-(toluéne-4-sulfonyl)-1H-pyrrolo[2,3-b]pyridin-2-yl]-5,6-diméthoxy-pyrrolo[3,2-b)pyridin-1-yl}-éthyl}-pipéridin-4-yl]-méthylamine sous forme d'une résine orangée dont les caractéristiques sont les suivantes:
- Rf CCM silice [éluant: chloroforme/ méthanol/ammoniaque (13/3/0,5 en volumes)]= 0,5
- Spectre de Masse: ES m/z=623 MH+

### Stade 19 (b) : C-(1-{2-[3-(4-Chloro-1H-pyrrolo[2,3-b]pyridin-2-yl)-5,6-diméthoxy-pyrrolo[3,2-b]pyridin-1-yl]-éthyl}-pipéridin-4-yl)-méthylamine

Le C-(1-{2-[3-(4-chloro-1H-pyrrolo[2,3-b]pyridin-2-yl)-5,6-diméthoxy-pyrrolo[3,2-b]pyridin-1-yl]-éthyl}-pipéridin-4-yl)-méthylamine peut être préparé comme dans l'exemple 5 stade 5 (d) mais à partir de 0,108 g C-[1-(2-{3-[4-chloro-1-(toluène-4-sulfonyl)-1H-pyrrolo[2,3-b]pyridin-2-yl]-5,6-diméthoxy-pyrrolo[3,2-b]pyridin-1-yl}-éthyl)-pipéridin-4-yl]-méthylamine et 0,194 g de potasse dans 30 cm³ de méthanol. On obtient ainsi 0,04 g de C-(1-{2-[3-(4-chloro-1H-pyrrolo[2,3-b]pyridin-2-yl)-5,6-diméthoxy-pyrrolo[3,2-b]pyridin-1-yl]-éthyl}-pipéridin-4-yl)-méthylamine sous forme d'une poudre crème dont les caractéristiques sont les suivantes:
- Spectre de Masse: IE m/z=468 M^{+.} ; m/z=127 C₇H₁₅N₂⁺ pic de base ; m/z=110 (m/z=127 - NH₃)⁺
- Spectre RMN 1H (300MHz)- δ en ppm- dans le DMSO-d6: De 0,98 à 1,21 (m, 3H) ; 1,65 (m, 2H) ; 1,96 (m, 2H) ; 2,38 (d, J = 6, 0 Hz, 2H) ; 2,68 (t, J = 6,5 Hz, 2H) ; 2,90 (m, 2H) ; 3,88 (s, 3H) ; 4,06 (s, 3H) ; 4,29 (t, J = 6,5 Hz, 2H) ; 7,11 (d, J = 5, 5 Hz, 1H) ; 7,25 (s, 1H) ; 7,65 (s, 1H) ; 8,02 (s, 1H) ; 8,05 (d, J = 5, 5 Hz, 1H) ; 12,1 (m étalé, 1H).

### Exemple 21 : [3-(4-chloro-1H-pyrrolo[2,3-b]pyridin-2-yl)-5,6-diméthoxy-pyrrolo[3,2-b]pyridin-1-yl]-acétate de potassium

### Stade 21 (a): [3-(4-chloro-1-(toluène-4-sulfonyl)-1H-pyrrolo[2,3-b]pyridin-2-yl) -5,6-diméthoxy-pyrrolo [3,2-b]pyridin-1-yl]-acétate de tert-butyle

Le [3-(4-chloro-1-(toluène-4-sulfonyl)-1H-pyrrolo[2,3-b]pyridin-2-yl)-5,6-diméthoxy-pyrrolo[3,2-b]pyridin-1-yl]-acétate de tert-butyle est préparé de la manière suivante:

A une solution de 0,5 g de 3-[4-chloro-1-(toluène-4-sulfonyl)-1H-pyrrolo[2,3-b]pyridin-2-yl]-5,6-diméthoxy-1H-pyrrolo[2,3-b]pyridine dans 10 cm³ bromoacétate de tert-butyle, sous atmosphère inerte d'argon à une température voisine de 20°C, sont ajoutés 0,333 g de carbonate de potassium, 0,387 g de potasse et 0,0067 g de bromure de tétrabutyle ammonium. Le milieu réactionnel est agité à la même température pendant 16 heures. 100 cm³ de dichlorométhane sont ajoutés et la phase organique est lavée par 3x100 cm³ d'eau. Après décantation, la phase organique est séchée sur sulfate de magnésium, filtrée puis concentrée sous pression réduite. Le résidu obtenu est purifié par flash-chromatographie (silice, cyclohexane/acétate d'éthyle 75/25 en volumes). Les fractions contenant le produit sont concentrées sous pression réduite. On obtient ainsi 0,51 g de [3-(4-chloro-1-(toluène-4-sulfonyl)-1H-pyrrolo[2,3-b]pyridin-2-yl)-5,6-diméthoxy-pyrrolo[3,2-b]pyridin-1-yl]-acétate de tert-butyle dont les caractéristiques sont les suivantes:
- Rf CCM silice [éluant: cyclohexane/acétate d'éthyle (50/50 en volumes)]= 0,14
- Spectre de Masse: IE m/z=596 M^{+.} ; m/z=540 (M - C₄H₈)^{+.} ; m/z=985 (m/z=540 - C₇H₇SO₂)⁺ pic de base ; m/z=341 (m/z=385 - CO₂)⁺

### Stade 21(b) : [3-(4-chloro-1H-pyrrolo[2,3-b]pyridin-2-yl)-5,6-diméthoxy-pyrrolo[3,2-b]pyridin-1-yl]-acétate de potassium

Le [3-(4-chloro-1H-pyrrolo[2,3-b]pyridin-2-yl)-5,6-diméthoxy-pyrrolo[3,2-b]pyridin-1-yl]-acétate de potassium peut être préparé comme dans l'exemple 5 stade 5(d)mais à partir de 0,51 g de [3-(4-chloro-1-(toluène-4-sulfonyl)-1H-pyrrolo[2,3-b]pyridin-2-yl)-5,5-diméthoxy-pyrrolo[3,2-b]pyridin-1-yl] -acétate de tert-butyle et 3,34 g de potasse dans 33,4 cm³ de méthanol. On obtient ainsi 0,125 g de [3-(4-chloro-1H-pyrrolo[2,3-b]pyridin-2-yl)-5,6-diméthoxy-pyrrolo[3,2-b]pyridin-1-yl]-acétate de potassium sous forme d'un solide jaune dont les caractéristiques sont les suivantes:
- Spectre de Masse: IE m/z=386 M^{+.} pic de base ; m/z=341 (M - CO₂H)⁺ ; m/z=44 CO₂^{+.}
- Spectre RMN 1H (300MHz) - δ en ppm- dans le DMSO-d6: 3,82 (s, 3H) ; 4,03 (s, 3H) ; 4,67 (s, 2H) ; 7,11 (d, J = 5,5 Hz, 1H) ; 7,23 (s, 1H) ; 7,49 (s, 1H) ; 7,92 (s, 1H) ; 8,03 (d, J = 5,5 Hz, 1H) ; 12,05 (m étalé, 1H) .

### Exemple 22 : : 2-[3-(4-Chloro-1H-pyrrolo[2,3-b]pyridin-2-yl)-5,6-diméthoxy-pyrrolo[3,2-b]pyridin-1-yl]-1-(4-méthyl-pipérazin-1-yl)-éthanone

Le 2-[3-(4-chloro-1H-pyrrolo[2,3-b]pyridin-2-yl)-5,6-diméthoxy-pyrrolo[3,2-b]pyridin-1-yl]-1-(4-méthyl-pipérazin-1-yl)-éthanone est préparé de la manière suivante:

A une solution de 0,15 g de 3-(4-chloro-1H-pyrrolo[2,3-b]pyridin-2-yl)-5,6-diméthoxy-pyrrolo[3,2-b]pyridin-1-yl]- acétate de potassium dans 4 cm³ de diméthylformamide, sous atmosphère inerte d'argon à une température voisine de 20°C, on ajoute 0,146 g de O-(1H-benzotriazol-1-yl)-N,N,N',N'-tétraméthyluronium hexafluorophosphate. Le milieu réactionnel est agité à cette même température pendant 1 heure. 0,046 g de 1-méthylpipérazine puis 0,067 cm³ de diisopropyléthylamine sont ajoutés. Après 16 heures d'agitation à la même température, 20 cm³ d'eau et 40 cm³ de dichlorométhane sont additionnés. Après décantation, la phase organique est séchée sur sulfate de magnésium, filtrée puis concentrée sous pression réduite. Le résidu obtenu est purifié par flash chromatographie (silice, dichlorométhane/méthanol 95/05 en volumes). Les fractions contenant le produit sont concentrées sous pression réduite. On obtient ainsi 0,0647 g de 2-[3-(4-chloro-1H-pyrrolo[2,3-b]pyridin-2-yl)-5,6-diméthoxy-pyrrolo[3,2-b)pyridin-1-yl]-1-(4-méthyl-pipérazin-1-yl)-éthanone dont les caractéristiques sont les suivantes:
- Spectre de Masse: ES m/z=469 MH⁺ pic de base ; m/z=235 (M + 2H)²⁺ / 2
- Spectre RMN 1H (300MHz)- δ en ppm- dans le DMSO-d6: 2,22 (s, 3H) ; 2,31 (m, 2H) ; 2,42 (m, 2H) ; 3,48 (m, 2H) ; 3,59 (m, 2H) ; 3,85 (s, 3H) ; 4,06 (s, 3H) ; 5,25 (s, 2H) ; 7,12 (d, J = 5,5 Hz, 1H) ; 7,28 (d, J = 2,0 Hz, 1H) ; 7,55 (s, 1H) ; 7,89 (s, 1H) ; 8,05 (d, J = 5, 5 Hz, 1H) ; 12,05 (s large, 1H)

### Exemple 23 : 2-[3-(4-Chloro-1H-pyrrolo[2,3-b]pyridin-2-yl)-5,6-diméthoxy-pyrrolo[3,2-b]pyridin-1-yl]-1-(4-hydroxy-pipéridin-1-yl)-éthanone

Le 2-[3-(4-chloro-1H-pyrrolo[2,3-b]pyridin-2-yl)-5,6-diméthoxy-pyrrolo[3,2-b]pyridin-1-yl]-1-(4-hydroxy-pipéridin-1-yl)-éthanone peut être préparé comme dans l'exemple 22 mais à partir de 0,15 g de 3-(4-chloro-1H-pyrrolo[2,3-b]pyridin-2-yl)-5,6-diméthoxy-pyrrolo[3,2-b]pyridin-1-yl)-acétate de potassium dans 4 cm³ de diméthylformamide, et 0,146 g de O-(1H-benzotriazol-1-yl)-N,N,N',N'-tétraméthyluronium hexafluorophosphate, et 0,046 g de 4-hydroxypipérazine. On obtient ainsi 0,0546 g de 2-[3-4-chloro-1H-pyrrolo[2,3-b]pyridin-2-yl)-5,6-diméthoxy-pyrrolo[3,2-b]pyridin-1-yl]-1-(4-hydroxy-pipéridin-1-yl)-éthanone dont les caractéristiques sont les suivantes:
- Spectre de Masse: IE m/z=469 M^{+.} pic de base ; m/z=341 (M - C₆H₁₀NO₂)^{+.}
- Spectre RMN 1H (300MHz)- δ en ppm- dans le DMSO-d6: De 1,22 à 1,55 (m, 2H) ; de 1,69 à 1,92 (m, 2H) ; 3,11 (m, 1H) ; de 3,25 à 3,38 (m partiellement masqué, 1H) ; de 3,72 à 3,94 (m, 3H) ; 3,84 (s, 3H) ; 4,06 (s, 3H) ; 4,78 (d, J = 4,0 Hz, 1H) ; 5,24 (m, 2H) ; 7,12 (d, J = 5,5 Hz, 1H) ; 7,28 (d, J = 2, 0 Hz, 1H) ; 7,55 (s, 1H) ; 7,90 (s, 1H) ; 8,04 (d, J = 5, 5 Hz, 1H) ; 12,05 (s large, 1H).

### Exemple 24: 2-(3-(4-Chloro-1H-pyrrolo[2,3-b]pyridin-2-yl)-5,6-diméthoxy-pyrrolo[3,2-b]pyridin-1-yl]-1-morpholin-4-yl-éthanone

Le 2-[3-(4-chloro-1H-pyrrolo[2,3-b]pyridin-2-yl)-5,6-diméthoxy-pyrrolo[3,2-b]pyridin-1-yl]-1-morpholin-4-yl-éthanone peut être préparé comme dans l'exemple 22 mais à partir de 0,15 g de 3-(4-chloro-1H-pyrrolo[2,3-b]pyridin-2-yl)-5,6-diméthoxy- pyrrolo[3,2-b]pyridin-1-yl]-acétate de potassium dans 4 cm³ de diméthylformamide,, et 0,146 g de O-(1H-benzotriazol-1-yl)-N,N,N',N'-tétraméthyluronium hexafluorophosphate, et 0,040 g de morpholine. Le milieu réactionnel est agité à cette même température pendant 1 heure. 0,046 g de 1-méthylpipérazine. On obtient ainsi 0,0612 g de 2-[3-(4-chloro-1H-pyrrolo[2,3-b]pyridin-2-yl)-5,6-diméthoxy-pyrrolo[3,2-b]pyridin-1-yl]-1-morpholin-4-yl-éthanone dont les caractéristiques sont les suivantes:
- Spectre de Masse: ES m/z=456 MH⁺ pic de base
- Spectre RMN 1H (300MHz) - δ en ppm- dans le DMSO-d6: 3,48 (m, 2H) ; 3,61 (m, 4H) ; 3,71 (m, 2H) ; 3,85 (s, 3H) ; 4,06 (s, 3H) ; 5,27 (s, 2H) ; 7,13 (d, J = 5,5 Hz, 1H) ; 7, 28 (d, J = 2,0 Hz, 1H) ; 7,56 (s, 1H) ; 7,89 (s, 1H) ; 8,05 (d, J = 5,5 Hz, 1H) ; 12,1 (s large, 1H)

### Exemple 25: 2-(1-{3-[3-(4-Chloro-1H-pyrrolo[2,3-b]pyridin-2-yl)-5,6-diméthoxy-pyrrolo[3,2-b]pyridin-1-yl]-propyl}-pipéridin-4-yl)-éthanol

### Stade 25 (a)_: 1-(3-chloro-propyl) -3-[4-chloro-1- (toluène-4-sulfonyl)-1H-pyrrolo[2,3-b]pyridin-2-yl]-5,6-dimethoxy-1H-pyrrolo[3,2-b]pyridine

Le 1-(3-chloro-propyl)-3-[4-chloro-1-(toluène-4-sulfonyl)-1H-pyrrolo[2,3-b]pyridin-2-yl]-5,6-diméthoxy-1H-pyrrolo[3,2-b]pyridine peut être préparé comme dans l'exemple 5 stade 5(a) mais à partir de 1,5 g 3-(4-chloro-1-(toluène-4-sulfonyl)-1H-pyrrolo[2,3-b]pyridin-2-yl)-5,6-diméthoxy-1H-pyrrolo[3,2-b]pyridine, de 75 cm³ de 1,3-bromochloropropane, de 1,37 g de potasse et 1 g de carbonate de potassium et 0,02 g de bromure de tétrabutylammonium. On obtient ainsi 1,69 g de 1-(3-chloro-propyl)-3-[4-chloro-1-(toluène-4-sulfonyl)-1H-pyrrolo[2,3-b]pyridin-2-yl]-5,6-diméthoxy-1H-pyrrolo[3,2-b]pyridine dont les caractéristiques sont les suivantes:
- Rf CCM silice [éluant: cyclohexane/acétate d'éthyle (50/50 en volumes)]= 0,57
- Spectre de Masse: ES m/z=559 MH⁺ pic de base

### Stade 25 (b): 1-(3-iodo-propyl)-3-[4-chloro-1-(toluène-4-sulfonyl)-1H-pyrrolo[2,3-b]pyridin-2-yl]-5,6-diméthoxy-1H-pyrrolo[3,2-b]pyridine

Le 1-(3-iodo-propyl)-3-[4-chloro-1-(toluène-4-sulfonyl)-1H-pyrrolo[2,3-b]pyridin-2-yl]-5,6-diméthoxy-1H-pyrrolo[3,2-b]pyridine peut être préparé comme dans l'exemple 5 stade 5(b) mais à partir de 1,65 g de 1-(3-chloro-propyl)-3-[4-chloro-1-(toluène-4-sulfonyl)-1H-pyrrolo[2,3-b]pyridin-2-yl]-5,6-diméthoxy-1H-pyrrolo[3,2-b]pyridine et 0,663g d'iodure de sodium dans 150 cm³ de méthyléthylcétone. On obtient ainsi 2 g de 1-(3-iodo-propyl)-3-[4-chloro-1-(toluène-4-sulfonyl)-1H-pyrrolo[2,3-b]pyridin-2-yl]-5,6-diméthoxy-1H-pyrrolo[3,2-b]pyridine dont les caractéristiques sont les suivantes:
- Rf CCM silice [éluant: cyclohexane/acétate d'éthyle (75/25 en volumes)]= 0,21
- Spectre de Masse: IE m/z=650 M^{+.} pic de base; m/z=495 (M - C₇H₇SO₂)⁺; m/z=368 (m/z=495 - I)^{+.}; m/z=91 C₇H₇⁺

### Stade 25 (c) : 2-[1-(3-{3-(4-Chloro-1-(toluène-4-sulfonyl)-1H-pyrrolo[2,3-b]pyridin-2-yl]-5,6-diméthoxy-pyrrolo[3,2-b]pyridin-1-yl}-propyl)-pipéridin-4-yl]-éthanol

Le 2-[1-(3-{3-[4-chloro-1-(toluène-4-sulfonyl)-1H-pyrrolo[2,3-b]pyridin-2-yl]-5,6-diméthoxy-pyrrolo[3,2-b]pyridin-1-yl}-propyl)-pipéridin-4-yl]-éthanol peut être préparé comme dans l'exemple 5 stade 5(c) mais à partir de 0, 5 g de 1-(3-iodo-propyl)-3-[4-chloro-1-(toluène-4-sulfonyl)-1H-pyrrolo[2,3-b]pyridin-2-yl]-5,6-diméthoxy-1H-pyrrolo[3,2-b]pyridine et 0,298 g de 4-pipéridineéthanol et 0,318 g de carbonate de potassium dans 20 cm³ de diméthylformamide. On obtient ainsi 0,405 g de 2-[1-(3-(3-[4-chloro-1-(toluène-4-sulfonyl)-1H-pyrrolo[2,3-blpyridin-2-yl]-5,6-diméthoxy-pyrrolo[3,2-b]pyridin-1-yl}-propyl)-pipéridin-4-yl]-éthanol dont les caractéristiques sont les suivantes:
- Rf CCM silice [éluant: dichlorométhane/méthanol (90/10 en volumes)]= 0,19
- Spectre de Masse: IE m/z=651 M^{+.} ; m/z=496 (M - C₇H₇SO₂)⁺ ; m/z=342 (m/z=496 - C₉H₁₆NO)^{+.} pic de base ; m/z=142 C₉H₁₆NO⁺

### Stade 25 (d) : 2-(1-{3-[3-(4-Chloro-1H-pyrrolo[2,3-b]pyridin-2-yl) -5,6-dimethoxy-pyrrolo[3,2-b]pyrimdin-1-yl]-propyl}-pipéridin-4-yl)-éthanol

Le 2-(1-{3-[3-(4-chloro-1H-pyrrolo[2,3-b]pyridin-2-yl)-5,6-diméthoxy-pyrrolo[3,2-b]pyridin-1-yl]-propyl}-pipéridin-4-yl)-éthanol peut être préparé comme dans l'exemple 5 stade 5 (d) mais à partir de 0,4 g de 2-[1-(3-{3-[4-chloro-1-(toluène-4-sulfonyl)-1H-pyrrolo[2,3-b]pyridin-2-yl]-5,6-diméthoxy-pyrrolo[3,2-b]pyridin-1-yl}-propyl)-pipéridin-4-yl]-éthanol et 0,89 g de potasse dans 25 cm³ de méthanol. On obtient ainsi 0,22 g de 2-(1-{3-[3-(4-chloro-1H-pyrrolo[2,3-b]pyridin-2-yl)-5,6-diméthoxy-pyrrolo[3,2-b]pyridin-1-yl)-propyl}-pipéridin-4-yl)-éthanol dont les caractéristiques sont les suivantes:
- Spectre RMN 1H (300MHz )- δ en ppm- dans le BMSO-d.6: De 1,08 à 1,24 (m, 2H) ; de 1,28 à 1,41 (m, 3H) ; 1,61 (m, 2H) ; 1,81 (m, 2H) ; 1,95 (m, 2H) ; 2,21 (t, J = 6,5 Hz, 2H) ; 2,79 (m, 2H) ; 3,42 (m, 2H) ; 3,88 (s, 3H) ; 4,05 (s, 3H) ; 4,23 (t, J = 6,5 Hz, 2H) ; 4, 29 (t, J = 5,5 Hz, 1H) ; 7,12 (d, J = 5,5 Hz, 1H) ; 7,22 (s large, 1H) ; 7,59 (s, 1H) ; 8,02 (s, 1H) ; 8,06 (d, J = 5,5 Hz, 1.H) ; 12,05 (s large, 1H)
- Spectre de Masse: IE m/z=497 M^{+.} ; m/z=342 (M C₉H₁₇NO) + pic de base ; m/z=1142 C₈H₁₆NO⁺

### Exemple 26 : 1-{3-[3-(4-Chloro-1H-pyrrolo[2,3-b]pyrodin-2-yl) -5,6-diméthoxy-pyrrolo [3,2-b]pyridin-1-yl]-propyl}-pipéridin-4-ol

### Stade 26 (a) : 1-(3-{3-[4-Chloro-1-(toluéne-4-sulfonyl)-1H-pyrrolo[2,3-b]pyridin-2-yl]-5,6-diméthoxy-pyrrolo[3,2-b]pyridin-1-yl}-propyl)-pipéridin-4-ol

Le 1-(3-{3-[4-chloro-1-(toluéne-4-sulfonyl)-1H-pyrrolo[2,3-b]pyridin-2-yl]-5,6-diméthoxy-pyrrolo[3,2-b]pyridin-1-yl}-propyl)-pipéridin-4-ol peut être préparé comme dans l'exemple 5 stade 5(c) mais à partir de 0,5 g de 1-(3-iodo-propyl)-3-[4-chloro-1-(toluène-4-sulfonyl) - 1H-pyrrolo[2,3-b]pyridin-2-yl]-5,6-diméthoxy-1H-pyrrolo[3,2-b]pyridine et 0,233 g de 4-hydroxypipéridine et 0,318 g de carbonate de potassium dans 20 cm³ de diméthylformamide. On obtient ainsi 0,565 g de 1-(3-{3-[4-chloro-1-(toluène-4-sulfonyl)-1H-pyrrolo[2,3-b]pyridin-2-yl]-5,6-diméthoxy-pyrrolo[3,2-b]pyridin-1-yl}-propyl)-pipéridin-4-ol dont les caractéristiques sont les suivantes:
- Rf CCM silice [éluant: dichlorométhane/méthanol (90/10 en volumes)]= 0,17
- Spectre de Masse.: IE m/z=623 M⁺ ; m/z=496 (M - C₇H₁₃NO)⁺ ; m/z=341 (m/z=496 - C₇H₇SO₂)⁺ pic de base ; m/z=114 C₆H₁₂NO⁺

### Stade 26(b) : 1-{3-[3-(4-Chloro-1H-pyrrolo[2,3-b]pyridin-2-yl)-5,6-diméthoxy-pyrrolo[3,2-b]pyridin-1-yl]-propyl}-pipéridin-4-ol

Le 1-{3-[3-(4-chloro-1H-pyrrolo[2,3-b]pyridin-2-yl)-5,6-diméthoxy-pyrrolo[3,2-b]pyridin-1-yl]-propyl}-pipéridin-4-ol peut être préparé comme dans l'exemple 5 stade 5 (d) mais à partir de 0,368 g de 1-(3-{3-[4-Chloro-1-(toluène-4-sulfonyl)-1H-pyrrolo[2,3-b]pyridin-2-yl]-5,6-diméthoxy-pyrrolo[3,2-b]pyridin-1-yl)-propyl)-pipéridin-4-ol et 0,857 g de potasse dans 25 cm³ de méthanol. On obtient ainsi 0,188 g de 2-(1-{3-[3-(4-chloro-1H-pyrrolo[2,3-b]pyridin-2-yl)-5,6-diméthoxy-pyrrolo[3,2-b]pyridin-1-yl]-propyl}-pipéridin-4-yl)-éthanol dont les caractéristiques sont les suivantes:
- Spectre RMN 1H (300MHz) - δ en ppm- dans le DMSO-d6: 1,42 (m, 2H) ; 1,72 (m, 2H) ; de 1,88 à 2,02 (m, 4H) ; 2,21 (t, J = 6,5 Hz, 2H) ; 2,66 (m, 2H) ; 3,43 (m, 1H) ; 3,88 (s, 3H) ; 4,05 (s, 3H) ; 4,23 (t, J = 6,5 Hz, 2H) ; 4,52 (d, J = 3,5 Hz, 1H) ; 7,12 (d, J = 5,5 Hz, 1H) ; 7,22 (d, J = 2,0 Hz, 1H) ; 7,58 (s, 1H) ; 8,03 (s, 1H) ; 8,05 (d, J = 5,5 Hz, 2H) ; 12,05 (s large, 1H).
- Spectre de Masse: IE m/z=469 M^{+.} ; m/z=342 (M -
C₇H₁₃NO)⁺ pic de base ; m/z=114 C₆H₁₂NO⁺

### Exemple 27 : {3-[3-(4-Chloro-1H-pyrrolo[2,3-b]pyridin-2-yl)-5,6-diméthoxy-pyrrolo[3,2-b]pyridin-1-yl]-propyl}-diéthyl-amine

### Stade 27 (a): (3-{3-[4-Chloro-1-(toluène-4-sulfonyl)-1H-pyrrolo[2,3-b]pyridin-2-yl]-5,6-diméthoxy-pyrrolo[3,2-b]pyridin-1-yl}-propyl)-diéthyl-amine

Le (3-{3-[4-chloro-1-(toluène-4-sulfonyl)-1H-pyrrolo[2,3-b]pyridin-2-yl]-5,6-diméthoxy-pyrrolo[3,2-b]pyridin-1-yl}-propyl)-diéthyl-amine peut être préparé comme dans l'exemple 5 stade 5(c) mais à partir de 0,15 g de 1-(3-iodo-propyl)-3-[4-chloro-1-(toluène-4-sulfonyl)-1H-pyrrolo[2,3-b]pyridin-2-yl]-5,6-diméthoxy-1H-pyrrolo[3,2-b]pyridine et 0,101 g de diéthylamine dans 3 cm³ de diméthylformamide. On obtient ainsi 0,11 g de (3-{3-[4-chloro-1-(toluène-4-sulfonyl)-1H-pyrrolo[2,3-b]pyridin-2-yl]-5,6-diméthoxy-pyrrolo[3,2-b]pyridin-1-yl}-propyl)-diéthyl-amine dont les caractéristiques sont les suivantes:
- Spectre de Masse: IE m/z=595 M^{+.}; m/z=496 (M - C₆H₁₃N)⁺ ; m/z=341 (m/z=496 - C₇H₇SO₂)⁺ pic de base ; m/z=86 C₅H₁₂N⁺

### Stade 27 (b) : {3-[3-(4-Chloro-1H-pyrrolo[2,3-b]pyridin-2-yl)-5,6-diméthoxy-pyrrolo[3,2-b]pyridin-1-yl]-propyl}-diéthyl-amine

Le {3-[3-(4-chloro-1H-pyrrolo[2,3-b]pyridin-2-yl)-5,6-diméthoxy-pyrrolo[3,2-b]pyridin-1-yl]-propyl}-diéthylamine peut être préparé comme dans l'exemple 5 stade 5(d) mais à partir de 0,11 g de (3-{3-[4-chloro-1-(toluène-4-sulfonyl)-1H-pyrrolo[2,3-b]pyridin-2-yl]-5,6-diméthoxy-pyrrolo[3,2-b]pyridin-1-yl}-propyl)-diéthyl-amine et 0,228 g de potasse dans 30 cm³ de méthanol. On obtient ainsi 0,06 g de 3-[3-(4-chloro-1H-pyrrolo[2,3-b]pyridin-2-yl)-5,6-diméthoxy-pyrrolo[3,2-b]pyridin-1-yl]-propyl}-diéthyl-amine dont les caractéristiques sont les suivantes:
- Spectre RMN 1H (300MHz )- δ en ppm- dans le DMSO-d6: 0,93 (t, J = 7,0 Hz, 6H) ; 1,93 (m, 2H) ; 2,37 (t, J = 7,0 Hz, 2H) ; 2,45 (q partiellement masqué, J = 7,0 Hz, 4H) ; 3,87 (s, 3H) ; 4,05 (s, 3H) ; 4,23 (t, J = 7,0 Hz, 2H) ; 7,12 (d, J = 5,5 Hz, 1H) ; 7,24 (s large, 1H) ; 7,59 (s, 1H) ; 8,04 (s, 1H) ; 8,05 (d, J = 5,5 Hz, 1H) ; 12,1 (s large, 1H).
- Spectre de Masse: IE m/z=441 M^{+.} ; m/z=342 (M - C₆H₁₃N)⁺ pic de base ; m/z=86 C₅H₁₂N⁺

### Exemple 29 : 3-(4-Chloro-1H-pyrrolo[2,3-b]pyridin-2-yl)-5,6-diméthoxy-1-[3-(4-méthyl-perhydro-1,4-diazépin-1-yl)-propyl]-1H-pyrrolo[3,2-b]pyridine

### Stade 29 (a): 3-[4-Chloro-1-(toluéne-4-sulfonyl)-1H-pyrrolo[2,3-b]pyridin-2-yl]-5,6-diméthoxy-1-[3-(4-méthylperhydro-1,4-diazépin-1-yl)-propyl]-1H-pyrrolo[3,2-b]pyridina

Le 3-[4-chloro-1-(toluène-4-sulfonyl)-1H-pyrrolo[2,3-b]pyridin-2-yl]-5,6-diméthoxy-1-[3-(4-méthyl-perhydro-1,4-diazépin-1-yl)-propyl]-1H-pyrrolo[3,2-b]pyridine peut être préparé comme dans l'exemple 5 stade 5 (c) mais à partir de 0,835 g de 1-(3-iodo-propyl)-3-[4-chloro-1-(toluène-4-sulfonyl)-1H-pyrrolo[2,3-b]pyridin-2-yl]-5,6-diméthoxy-1H-pyrrolo[3,2-b]pyridine et 1,59cm³ (12, 8 mmol) de 1-méthyl-perhydro-1,4-diazépine dans 40 cm³ de dichlorométhane. On obtient ainsi 0,32 g de 3-[4-chloro-1-(toluène-4-sulfonyl)-1H-pyrrolo[2,3-b]pyridin-2-yl]-5,6-diméthoxy-1-[3-(4-méthyl-perhydro-1,4-diazépin-1-yl)-propyl]-1H-pyrrolo[3,2-b]pyridine dont les caractéristiques sont les suivantes:
- Rf CCM silice [éluant: dichlorométhane/méthanol (90/10 en volumes)]= 0,2
- Spectre de Masse: ES m/z=637 MH⁺ pic de base

### Stade 29(b): 3-(4-Chloro-1H-pyrrolo[2,3-b]pyridin-2-yl)-5,6-diméthoxy-1-[3-(4-méthyl-perhydro-1,4-diazépin-1-yl)-propyl]-1H-pyrrolo[3,2-b]pyridine

Le 3-(4-chloro-1H-pyrrolo[2,3-b]pyridin-2-yl)-5,6-diméthoxy-1-[3-(4-méthyl-perhydro-1,4-diazépin-1-yl)-propyl]-1H-pyrrolo[3,2-b]pyridine peut être préparé comme dans l'exemple 5 stade 5(d) mais à partir de 0,32 g de 3-[4-chloro-1-(toluène-4-sulfonyl)-1H-pyrrolo[2,3-b]pyridin-2-yl]-5,6-diméthoxy-1-[3-(4-méthyl-perhydro-1,4-diazépin-1-yl)-propyl]-1H-pyrrolo[3,2-b]pyridine et 0,62 g de potasse dans 25 cm³ de méthanol. On obtient ainsi 0,015 g de 3-(4-chloro-1H-pyrrolo[2,3-b]pyridin-2-yl)-5,6-diméthoxy-1-[3-(4-méthyl-perhydro-1,4-diazépin-1-yl)-propyl]-1H-pyrrolo[3,2-b]pyridine dont les caractéristiques sont les suivantes:
- Spectre RMN 1H (300MHz)- □ en ppm- dans le DMSO-d6: 1,70 (m, 2H) ; 1,93 (m, 2H) ; 2,23 (s, 3H) ; 2,39 (t large, J = 6,5 Hz, 2H) ; de 2,43 à 2,55 (m partiellement masqué, 4H) ; 2,60 (m, 4H) ; 3,88 (s, 3H) ; 4,05 (s, 3H) ; 4,24 (t large, J = 6,5 Hz, 2H) ; 7,12 (d, J = 5,5 Hz, 1H) ; 7,24 (s large, 1H) ; 7,58 (s, 1H) ; 8,02 (s, 1H) ; 8,05 (d, J = 5,5 Hz, 1H) ; 12,05 (s large, 1H) .
- Spectre de Masse: ES m/z=483 MH+ ; m/z=242 (M + 2H)2+ / 2 pic de base

### Exemple 30: 3-(4-Chloro-1H-pyrrolo[2,3-b]pyridin-2-yl)-5,6-diméthoxy-1-(3-pipéridin-1-yl-propyl)-1H-pyrrolo[3,2-b]pyridine

### Stade 30 (a) : 3-[4-Chloro-1-(toluéne-4-sulfonyl)-1H-pyrrolo[2,3-b]pyridin-2-yl]-5,6-diméthoxy-1-(3-pipéridin-1-yl-propyl) -1H-pyrrolo[3,2-b]pyridine

Le 3-[4-chloro-1-(toluène-4-sulfonyl)-1H-pyrrolo[2,3-b]pyridin-2-yl]-5,6-diméthoxy-1-(3-pipéridin-1-yl-propyl)-1H-pyrrolo[3,2-b]pyridine peut être préparé comme dans l'exemple 5 stade 5(c) mais à partir de 0,835 g de 1-(3-iodo-propyl)-3-[4-chloro-1-(toluène-4-sulfonyl)-1H-pyrrolo[2,3-b]pyridin-2-yl]-5,6-diméthoxy-1H-pyrrolo[3,2-b]pyridine et 1,26cm³ de pipéridine dans 40 cm³ de dichlorométhane. On obtient ainsi 0,39 g de 3-[4-chloro-1-(toluène-4-sulfonyl)-1H-pyrrolo[2,3-b]pyridin-2-yl]-5,6-diméthoxy-1-(3-pipéridin-1-yl-propyl)-1H-pyrrolo[3,2-b]pyridine dont les caractéristiques sont les suivantes:
- Rf CCM silice [éluant: dichlorométhane/méthanol (90/10 en volumes)]= 0,24
- Spectre de Masse: IE m/z=607 M^{+.} ; m/z=496 (M - C₇H₁₃N)⁺ ; m/z=452 (M - C₇H₇SO₂)⁺ ; m/z=341 (m/z=496 - C₇H₇SO₂)⁺ pic de base ; m/z=98 C₆H₁₂N⁺

### stade 30 (b): 3-(4-Chloro-1H-pyrrolo[2,3-b]pyridin-2-yl)-5,6-diméthoxy-1-(3-piperidin-1-yl-propyl)-1H-pyrrolo[3,2-b]pyridine

Le 3-(4-chloro-1H-pyrrolo[2,3-b]pyridin-2-yl)-5,6-diméthoxy-1-(3-pipéridin-1-yl-propyl)-1H-pyrrolo[3,2-b]pyridine peut être préparé comme dans l'exemple 5 stade 5(d) mais à partir de 0,39 g de 3-[4-chloro-1-(toluène-4-sulfonyl)-1H-pyrrolo[2,3-b]pyridin-2-yl]-5,6-diméthoxy-1-(3-pipéridin-1-yl-propyl)-1H-pyrrolo[3,2-b]pyridine et 0,792 g de potasse dans 25 cm³ de méthanol. On obtient ainsi 0,035 g de 3-(4-chloro-1H-pyrrolo[2,3-b]pyridin-2-yl)-5,6-diméthoxy-1-(3-pipéridin-1-yl-propyl)-1H-pyrrolo[3,2-b]pyridine dont les caractéristiques sont les suivantes:
- Spectre RMN 1H (300MHz )- δ en ppm- dans le DMSO-d6: 1,38 (m, 2H) ; 1,51 (m, 4H) ; 1,95 (m, 2H) ; 2,20 (t, J = 7,0 Hz, 2H) ; 2,28 (m large, 4H) ; 3,88 (s, 3H) ; 4,05 (s, 3H) ; 4,23 (t, J = 7,0 Hz, 2H) ; 7,12 (d, J = 5,5 Hz, 1H) ; 7,24 (d, J = 2,0 Hz, 1H) ; 7,59 (s, 1H) ; 8,03 (s, 1H) ; 8,05 (d, J = 5,5 Hz, 1H) ; 12,05 (s large, 1H).
- Spectre de Masse: ES m/z=454 MH⁺ ; m/z=227,8 (M + 2H)²⁺ / 2 pic de base

### Exemple 31: 2-(5,6-Diméthoxy-1H-pyrrolo[3,2-b]pyridin-3-yl)-1H-pyrrolo[2,3-b]pyridine-4-carbonitrile

### Stade 31(a): 1-(Toluéne-4-sulfonyl)-2-tributylstannanyl-1H-pyrrolo[2,3-b]pyridine-4-carbonitrile

Le 1-(toluène-4-sulfonyl)-2-tributylstannanyl-1H-pyrrolo[2,3-b]pyridine-4-carbonitrile peut être préparé comme dans l'exemple 4 stade 4 (d) mais à partir de 0,5 g 1-(toluène-4-sulfonyl)-1H-pyrrolo[2,3-b]pyridine-4-carbonitrile, 1,2cm³ de n-butyl lithium et 0,52cm³ de chlorure de tributyl étain dans 30 cm³ de tétrahydrofurane. On obtient ainsi 0,56 g de 1-(toluène-4-sulfonyl)-2-tributylstannanyl-1H-pyrrolo[2,3-b]pyridine-4-carbonitrile dont les caractéristiques sont les suivantes:
- Rf CCM silice [éluant: cyclohexane/acétate d'éthyle (75/25 en volumes)]= 0,57
- Spectre de Masse: IE m/z=586 M^{+.} m/z=530 (M - C₄H₈)^{+.} pic de base ; m/z=416 (m/z=530 - 2C₄H₉)^{+.} ; m/z=262 (m/z=416 - C₇H₇SO₂ + H)^{+.}

Le 1-(toluène-4-sulfonyl)-1H-pyrrolo[2,3-b]pyridine-4-carbonitrile est préparé comme décrit dans le brevet WO0147922A2.

### stade 31(b): ester tert-butylique de l'acide 3-[4-Cyano-1-(toluène-4-sulfonyl)-1H-pyrrolo[2,3-b]pyridin-2-yl]-5,6-diméthoxy-pyrrolo[3,2-b]pyridine-1-carboxylique

L' ester tert-butylique de l'acide 3-[4-cyano-1-(toluène-4-sulfonyl)-1H-pyrrolo[2,3-b]pyridin-2-yl]-5,6-diméthoxy-pyrrolo[3,2-b]pyridine-1-carboxylique peut être préparé comme dans l'exemple 4 stade 4(e) mais à partir de 0,56 g de 1-(toluène-4-sulfonyl)-2-tributylstannanyl-1H-pyrrolo[2,3-b]pyridine-4-carbonitrile, 0,35 g 4-chloro-1-(toluène-4-sulfonyl)-2-tributylstannanyl-1H-pyrrolo[2,3-b]pyridine, 0,0166 g d'iodure de cuivre et 0,1g de tétrakis (triphénylphosphine) palladium dans 30 cm³ de toluène. On obtient ainsi 0,11 g d'ester tert-butylique de l'acide-3-[4-cyano-1-(toluène-4-sulfonyl)-1H-pyrrolo[2,3-b]pyridin-2-yl]-5,6-diméthoxy-pyrrolo[3,2-b]pyridine-1-carboxylique dont les caractéristiques sont les suivantes:
- Rf CCM silice [éluant: dichlorométhane] = 0,29
- Spectre de Masse: ES m/z=574 MH⁺ pic de base

### Stade 31 (c): 2-(5,6-Diméthoxy-1H-pyrrolo[3,2-b]pyridin-3-yl)-1-(toluène-4-sulfonyl)-1H-pyrrolo[2,3-b]pyridine-4-carbonitrile

Le 2-(5,6-diméthoxy-1H-pyrrolo[3,2-b]pyridin-3-yl)-1-(toluène-4-sulfonyl)-1H-pyrrolo[2,3-b]pyridine-4-carbonitrile peut être préparé comme dans l'exemple 1 stade 1 (m) mais à partir de 0,11 g d'ester tert-butylique de l'acide-3-[4-cyano-1-(toluène-4-sulfonyl)-1H-pyrrolo[2,3-b]pyridin-2-yl]-5,6-diméthoxy-pyrrolo[3,2-b]pyridine-1-carboxylique et 1,1 cm³ d'acide trifluoroacétique dans 25 cm³ de méthanol dans 5 cm³ de dichlorométhane. On obtient ainsi 0,06 g 2-(5,6-diméthoxy-1H-pyrrolo[3,2-b]pyridin-3-yl)-1-(toluène-4-sulfonyl)-1H-pyrrolo[2,3-b]pyridine-4-carbonitrile dont les caractéristiques sont les suivantes:
- Rf CCM silice [éluant: dichlorométhane/méthanol (95/5 en volumes)]= 0,4
- Spectre de Masse: ES m/z=474 MH+ pic de base

### Stade 31 (d): 2-(5,6-Diméthoxy-1H-pyrrolo[3,2-b]pyridin-3-yl)-1H-Pyrrolo[2,3-b]pyridin-4-carbonitrile

Le 2-(5,6-diméthoxy-1H-pyrrolo[3,2-b]pyridin-3-yl)-1H-pyrrolo[2,3-b]pyridine-4-carbonitrile peut être préparé comme dans l'exemple 5 stade 5(d) mais à partir de 0,06 g de 2-(5,6-diméthoxy-1H-pyrrolo[3,2-b]pyridin-3-yl)-1-(toluène-4-sulfonyl)-1H-pyrrolo[2,3-b]pyridine-4-carbonitrile et 0,156 g de potasse dans 5 cm³ de méthanol. On obtient ainsi 0,0195 g de 2-(5,6-diméthoxy-1H-pyrrolo[3,2-b]pyridin-3-yl)-1H-pyrrolo[2,3-b]pyridine-4-carbonitrile dont les caractéristiques sont les suivantes:
- Spectre RMN 1H (300MHz) - δ en ppm- dans le DMSO-d6: 3,85 (s, 3H) ; 4,06 (s, 3H) ; 7,38 (s, 1H) ; 7,41 (s, 1H) ; 7, 92 (d, J = 5,5 Hz, 1H) ; 8,09 (s, 1H) ; 8,23 (d, J = 5,5 Hz, 1H) ; 11,5 (m étalé, 1H) ; 12,4 (s large, 1H)
- Spectre de Masse: IE m/z=319 M^{+.} pic de base; m/z=304 (M - CH₃)⁺

### Exemple 32 : 2-(5,6-Diméthoxy-1-méthyl-3H-pyrrolo[3,2-b]pyridin-3-yl)-1E-pyrrolo[2,3-b]pyridine-4-carbonitrile

### Stade 32 (a): 2-(5,6-Dimethoxy-1-méthyl-1H-pyrrolo[3,2-b]pyridin-3-yl)-1-(toluène-4-sulfonyl)-1H-pyrrolo[2,3-b]pyridine-4-carbonitrile .

Le 2-(5,6-diméthoxy-1-méthyl-1H-pyrrolo[3,2-b]pyridin-3-yl)-1-(toluène-4-sulfonyl)-1H-pyrrolo[2,3-b]pyridine-4-carbonitrile peut être préparé comme dans l'exemple 1 stade 1(n) mais à partir de 0,1 g de 2-(5,6-diméthoxy-1H-pyrrolo[3,2-b]pyridin-3-yl)-1-(toluène-4-sulfonyl)-1H-pyrrolo[2,3-b]pyridine-4-carbonitrile, 0,010g d'hydrure de sodium et 0,016cm³ d'iodure de méthyle dans 5 cm³ de diméthylformamide. On obtient ainsi 0,09 g 2-(5,6-diméthoxy-1-méthyl-1H-pyrrolo[3,2-b]pyridin-3-yl)-1-(toluène-4-sulfonyl)-1H-pyrrolo[2,3-b]pyridine-4-carbonitrile dont les caractéristiques sont les suivantes:
- Rf CCM silice [éluant: dichlorométhane/méthanol (98/2 en volumes)]= 0,31
- Spectre de Masse: IE m/z=487 M^{+.} ; m/z=332 (M - C₇H₇SO₂)⁺ pic de base

### Stade 32 (b) : 2-(5,6-Diméthoxy-1-éthyl-1H-pyrrolo[3,2-b]pyridin-3-yl)-1H-pyrrolo[2,3-b]pyridine-4-carbonitrile

Le 2-(5,6-diméthoxy-1-méthyl-1H-pyrrolo[3,2-b]pyridin-3-yl)-1H-pyrrolo[2,3-b]pyridine-4-carbonitrile peut être préparé comme dans l'exemple 5 stade 5(d) mais à partir de 0,09 g de 2-(5,6-diméthoxy-1-méthyl-1H-pyrrolo[3,2-b]pyridin-3-yl)-1-(toluène-4-sulfonyl)-1H-pyrrolo[2,3-b]pyridine-4-carbonitrile et 0,228 g de potasse dans 5 cm³ de méthanol. On obtient ainsi 0,02 g de 2-(5,6-diméthoxy-1-méthyl-1H-pyrrolo[3,2-b]pyridin-3-yl)-1H-pyrrolo[2,3-b]pyridine-4-carbonitrile dont les caractéristiques sont les suivantes:
- Spectre RMN 1H (300MHz ) - δ en ppm- dans le DMSO-d6: 3,87 (s, 3H) ; 3,89 (s, 3H) ; 4,06 (s, 3H) ; 7,36 (s, 1H) ; 7,42 (d, J = 5,5 Hz, 1H) ; 7,63 (s, 1H) ; 8,03 (s, 1H) ; 8,23 (d, J = 5,5 Hz, 1H) ; 12, 4 (m étalé, 1H)
- Spectre de Masse: IE m/z=333 M^{+.} pic de base; m/z=318 (M - CH₃)⁺

### Exemple 33: 2-[3-(4-Chloro-1H-pyrrolo[2,3-b]pyridin-2-yl)-5,6-diméthoxy-pyrrolo[3,2-b]pyridin-1-yl]-N-méthoxy-N-méthyl-acétamide

2-[3-(4-chloro-1H-pyrrolo[2,3-b]pyridin-2-yl)-5,6-diméthoxy-pyrrolo[3,2-b]pyridin-1-yl]-N-méthoxy-N-méthyl-acétamide est préparé de la manière suivante:

A une solution de 1,8 g d'acide-{3-(4-chloro-1H-pyrrolo[2,3-b]pyridin-2-yl)-5,6-diméthoxy-pyrrolo[3,2-b]pyridin-1-yl]}-carboxylique dans 30 cm³ de diméthylformamide et 30 cm³ de 1-,2-dichloroéthane, à une température voisine de 20°C, on ajoute 0,692 g de 1-hydroxybenzotriazole et 0,981 g de chlorhydrate de 1-(diméthylaminopropyl)-3-éthylcarbodiimide. Le milieu réactionnel est agité à cette même température pendant 1 heure. 0,5 g de chlorhydrate de N,O-diméthylhydroxylamine puis 2,2 cm³ de triéthylamine sont ajoutés. Après 20 heures d'agitation à la même température, 100 cm³ de dichlorométhane sont additionnés, la phase organique est lavée par 3 fois 100 cm³ d'une solution aqueuse saturée en hydrogénocarbonate de sodium puis par 2 fois 100 cm³ d'eau. Après décantation, la phase organique est séchée sur sulfate de magnésium, filtrée puis concentrée sous pression réduite. Le résidu obtenu est purifié par flash chromatographie (silice, dichlorométhane/méthanol 98/02 en volumes). Les fractions contenant le produit sont concentrées sous pression réduite. On obtient ainsi 2,11 g d'un brut de 2-[3-(4-chloro-1H-pyrrolo[2,3-b]pyridin-2-yl)-5,6-diméthoxy-pyrrolo[3,2-b]pyridin-1-yl]-N-méthoxy-N-méthyl-acétamide. 0,1 g de ce solide est repris dans 2 cm³ de pentane puis filtré, essoré. Le gâteau est ensuite lavé par 2 fois 1 cm³ de pentane puis séché à l'étuve à 35°C pendant 8h. On obtient ainsi 0,062g de 2-[3-(4-chloro-1H-pyrrolo[2,3-b]pyridin-2-yl)-5,6-diméthoxy-pyrrolo[3,2-b]pyridin-1-yl]-N-méthoxy-N-méthyl-acétamide dont les caractéristiques sont les suivantes:
- Spectre RMN 1H (300MHz) - δ en ppm- dans le DMSO-d6: 3,18 (s large, 3H) ; 3, 85 (s, 3H) ; 3, 86 (s large, 3H) ; 4,05 (s, 3H) ; 5,29 (s large, 2H) ; 7,14 (d, J = 5,5 Hz, 1H) ; 7,27 (d, J = 2,0 Hz, 1H) ; 7,59 (s, 1H) ; 7,93 (s, 1H) ; 8,06 (d, J = 5,5 Hz, 1H) ; 12,1 (m large, 1H).
- Spectre de Masse: IE m/z=429 M^{+.} pic de base ; m/z=399 (M - OCH₃ + H)^{+. ;} m/z=341 (M - C₃H₆NO₂) ^{+.}

### Exemple 38 : 2-(5,6-diméthoxy-1-méthyl-1H-pyrrolo[3,2-b]pyridin-3-yl)-1H-pyrrolo[2,3-b]pyridin-4-carbaldéhyde:

Le produit est préparé en suivant le mode opératoire décrit à l'exemple 34 stade (k) à partir de 0,1 g du 2-(5,6-diméthoxy-1-méthyl-1H-pyrrolo[3,2-b]pyridin-3-yl)-1-(toluène-4-sulfonyl)-1H-pyrrolo[2,3-b]pyridin-4-carbaldéhyde au lieu du cyclopropyl-[2-(5,6-diméthoxy-1-méthyl-1H-pyrrolo[3,2-b]pyridin-3-yl)-1-(toluène-4-sulfonyl)-1H-pyrrolo[2,3-b]pyridin-4-ylméthyl]-amine utilisé à l'exemple 34 stade (k) et de 0,82 cm³ d'hydroxyde de potassium 5N. On obtient 0,1 g du 2-(5,6-diméthoxy-1-méthyl-1H-pyrrolo[3,2-b]pyridin-3-yl)-1H-pyrrolo[2,3-b]pyridin-4-carbaldéhyde dont les caractéristiques sont les suivantes :
- Spectre de R.M.N. ¹H (300 MHz, (CD₃)₂SO d6, δ en ppm) : 3,88 (s, 3H) ; 3,90 (s, 3H) ; 4,10 (s, 3H) ; 7,52 (d, J = 5,5 Hz, 1H) ; 7,61 (s, 1H) ; 7,84 (d, J = 2,0 Hz, 1H) ; 8,03 (s, 1H) ; 8,32 (d, J = 5,5 Hz, 1H) ; 10,35 (s, 3H) ; 12,15 (m large, 1H).
- Spectre de masse : IE m/z=336 M^{+.} ; pic de base

### Exemple 49 : 4-{[2-(5,6-Diméthoxy-1-méthyl-1H-pyrrolo[3,2-b]pyridin-3-yl)-1H-pyrrolo[2,3-b]pyridin-4-ylméthyl]-amino}-phénol :

A une solution de 0,085 g du 4-{[1-[2-(5,6-diméthoxy-1-méthyl-1H-pyrrolo[3,2-b]pyridin-3-yl)-1H-pyrrolo[2,3-b]pyridin-4-yl]-meth-(E)-ylidene]-amino}-phénol dans 20 cm³ de méthanol, à une température voisine de 20°C, est ajouté 0,013 g de chlorure de zinc et 0,013 g de cyanoborohydrure de sodium. Le milieu réactionnel est agité à la même température pendant 4 heures. Le milieu réactionnel est concentré sous pression réduite. Le résidu obtenu est repris avec 10 cm³ d'eau et 25 cm³ de dichlorométhane, puis alcalinisé avec de la soude 0,1N.
Après décantation, la phase organique est séchée sur sulfate de magnésium, filtrée puis concentrée sous pression réduite pour donner 0,04 g du 4-{[2-(5,6-diméthoxy-1-méthyl-1H-pyrrolo[3,2-b]pyridin-3-yl)-1H-pyrrolo[2,3-b]pyridin-4-ylméthyl]-amino}-phénol dont les caractéristiques sont les suivantes :
- Spectre de R.M.N. ¹H (300 MHz, (CD₃)₂SO d6, δ en ppm) : 3,85 (s, 3H) ; 3,88 (s, 3H) ; 4,02 (s, 3H) ; 4,48 (d large, J = 6,0 Hz, 2H) ; 5,71 (t large, J = 6,0 Hz, 1H) ; 6,43 (d large, J = 9,0 Hz, 2H) ; 6,49 (d large, J = 9,0 Hz, 2H) ; 6,98 (d, J = 5,5 Hz, 1H) ; 7,32 (d, J = 2,0 Hz, 1H) ; 7,59 (s, 1H) ; 7,89 (s, 1H) ; 8,01 (d, J = 5,5 Hz, 1H) ; 8,37 (s, 1H) ; 11,7 (m large, 1H).
- Spectre de masse : ES m/z=430 MH⁺ ; pic de base

### EXEMPLE 67: COMPOSITION PHARMACEUTIQUE

On a préparé des comprimés répondant à la formule suivante :

| | |
|---|---|
| Produit de l'exemple 8 | 0,2 g |
| Excipient pour un comprimé terminé à | 1 g |

(détail de l'excipient : lactose, talc, amidon, stéarate de magnésium).

La composition pharmaceutique ci-dessus qui constitue l'exemple 67 de la présente invention peut être préparée de la même manière avec les autres produits de formule (I) selon la présente invention et notamment avec les produits en exemples dans la partie expérimentale ci-dessus.

## Revendications

1. Produits de formule (I) 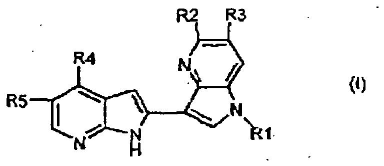 choisis parmi les composés suivants :
3 - (4-chloro-1H-pyrrolo[2,3-b]-pyridin-2-yl)-5,6-diméthoxy-1-methyl-1H-pyrrolo [3,2-b]-pyridine,
3 - (4-Chloro-1H-pyrrolo [2,3-b] pyridin-2-yl) -5,6-diméthoxy-1- (2-morpholin-4-yléthyl)-1H-pyrrolo[3,2-b]pyridine ;
3 - (4-Chloro-1H-pyrrolo[2,3-b]pyridin-2-yl)-5,6-dimétoxy-1-[2-(4-méthylpipérazin-1- yl) éthyl]-1H-pyrrolo[3,2-b]pyridine :
3 - (5-fluoro-1H-pyrrolo[2,3-b]pyridin-2-yl)5,6-diméthoxy-1-(2-morpholin-4-yl-éthyl)-1H-pyrrolo[3,2-b]pyridine ;
3-(4-chloro-5-fluoro-1H-pyrrolo[2,3-b]pyridin-2-yl)-5,6-diméthoxy-1-(2-morpholin-4-yl-éthyl)-IH-pyrrolo[3,2-b]pyridine ;
3-(4-Chloro-5-fluoro-1H-pyrrolo[2,3-b]pyridin-2-yl)-5,6-diméthoxy-1-[2-(4-méthylpipérazin-1-yl)-éthyl]-1H-pyrrolo[3,2-b]pyridine
3-[4-Chloro-5-fluoro-1H-pyrrolo[2.3-b]pyridin-2-yl] -5,6-diméthoxy-1-(3-pipéridin-1-yl-propyl)-1H-pyrrolo[3,2-b]pyridine ;
1-{3-[3-(4-Chloro-5-fluoro-1H-pyrrolo[2,3-b]pyridin-2-yl)-5,6-diméthoxy-pyrrolo[3,2-b] pyridin-1-yl]-propyl}-pipéridin-4-ol ;
C-(1-{3-[3-(4-chloro-5-fluoro-1H-pyrrolo[2,3-b]pyridin-2-yl)-5,6-diméthoxy-pyrrolo[3,2-b] pyridin-1-yl]-propyl}-pipéridin-4-yl)-méthylamine ;
3-(4-Chloro-5-fluoro-1H-pyrrolo[2,3-b]pyridin-2-yl)-5,6-dimethoxy-1-[3-(4-méthyl-pipérazin-1-yl)-propyl]-1H-pyrrolo[3,2-b]pyridine ;
C-(1-{2[3-(4-Chloro-1H-pyrrolo[2,3-b]pyridin-2-yl)-5,6-diméthoxy-pyrrolo[3,2-b]pyridin-1-yl]-éthyl}-piperidin-4-yl)-méthylamine ;
[3-(4-chloro-1H-pyrrolo [2,3-b] pyridin-2- yl) -5,6-diméthoxy-pyrrolo [3,2-b] pyridin-1-yl]-acétate de potassium ;
2-[3-(4-Chloro-1H-pyrrolo [2,3-b] pyridin-2- yl)-5,6-diméthoxy-pyrrolo [3,2-b] pyridin-1-yl]-1-(4-méthyl-piperazin-1-yl)-éthanone ;
2-[3-(4-Chloro-1H-pyrrolo[2,3-b]pyridin-2-yl)-5,6-diméthoxy-pyrrolo[3,2-b]pyridin-1-yl]-1-(4-hydroxy-pipéridin-1-yl)-éthanone ;
2-[3-(4-Chloro-1H-pyrrolo[2,3-b]pyridin-2-yl)-5,6-dimethoxy-pyrrolo[3,2-b]pyridin-1-yl]-1-morpholin-4-yl-éthanone ;
2-(1-{3-[3-(4-Chloro-1H-pyrrolo[1,3-b]pyridin-2-yl)-5,6-diméthoxy-pyrrolo[3,2-b]pyridin-1-yl]-propyl}-pipéridin-4-yl)-éthanol;
1-{3-[3-(4-Chloro-1H-pyrrolo[2,3-b]pyridin-2-yl)-5,6-diméthoxy-pyrrolo[3,2-b]pyridin-1-yl] -propyl}-pipéridin-4-ol;
{3-[3-(4-Chloro-1H-pyrrolo[2,3-b] pyridin-2-yl)-5,6-diméthoxy-pyrrolo[3,2-b]pyridin-1-yl]-propyl}-diéthyl-amine ;
3-(4-Chloro-1H-pyrrolo[2,3-b]pyridin-2-yl)-5,6-diméthoxy-1-[3-(4-méthyl-perhydro-1,4-diazépin-1-yl)-propyl]-1H-pyrrolo[3,2-b]pyridine ;
3-(4-Chloro-1H-pyrrolo[2,3-b]pyridin-2-yl)-5,6-diméthoxy-1-(3-pipéridin-1-yl-propyl)-1H-pyrrolo[3,2-b]pyridine ;
2-(5,6-Diméthoxy-1H-pyrrolo[3,2-b]pyridin-3-yl)-1H-pyrrolo[2,3-b]pyridine-4-carbonitrile ;
2-(5,6-Diméthoxy-1-méthyl-1H-pyrrolo[3,2-b]pyridin-3-yl)-1H-pyrrolo[2,3-b]pyridine-4-carbonitrile ;
2-[3-(4-Chloro-1H-pyrrolo[2,3-b]pyridin-2-yl)-5,6-diméthoxy-pyrrolo[3,2-b]pyridin-1-yl]-N-méthoxy-N-méthyl-acétamide ;
2-(5,6-diméthoxy-1-méthyl-1H-pyrrolo[3,2-b]pyridin-3-yl)-1H-pyrrolo[2,3-b]pyridin-4-carbaldéhyde ;
4-{[2-(5,6-D)methoxy-1-methy)-1H-pyrrolo[3,2-b]pyridin-3-yl)-1H-pyrrolo[2,3-b]pyridin-4-ylméthyl]-amino}-phénol ;
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I). minérales et organiques desdits produits de formule (I).

2. A titre de médicaments, les produits de formule (I) telle que définie à la revendication 1, lesdits produits de formule (I) étant sous toutes les formes isomères possible racémiques, énantiomères et diastéréoisomères ainsi que les sels d'addition avec les acides minéraux et organiques ou avec les bases minérales et organiques pharmaceutiquement acceptables desdits produits de formule (I).

3. Les compositions pharmaceutiques contenant à titre de principe actif, l'un au moins des médicaments tels que définis à la revendication 2.

4. Compositions pharmaceutiques telles que définies aux autres revendications contenant en plus, des principes actifs d'autres médicaments de chimiothérapie contre le cancer.

5. Compositions pharmaceutiques selon l'une quelconque' des autres revendications **caractérisées en ce qu'**elles sonc utilisées comme médicaments, en particulier pour la chimiothérapie de cancers.

6. Utilisation de produits de formule (I) telle que définie à la revendication 1 ou de sels pharmaceutiquement acceptables desdits produits de formule (I) pour la préparation d'un médicament destiné à prévenir ou traiter une maladie appartenant au groupe suivants désordres de la prolifération de vaisseaux sanguins, désordres fibrotiques,
désordres des prolifération de cellules mésangiales, l'acromégalie, désordres métaboliques, allergies, asthme, maladie de Crohn's, thromboses, maladies du système nerveux, rétinopathies, psoriasis, arthrite rhumatoïde, diabètes, dégénération musculaire, gériatrie, dégénerescence musculaire due à l'âge, maladies en oncologie, cancers.

7. Utilisation de produits de formule (I) telle que définie à la revendication 1 ou de sels pharmaceutiquement acceptables desdits produits de formule (I) pour la préparation d'un médicament destiné à traiter des maladies en oncologie.

8. Utilisation de produits de formule (I) telle que définie à la revendication 1 ou de sels pharmaceutiquement acceptables desdits produits de formule (I) pour la préparation d'un médicament destiné à traiter des cancers.

9. Utilisation de produits de formule (I) selon la revendication précédente dans laquelle la maladie à traiter est un cancer de tumeurs solides.

10. Utilisation de produits de formule (I) selon la revendication précédente dans laquelle la maladie à traiter est un cancer résistant aux agents cytotoxiques.

11. Utilisation de produits de formule (I) telle que définie à la revendication 1 ou de sels pharmaceutiquenent acceptables desdits produits de formule (I) pour la préparation d'un médicament destiné à traiter des cancers parmi lesquels les cancers du sein, de l'estomac, du colon, des poumons, des ovaires, de l'utérus, du cerveau, du rein, du larynx, du système lymphatique, de la thyroïde, du tractus uro-génital, du tractus incluant vésicule et prostate, du cancer des os, du pancréas, les mélanomes.

12. utilisation de produits de formule (I) selon la revendication précédente dans laquelle la maladie à traiter est un cancer du sein, du colon ou des poumons.

13. Utilisation de produits de formule (I) telle que définis à la revendication 1 ou de sels pharmaceutiquement acceptables desdits produits de formule (I) pour la préparation d'un médicament destiné à le chimiothérapie de cancers.

14. Utilisation de produits de formule (I) telle que définis à la revendication 1 ou de sels pharmaceutiquement acceptables desdits produits
de formule (I) pour la préparation de médicaments destinés à la chimiothérapie de cancers utilisés seuls ou en association.

15. Utilisation de produits de formule (I) telle que définie à la revendication 1 ou de seuls pharmaceutiquement acceptables desdits produits de formule (I) pour la préparation de médicaments destinés à être utilisés seuls ou en association avec chimiothérapie ou radiothérapie au alternativement en association avec d'autres agents thérapeutiques.

16. Utilisation de produits de formule (I) selon la revendication précédente dans laquelle les agents thérapeutiques peuvent être des agents anti-tumoraux utilisés communément.

17. Produits de formule (I) tels que définis à la revendication 1 comme inhibiteurs de protéines kinases IGFIR, lesdits produits de formule (I) étant sous toutes les formes isoméres possibles racémiques, énantioméres et diastéréo-isoméres, ainsi que les sels d'addition avec les acides minéraux et organiques ou avec les bases minérales et organiques pharmaceutiquement acceptables desdits produits de formule (I).

## Claims

1. Products of formula (I) 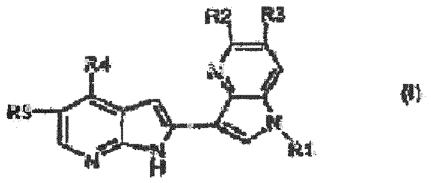 chosen from the following compounds:
3-(4-Chloro-1H-pyrrolo[2,3-b]pyridin-2-yl)-5,6-dimethoxy-1-methyl-1H-pyrrolo[3,2-b]pyridine;
3-(4-Chloro-1H-pyrrolo[2,3-b]pyridin-2-yl)-5,6-dimethoxy-1-(2-morpholin-4-yl)ethyl-1H-pyrrolo[3,2-b]pyridine;
3-(4-Chloro-1H-pyrrolo[2,3-b]pyridin-2-yl)-5,6-dimethoxy-1-[2-(4-methylpiperazin-1-yl)ethyl]-1H-pyrrolo[3,2-b]pyridine;
3-(5-Fluoro-1H-pyrrolo[2,3-b]pyridin-2-yl)-5,6-dimethoxy-1-(2-morpholin-4-yl)ethyl-1H-pyrrolo[3,2-b]pyridine;
3-(4-Chloro-5-fluoro-1H-pyrrolo[2,3-b]pyridin-2-yl)-5,6-dimethoxy-1-(2-morpholin-4-yl)ethyl-1H-pyrrolo[3,2-b]pyridino;
3-(4-Chloro-5-fluoro-1H-pyrrolo[2,3-b]pyridin-2-yl)-5,6-dimethoxy-1-[2-(4-methylpiperazin-1-yl)ethyl]-1H-pyrrolo[3,2-b]pyridine;
3-[4-Chloro-5-fluoro-1H-pyrrolo[2,3-b]pyridin-2-yl]-5,6-dimethoxy-1-(3-(piperidin-1-yl)propyl)-1H-pyrrolo[3,2-b]pyridine;
1-{3-[3-(4-Chloro-5-fluoro-1H-pyrrolo[2,3-b]pyridin-2-yl)-5,6-dimethoxy-1H-pyrrolo[3,2-b]pyridin-1-yl]propyl}piperidin-4-ol;
C-(1-{3-[3-(4-Chloro-5-fluoro-1H-pyrrolo[2,3-b]pyridin-2-yl)-5,6-dimethoxy-1H-pyrrolo[3,2-b]pyridin-1-yl]propyl}piperidin-4-yl)methylamine;
3-(4-Chloro-5-fluoro-1H-pyrrolo[2,3-b]pyridin-2-yl)-5,5-dimethoxy-1-[3-(4-methylpiperazin-1-yl)propyl]-1H-pyrrolo[3,2-b]pyridine;
C-(1-{2-[3-(4-Chloro-1H-pyrrolo[2,3-b]pyridin-2-yl)-5,6-dimethoxy-1H-pyrrolo[3,2-b]pyridin-1-yl]ethyl}piperidin-4-yl)methylamine;
Potassium [3-(4-chloro-1H-pyrrolo[2,3-b]pyridin-2-yl)-5,6-dimethoxy-1H-pyrrolo[3,2-b]pyridin-1-yl]-acetate;
2-[3-(4-Chloro-1H-pyrrolo[2,3-b] pyridin-2-yl)-5,6-dimethoxy-1H-pyrrolo[3,2-b]pyridin-1-yl]-1-(4-methylpiperazin-1-yl)ethanone;
2-[3-(4-Chloro-1H-pyrrolo[2,3-b]pyridin-2-yl)-5,6-dimethoxy-1H-pyrrolo[3,2-b]pyridin-1-yl]-1-(4-hydroxypiperidin-1-yl)ethanone;
2-[3-(4-Chloro-1H-pyrrolo[2,3-b]pyridin-2-yl)-5,6-dimethoxy-1H-pyrrolo[3,2-b]pyridin-1-yl]-1-(morpholin-4-yl)ethanone;
2-(1-{3-[3-(4-Chloro-1H-pyrrolo[2,3-b]pyridin-2-yl)-5,6-dimethoxy-1H-pyrrolo[3,2-b]pyridin-1-yl]propyl}piperidin-4-yl)ethanol;
1-{3-[3-(4-Chloro-1H-pyrrolo[2,3-b]pyridin-2-yl)-5,6-dimethoxy-1H-pyrrolo[3,2-b]pyridin-1-yl]propyl}piperidin-4-ol;
{3-[3-(4-Chloro-1H-pyrrolo[2,3-b]pyridin-2-yl)-5,6-dimethoxy-1H-pyrrolo[3,2-b]pyridin-1-yl]propyl}diethylamine;
3-(4-Chloro-1H-pyrrolo[2,3-b]pyridin-2-yl)-5,6-dimethoxy-1-[3-(4-methylperhydro-1,4-diazepin-1-yl)propyl]-1H-pyrrolo[3,2-b]pyridine;
3-(4-Chloro-1H-pyrrolo[2,3-]pyridin-2-yl)-5,6-dimethoxy-1-(3-(piperidin-1-yl)propyl)-1H-pyrrolo[3,2-b]pyridine;
2-(5,6-Dimethoxy-1H-pyrrolo[3,2-b]pyridin-3-yl)-1H-pyrrolo[2,3-b]pyridine-4-carbonitrile;
2-(5,6-Dimethoxy-1-methyl-1H-pyrrolo[3,2-b]pyridin-3-yl)-1H-pyrrolo[2,3-b]pyridine-4-carbonitrile;
2-[3-(4-Chloro-1H-pyrrolo[2,3-b]pyridin-2-yl)-5,6-dimethoxy-1H-pyrrolo[3,2-b]pyridin-1-yl]-N-methoxy-N-methylacetamide;
2-(5,6-Dimethoxy-1-methyl-1H-pyrrolo[3,2-b]pyridin-3-yl)-1H-pyrrolo[2,3-]pyridine-4-carbaldehyde;
4-{[2-(5,6-Dimethoxy-1-methyl-1H-pyrrolo[3,2-b]pyridin-3-yl)-1H-pyrrolo[2,3-b]pyridin-4-ylmethyl]amino}phenol;
the said products of formula (I) being in all the possible isomeric forms, racemic, enantiomeric and diastereoisomeric, and the addition salts with inorganic and organic acids or with inorganic and organic bases of said products of formula (I).

2. As medicaments, the products of formula (I) as defined in Claim 1, the said products of formula (I) being in all the possible isomeric forms, racemic, enantiomeric and diastereoisomeric, and the addition salts with inorganic and organic acids or with inorganic and organic bases which are pharmaceutically acceptable of the said products of formula (I).

3. The pharmaceutical compositions comprising, as active principle, at least one of the medicaments as defined in Claim 2.

4. Pharmaceutical compositions as defined in the other claims, additionally comprising active principles of other medicaments for anticancer chemotherapy.

5. Pharmaceutical compositions according to any one of the other claims, **characterized in that** they are used as medicaments, in particular for cancer chemotherapy.

6. Use of products of formula (I) as defined in Claim 1 or of pharmaceutically acceptable salts of the said products of formula (I) in the preparation of a medicament intended to prevent or treat a disease belonging to the following group: blood vessel proliferation disorders, fibrotic disorders, mesangial cell proliferation disorders acromegaly, metabolic disorders, allergies, asthma, Crohn's disease, thromboses, diseases of the nervous system, retinopathies, psoriasis, rheumatoid arthritis, diabetes, muscle degeneration, geriatrics, age-related muscle degeneration, diseases in oncology or cancers.

7. Use of products of formula (I) as defined in Claim 1 or of pharmaceutically acceptable salts of the said products of formula (I) in the preparation of a medicament intended to treat diseases in oncology.

8. Use of products of formula (I) as defined in Claim 1 or of pharmaceutically acceptable salts of the said products of formula (I) in the preparation of a medicament intended to treat cancers.

9. Use of products of formula (I) according to the preceding claim in which the disease to be treated is a solid tumour cancer.

10. Use of products of formula (I) according to the preceding claim in which the disease to be treated is a cancer resistant to cytotoxic agents.

11. Use of products of formula (I) as defined in Claim 1 or of pharmaceutically acceptable salts of said products of formula (I) in the preparation of a medicament intended to treat cancers, including cancers of the breast, stomach, colon, lungs, ovaries, uterus, brain, kidney, larynx, lymphatic system, thyroid, urogenital tract, including bladder and prostate, bones and pancreas, and melanomas.

12. Use of products of formula (I) according to the preceding claim in which the disease to be treated is a cancer of the breast, colon or lungs.

13. Use of products of formula (I) as defined in Claim 1 or of pharmaceutically acceptable salts of the said products of formula (I) in the preparation of a medicament intended for cancer chemotherapy.

14. Use of products of formula (I) as defined in Claim 1 or of pharmaceutically acceptable salts of the said products of formula (I) in the preparation of medicaments intended for cancer chemotherapy, used alone or in combination.

15. Use of products of formula (I) as defined in Claim 1 or of pharmaceutically acceptable salts of the said products of formula (I) in the preparation of medicaments intended to be used alone or in combination with chemotherapy or radiotherapy or alternatively in combination with other therapeutic agents.

16. Use of products of formula (I) according to the preceding claim in which the therapeutic agents can be commonly used antitumor agents.

17. Products of formula (I) as defined in Claim 1 as protein kinase IGF-1R inhibitors, the said products of formula (I) being in all the possible isomeric forms, racemic, enantiomeric and diastereoisomeric, and the addition salts with inorganic and organic acids or with inorganic and organic bases which are pharmaceutically acceptable of the said products of formula (I).

## Patentansprüche

1. Produkte der Formel (I), 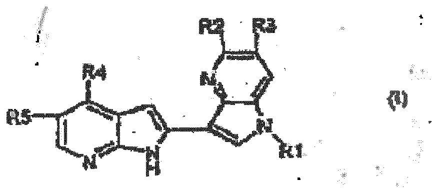 die aus den folgenden Verbindungen ausgewählt sind:
3-(4-Chlor-1H-pyrrolo[2,3-b]pyridin-2-yl)-5,6-dimethoxy-1-methyl-1H-pyrrolo[3,2-b]pyridin;
3-(4-Chlor-1H-pyrrolo[2,3-b]pyridin-2-yl)-5,6-dimethoxy-1-(2-morpholin-4-ylethyl)-1H-pyrrolo[3,2-b]pyridin;
3-(4-Chlor-1H-pyrrolo[2,3-b]pyridin-2-yl)-5,6-dimethoxy-1-[2-(4-methylpiperazin-1-yl)ethyl]-1H-pyrrolo[3,2-b]pyridin;
3-(5-Fluor-1H-pyrrolo[2,3-b]pyridin-2-yl)-5,6-dimethoxy-1-(2-morpholin-4-ylethyl)-1H-pyrrolo[3,2-b]pyridin;
3-(4-Chlor-5-fluor-1H-pyrrolo[2,3-b]pyridin-2-yl)-5,6-dimethoxy-1-(2-morpholin-4-ylethyl)-1H-pyrrolo[3,2-b]pyridin;
3-(4-Chlor-5-fluor-1H-pyrrolo[2,3-b]pyridin-2-yl)-5,6-dimethoxy-1-[2-(4-methylpiperazin-1-yl)ethyl]-1H-pyrrolo[3,2-b]pyridin;
3-[4-Chlor-5-fluor-1H-pyrrolo[2,3-b]pyridin-2-yl] 5,6-dimethoxy-1-(3-piperidin-1-ylpropyl)-1H-pyrrolo[3,2-b]pyridin;
1-{3-[3-(4-Chlor-5-fluor-1H-pyrrolo[2,3-b]pyridin-2-yl)-5,6-dimethoxypyrrolo[3,2-b]pyridin-1-yl]propyl}piperidin-4-ol;
C-(1-{3-[3-(4-Chlor-5-fluor-1H-pyrrolo[2,3-b]pyridin-2-yl)-5,6-dimethoxypyrrolo[3,2-b]pyridin-1-yl]propyl}piperidin-4-yl)methylamin;
3-(4-Chlor-5-fluor-1H-pyrrolo[2,3-b]pyridin-2-yl)-5,6-dimethoxy-1-[3-(4-methylpiperazin-1-yl)propyl]-1H-pyrrolo[3,2-b]pyridin;
C-(1-{2-[3-(4-Chlor-1H-pyrrolo[2,3-b]pyridin-2-yl)-5,6-dimethoxypyrrolo[3,2-b]pyridin-1-yl]ethyl}piperidin-4-y)methylamin;
Kalium[3-(4-chlor-1H-pyrrolo[2,3-b]pyridin-2-yl)-5,6-dimethoxypyrrolo[3,2-b]pyridin-1-yl]acetat;
2-[3-(4-Chlor-1H-pyrrolo[2,3-b]pyridin-2-yl)-5,6-dimethoxypyrrolo[3,2-b]pyridin-1-yl]-1-(4-methylpiperazin-1-yl)ethanon;
2-[3-(4-Chlor-1H-pyrrolo[2,3-b]pyridin-2-yl)-5,6-dimethoxypyrrolo[3,2-b]pyridin-1-yl]-1-(4-hydroxypiperidin-1-yl)ethanon;
2-[3-(4-Chlor-1H-pyrrolo[2,3-b]pyridin-2-yl)-5,6-dimethoxypyrrolo[3,2-b]pyridin-1-yl]-1-morpholin-4-ylethanon;
2-(1-{3-[3-(4-Chlor-1H-pyrrolo[2,3-b]pyridin-2-yl)-5,6-dimethoxypyrrolo[3,2-b]pyridin-1-yl]propyl}piperidin-4-yl)ethanol;
1-{3-[3-(4-Chlor-1H-pyrrolo[2,3-b]pyridin-2-yl)-5,6-dimethoxypyrrolo[3,2-b]pyridin-1-yl]propyl}piperidin-4-ol;
{3-[3-(4-Chlor-1H-pyrrolo[2,3-b]pyridin-2-yl)-5,6-dimethoxypyrrolo[3,2-b]pyridin-1-yl]propyl}diethylamin;
3-(4-Chlor-1H-pyrrolo[2,3-b]pyridin-2-yl)-5,6-dimethoxy-1-[3-(4-methylperhydro-1,4-diazepin-1-yl)propyl]-1H-pyrrolo[3,2-b]pyridin;
3-(4-Chlor-1H-pyrrolo[2,3-b]pyridin-2-yl)-5,6-dimethoxy-1-[3-piperidin-ylpropyl]-1H-(4-pyrrolo[3,2-b]pyridin;
2-(5,6-Dimethoxy-1H-pyrrolo[3,2-b]pyridin-3-yl)-1H-pyrrolo[2,3-b]pyridin-4-carbonitril;
2-(5,6-Dimethoxy-1-methyl-1H-pyrrolo[3,2-b]pyridin-3-yl)-1H-pyrrolo[2,3-b]pyridin-4-carbonitril;
2-[3-(4-Chlor-1H-pyrrolo[2,3-b]pyridin-2-yl)-5,6-dimethoxypyrrolo[3,2-b]pyridin-1-yl]N-methoxy-N-methylacetamid;
2-(5,6-Dimethoxy-1-methyl-1H-pyrrolo[3,2-b]pyridin-3-yl)-1H-pyrrolo[2,3-b]pyridin-4-carbaldehyd;
4-{[2-(5,6-Dimethoxy-1-methyl-1H-pyrrolo[3,2-b]pyridin-3-yl)-1H-pyrrolo[2,3-b]pyridin-4-ylmethyl]amino}phenol;
wobei die Produkte der Formel (I) in allen möglichen racemischen, enantiomeren und diastereoisomeren Isomerenformen vorliegen, sowie die Additionssalze mit anorganischen und organischen Säuren oder mit anorganischen und organischen Basen der Produkte der Formel (I).

2. Produkte der Formel (I) wie in Anspruch 1 definiert als Arzneimittel, wobei die Produkte der Formel (I) in allen möglichen racemischen, enantiomeren und diastereomeren Isomerenformen vorliegen, sowie die Additionssalze mit anorganischen und organischen Säuren oder mit anorganischen und organischen Basen der Produkte der Formel (I).

3. Pharmazeutische Zusammensetzungen, die als Wirkstoff mindestens eines der Arzneimittel wie in Anspruch 2 definiert umfassen.

4. Pharmazeutische Zusammensetzungen wie in den anderen Ansprüchen definiert, die weiterhin Wirkstoffe von anderen Arzneimitteln für die Chemotherapie gegen Krebs enthalten:

5. Pharmazeutische Zusammensetzungen nach einem der anderen Ansprüche, **dadurch gekennzeichnet, dass** sie als Arzneimittel eingesetzt werden, insbesondere für die Chemotherapie von Krebserkrankungen.

6. Verwendung von Produkten der Formel (I) wie in Anspruch 1 definiert oder von pharmazeutisch unbedenklichen Salzen dieser Produkte der Formel (I) für die Herstellung eines Arzneimittels für Vorbeugung oder Behandlung einer Krankheit aus der folgenden Gruppe: Störungen der Blutgefäßproliferation, fibrotische Störungen, Störungen der Mesangialzellenproliferation, Akromegalie, Stoffwechselstörungen, Allergien, Asthma, Morbus Crohn, Thrombosen, Erkrankungen des Nervensystems, Retinopathien, Psoriasis, rheumatoide Arthritis, Diabetes, Muskeldegeneration, Geriatrie, altersbedingte Muskelverkümmerung, onkologische Erkrankungen, Krebserkrankungen.

7. Verwendung von Produkten der Formel (I) wie in Anspruch 1 definiert oder von pharmazeutisch unbedenklichen Salzen dieser Produkte der Formel (I) für die Herstellung eines Arzneimittels für Vorbeugung oder Behandlung von onkologischen Erkrankungen.

8. Verwendung von Produkten der Formel (I) wie in Anspruch 1 definiert oder von pharmazeutisch unbedenklichen Salzen dieser Produkte der Formel (I) für die Herstellung eines Arzneimittels für Vorbeugung oder Behandlung von Krebserkrankungen.

9. Verwendung von Produkten der Formel (I) nach dem vorhergehenden Anspruch, wobei es sich bei der zu behandelnden Krankheit um einen Krebs mit soliden Tumoren handelt.

10. Verwendung von Produkten der Formel (I) nach dem vorhergehenden Anspruch, wobei es sich bei der zu behandelnden Krankheit um einen Krebs, der gegen Cytotoxika resistent ist, handelt.

11. Verwendung von Produkten der Formel (I) wie in Anspruch 1 definiert oder von pharmazeutisch unbedenklichen Salzen dieser Produkte der Formel (I) für die Herstellung eines Arzneimittels zur Behandlung von Krebserkrankungen, darunter Brustkrebs, Magenkrebs, Dickdarmkrebs, Lungenkrebs, Eierstockkrebs, Gebärmutterkrebs, Hirnkrebs, Nierenkrebs, Kehlkopfkrebs, Krebs des lymphatischen Systems, Schilddrüsenkrebs, Krebs des Urogenitaltrakts, einschließlich Blase und Prostata, Knochenkrebs, Bauchspeicheldrüsenkrebs, Melanome.

12. Verwendung von Produkten der Formel (I) nach dem vorhergehenden Anspruch, wobei es sich bei der zu behandelnden Krankheit um Brustkrebs, Dickdarmkrebs oder Lungenkrebs handelt.

13. Verwendung von Produkten der Formel (I) wie in Anspruch 1 definiert oder von pharmazeutisch unbedenklichen Salzen dieser Produkte der Formel (I) für die Herstellung eines Arzneimittels zur Chemotherapie von Krebserkrankungen.

14. Verwendung von Produkten der Formel (I) wie in Anspruch 1 definiert oder von pharmazeutisch unbedenklichen Salzen dieser Produkte der Formel (I) für die Herstellung eines Arzneimittels zur Chemotherpie von Krebserkrankungen, und zwar bei alleiniger Verwendung oder in Kombination.

15. Verwendung von Produkten der Formel (I) wie in Anspruch 1 definiert oder von pharmazeutisch unbedenklichen Salzen dieser Produkte der Formel (I) für die Herstellung von Arzneimitteln für die alleinige Verwendung oder die Verwendung in Kombination mit Chemotherapie oder Radiotherapie oder auch in Kombination mit anderen Therapeutika.

16. Verwendung von Produkten der Formel (I) nach dem vorhergehenden Anspruch, wobei es sich bei den Therapeutika um üblicherweise eingesetzte Antitumormittel handelt.

17. Produkte der Formel (I) wie in Anspruch 1 definiert als IGFIR-Proteinkinasehemmer, wobei die Produkte der Formel (I) in allen möglichen racemischen, enantiomeren und diastereomeren Isomerenformen vorliegen, sowie die pharmazeutisch unbedenklichen Additionssalze mit anorganischen und organischen Säuren oder mit anorganischen und organischen Basen dieser Produkte der Formel (I).
